(19) Europäisches Patentamt — European Patent Office — Office européen des brevets

(11) **EP 4 072 651 B1**

(12) **EUROPÄISCHE PATENTSCHRIFT**

(45) Veröffentlichungstag und Bekanntmachung des Hinweises auf die Patenterteilung:
**17.04.2024 Patentblatt 2024/16**

(21) Anmeldenummer: **20820947.8**

(22) Anmeldetag: **11.12.2020**

(51) Internationale Patentklassifikation (IPC):
**A61N 1/04** (2006.01)   **A61N 1/06** (2006.01)
**A61N 1/36** (2006.01)   **A61N 1/40** (2006.01)

(52) Gemeinsame Patentklassifikation (CPC):
**A61N 1/06; A61N 1/0468; A61N 1/36034; A61N 1/40**

(86) Internationale Anmeldenummer:
**PCT/EP2020/085655**

(87) Internationale Veröffentlichungsnummer:
**WO 2021/116358 (17.06.2021 Gazette 2021/24)**

(54) **THERAPIEVORRICHTUNG ZUR ZELLTHERAPIE ODER ZELLSTIMULATION**

THERAPEUTIC DEVICE FOR CELL THERAPY OR STIMULATION

DISPOSITIF THÉRAPEUTIQUE POUR LA THÉRAPIE OU STIMULATION CELLULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **10.12.2019 EP 19214663**

(43) Veröffentlichungstag der Anmeldung:
**19.10.2022 Patentblatt 2022/42**

(73) Patentinhaber: **Activcell Group AG
6295 Mosen (CH)**

(72) Erfinder:
• **ERDMANN, Raimund
5200 Brugg (CH)**
• **LAUBSCHER, Urs
4410 Liestal (CH)**

(74) Vertreter: **Herrmann, Johanna
Industrial Property Services GmbH
Rosenweg 14
4303 Kaiseraugst (CH)**

(56) Entgegenhaltungen:
DE-A1- 2 822 892      US-A1- 2004 267 333
US-A1- 2011 022 043   US-A1- 2012 107 896
US-A1- 2014 214 118   US-A1- 2014 219 894
US-A1- 2019 117 969   US-B2- 8 103 340

**Beschreibung**

Die vorliegende Erfindung betrifft eine Therapievorrichtung zur Zelltherapie oder Zellstimulation gemäss dem Oberbegriff des Anspruchs 1.

Aus dem Stand der Technik sind Therapiegeräte bekannt, welche die Technologie der physiologischen Elektrostimulation, in der Literatur auch als Elektrotherapie bezeichnet, verwenden. Derartige Therapiegeräte basieren auf dem Prinzip der Abgabe von elektrischer Energie an die biologische Zelle. Diese Technologie findet in der Medizin für die sogenannte Hochfrequenztherapie Verwendung. Ein mit einem HF-Generator erzeugtes elektrisches Feld wird mit einer Abgabeeinrichtung zur impulsweisen Abgabe der HF-Leistung an die Zelle kombiniert, um die Zellstimulation zu ermöglichen.

Das Einsatzgebiet der erfindungsgemässen Therapievorrichtung liegt im Bereich von Wellnessapplikationen, Fitness, Kosmetik, Schmerzreduktion, Wundheilung, zur Zelltherapie oder weiteren Zellstimulation zur Behandlung beim Menschen oder am Tier.

Im Dokument DE 2822892 A1 ist ein Beispiel für ein derartiges Therapiegerät gezeigt, nämlich ein Gerät zum Aufrechterhalten des negativen Potentials von menschlichen, tierischen und pflanzlichen Zellen und/oder zum Penetrieren von Substanzen in die Zellen. Ein mittels einer Steuerschaltung beeinflussbarer Generator erzeugt hochfrequente Impulse von einstellbarer Folgefrequenz und Dauer und ein Stromkreis erzeugt eine Gleichspannung und eine pulsierende Gleichspannung zur Ionisation und Impulse bestimmter Form und veränderlicher Folgefrequenz zur Faradisation des zu behandelnden Gewebes. Eine Therapiesitzung wird über ein vorbestimmtes Zeitintervall durchgeführt.

Obschon mit diesem vorbekannten Therapiegerät bezüglich seines Verwendungszweckes gute Resultate erzielt werden, treten für die Benutzer während des Betriebs folgende Nachteile auf:

- Das Gerät ist in der Anwendung während einer Therapie schwerfällig, da nur eine drahtgebundene Stromversorgung vorhanden ist,
- die Einstellung der Energiestufe erfolgt über eine manuelle Regelung am Spannungsversorgungsgerät,
- die Pulsfrequenz der Energieabgabe kann nur in einem kleinen Bereich variiert werden, nämlich in einem Bereich von 10 bis 1000 Hz,
- die abgegebene Energie kann in ihrer Signalform nicht beeinflusst werden.

Damit wird ein nachhaltiger Therapieerfolg eingeschränkt, eine lokale Anwendung von fallspezifischen Impulsformen ist nicht möglich, sodass eine zu hohe Anzahl wiederkehrender Therapiesitzungen abgehalten werden muss und/oder eine eingeschränkte Wirkung auf die Zellen erfolgt. Der die Elektrode enthaltende Behandlungskopf hat einen Durchmesser von ca. 17 cm und eine Höhe von ca. 10 cm. Im Behandlungskopf ist eine Antenne angeordnet, die als eine elektrisch isolierende Kunststoffplatte ausgebildet ist, an deren patientenseitiger Oberfläche ein kreisförmig angeordneter, leitfähiger Belag angeordnet ist. An der gegenüberliegenden Oberfläche ist ein spiralförmiger leitfähiger Belag angeordnet, der an seinen Enden mit einem Abstimmkondensator verbunden ist.

Aus der EP 2397187 A1 ist ein Therapiegerät bekannt, welches von einer Gleichstromquelle gespeist wird. Mittels dieses Therapiegeräts wird ein magnetisches Feld zur Induktion von Strömen erzeugt, wodurch elektrische Signale in Nervenbahnen des Körpers stimuliert werden, wodurch Moleküle, Organe oder Gewebe eines Organismus angeregt werden können. Aufgrund des damit verbundenen relativ hohen Energieaufwands wurde nach Wegen gesucht, insbesondere lebende Zellen enthaltende biologische Materialien mit geringem Energieaufwand lokal zu behandeln.

Beispielsweise wurde hierzu gemäss DE10324926 B3 eine stiftförmige Elektrode vorgesehen, die an einen Wechselhochspannungsgenerator angeschlossen ist. Die Elektrode weist eine abgerundete Spitze auf, die von einem Dielektrikum abgedeckt wird. Das Dielektrikum dient einerseits zur Isolierung der Elektrode 3, andererseits dient es zur dielektrischen Behinderung einer Gasentladung, welche durch Anlegen einer Wechselhochspannung an die Elektrode zwischen dem Dielektrikum und der Oberfläche eines biologischen Materials gezündet werden kann und die ein kaltes Plasma über der Oberfläche des biologischen Materials erzeugt. Als Dielektrikum kann eine Keramik, Glas oder ein plasmaresistenter Kunststoff zum Einsatz kommen. Im Plasma befindet sich freier Sauerstoff, der chemisch auf das biologische Material einwirkt, um unerwünschte Mikroorganismen, Bakterien, entartetes Gewebe an der Oberfläche des biologischen Materials abzutöten.

In der DE 10 2008 045 830 A1 oder der EP 2 163 143 B1 ist eine Vorrichtung zur Behandlung eines Objekts mit einem Plasma offenbart, wobei das Plasma mittels einer Elektrode und einer Gegenelektrode erzeugt wird. Zwischen dem behandelten Objekt und der Elektrode ist ein Dielektrikum angeordnet, sodass ein Plasma mittels einer dielektrisch behinderten Gasentladung erzeugt wird, wobei dieses Plasma auf das zu behandelnde Objekt angewandt wird. Gemäss eines Ausführungsbeispiels besteht die Elektrode aus einem ionisierten Gas, beispielsweise einem Edelgas, Inertgas oder Gasgemisch, wobei das ionisierte Gas dadurch erzeugt wird, dass durch das Anlegen einer Hochspannung, die grösser als die Durchbruchspannung des Gases ist, das Gas ionisiert wird und als Plasma vorliegt. Das Gas wird somit elektrisch leitend und kann selbst als Elektrode genutzt werden.

Das Dokument US6866082 A offenbart ein Handgerät, welches eine Elektrode zur Erzeugung einer Gasentladung in der Luft zwischen der Elektrode und einem zu behandelnden Körperteil enthält. Die Elektrode ist als ein mit Neon gefüllter Glaskörper ausgebildet, der mit einer Folie elektrisch an einen Hochspannungstransformator gekoppelt ist. Der in der Luft zwischen der Elektrode und dem zu behandelnden Körperteil befindliche Sauerstoff wird durch Funkenbildung durch die Entladung eines in der Vorrichtung befindlichen Kondensators zur Ozonbildung angeregt. Der mit Neongas gefüllte Glaskörper verhindert bei einem Schaden durch Glasbruch einen Stromschlag, indem das Neongas entweicht und hierdurch eine Isolation zum Transformatorstromkreis erhalten wird.

Im Dokument US 2014/219894 A1 wird eine Gasbehandlungsanlage mit einem Katalysatorteil offenbart, die in einer Position vorgesehen ist, in der ein Plasma vorhanden ist, das durch den Plasmaerzeugungsabschnitt im Strömungsweg erzeugt wird, wodurch die Reaktion des Gases beschleunigt wird. Jedoch ist eine Gasbehandlungsvorrichtung für gefährliche Gase offenbart, und eine derartige Vorrichtung würde aus diesem Grund nicht zu Therapiezwecken eingesetzt werden.

Ausgehend von diesem Stand der Technik liegt der Erfindung die Aufgabe zugrunde, eine Therapievorrichtung bereit zu stellen, welche die Nachteile des Standes der Technik überwindet. Insbesondere ist es Aufgabe der Erfindung, eine Therapievorrichtung zu entwickeln, die einfacher zu bedienen ist, deren Pulsfrequenz und/oder Energieabgabe und/oder die Signalform individuell auf die beabsichtigte Behandlung oder Therapie abgestimmt werden kann, indem die Pulsfrequenz und/oder die Energieabgabe und/oder die Signalform in weiten Bereichen variiert werden können.

Die Aufgabe wird durch die Merkmale des Patentanspruchs 1 gelöst. Vorteilhafte Ausführungsformen der Therapievorrichtung sind Gegenstand der Ansprüche 2 bis 15.

Die Therapievorrichtung zur Zellstimulation oder Zelltherapie gemäss der Erfindung umfasst ein Gehäuse, welches eine Elektrode, einen Generator zur Erzeugung von hochfrequenten Spannungspulsen, eine Prozessoreinheit umfassend ein Steuer-, Regel- und Berechnungsmodul, eine Speichereinheit, mindestens ein Bedienelement und einen steuerbaren Modulator enthält, mittels welchem der Generator steuerbar ist. Mittels des Modulators ist eine Spannungspulsfolge umfassend eine Mehrzahl von Spannungspulsen erzeugbar, wobei mittels des Modulators die Frequenz und die Dauer der Spannungspulse beliebig einstellbar sind. Die Elektrode, der Generator, die Prozessoreinheit, die Speichereinheit, das Bedienelement und der Modulator sind im Gehäuse angeordnet. Die Elektrode enthält einen Glaskörper, der einen Hohlraum enthält, in welchem sich ein Gas befindet. Die Elektrode weist ein erstes Ende auf, welches mit dem Modulator koppelbar ist. Die Elektrode umfasst ein zweites kuppelförmiges Ende, wobei das Gas durch die auf die Elektrode übertragenen Spannungspulse in den Zustand eines ein nicht-thermischen Primärplasmas versetzbar ist, wobei ein Sekundärplasma durch Ionisierung der Luft erzeugbar ist, die sich im Umgebungsbereich des zweiten Endes der Elektrode befindet.

Das Gas kann insbesondere ein Edelgas umfassen, beispielsweise Helium, Neon, Argon. Das Gas befindet sich im Inneren des Glaskörpers, ist also im Hohlraum des Glaskörpers eingeschlossen. Das Gas wird durch Einbringung der Spannungspulse ionisiert, sodass eine Gasentladung von der Elektrode auf das zu behandelnde Körperteil erfolgen kann. Das Gas bildet das Primärplasma aus, welches durch Anlegen der Spannung, also der Übertragung der Spannungspulsfolge erzeugbar ist. Der Glaskörper hat die Wirkung einer dielektrischen Barriere. Mittels des Primärplasmas kann am zweiten Ende der Elektrode ein Sekundärplasma erhalten werden, sodass die Luft ionisiert und damit elektrisch leitend werden kann und eine Kopplung auf das zu behandelnde Körperteil erfolgen kann. Die vom Primärplasma erzeugte Hochspannung ist grösser als die Durchbruchspannung, wodurch insbesondere freier Sauerstoff oder Ionen in der das Ende der Elektrode umgebenen Luft entstehen, welche mit der Oberfläche des zu behandelnden Körperteils interagieren, sodass eine Zellstimulation erfolgen kann. Das Körperteil bildet hierbei die Kathode aus. Insbesondere wenn der Luftspalt zwischen der Kathode und dem ersten Ende des Glaskörpers kleiner als 3 mm ist, erfolgt eine Gasentladung zur Ausbildung des Sekundärplasmas. Die Elektrode kann gemäss eines Ausführungsbeispiels eine antimikrobielle Beschichtung enthalten.

Als Plasma wird ein Aggregatzustand der Materie definiert, in welchem in einer Gasphase geladene Teilchen mit positiven und negativen Ladungen vorliegen. Die Summe der positiven und der negativen Ladungen ist dabei gleich, sodass sich in einem betrachteten Volumen die positiven und negativen Ladungen kompensieren, das heisst, es ergibt sich insgesamt ein neutraler Ladungszustand. Das Plasma enthält auch Atome oder Moleküle mit neutralem Ladungszustand, die allerdings in elektronisch, vibratorisch oder rotatorisch angeregten Zuständen vorliegen können, die demzufolge als angeregte oder reaktive Teilchen bezeichnet werden.

Unter einem nicht-thermischen Plasma wird ein Plasma verstanden, bei welchem eine die Verteilung der kinetischen Energie der Elektronen des Plasmas beschreibende Temperatur, nachfolgend als Elektronentemperatur bezeichnet, höher ist als eine die Verteilung der kinetischen Energie der Ionen des Plasmas beschreibende Temperatur, nachfolgend als Ionentemperatur bezeichnet. Wenn die Ionentemperatur im Bereich von 25°C bis maximal 100°C liegt, wird das nichtthermische Plasma als Kaltplasma bezeichnet.

Die Therapievorrichtung gemäss eines Ausführungsbeispiels der vorliegenden Erfindung enthält somit ein direktes atmosphärisches Kaltplasmatherapiegerät. Mittels der das Primärplasma enthaltenden Elektrode wird ein sekundäres Kaltplasma erzeugt. Die Erzeugung eines sekundären Kaltplasmas hat den Vorteil, dass keine oder allenfalls eine geringfügige Erwärmung des zu behandelnden Körperteils erfolgt. Die Zellen des zu behandelnden Körperteils werden somit keinem unzulässigen Wärmeeinfluss ausgesetzt, welcher zu einer Schädigung der Zellen oder von deren Bestandteilen führen könnten.

Die Therapievorrichtung reagiert auf Berührung durch eine Veränderung der Intensität des Plasmas, sodass dessen Intensität in Abhängigkeit von der Halteposition veränderbar ist. Die höchste Intensität wurde in den Versuchsreihen festgestellt, wenn die Therapievorrichtung in der Nähe des der Elektrode gegenüberliegenden Endes gehalten wird. In den nachfolgend beschriebenen Messungen wurde daher die Therapievorrichtung in eine geerdete Aluminiumfolie gewickelt, um eine allfällige Beeinflussung der Messergebnisse durch die manuelle Manipulation der Therapievorrichtung auszuschliessen.

Gemäss eines Ausführungsbeispiels ist der Generator als Tesla-Spule ausgebildet. Mittels des Modulators wird die von der Energiespeichereinheit bereitgestellte Spannung auf die Eingangsspannung des Generators transformiert. Gemäss eines Ausführungsbeispiels liegt die maximale Frequenz im Bereich von 10 bis 100 Hz. Gemäss eines Ausführungsbeispiels liegt die maximale Spannung am Ausgang des Modulators im Bereich von 8 V bis 65 V. Der Modulator kann einen Transformator enthalten, mittels welchem die von der Energiespeichereinheit bereitgestellte Spannung auf die vom Generator benötigte Eingangsspannung transformiert werden kann. Gemäss eines Ausführungsbeispiels liegt die Spannung am Ausgang des Generators im Bereich von 5 kV bis einschliesslich 25 kV.

Gemäss eines Ausführungsbeispiels ist mittels des Modulators ist die Frequenz oder die Amplitude einstellbar. Die Amplitude und/oder die Frequenz der Spannung können mittels des Modulators moduliert werden. Gemäss eines Ausführungsbeispiels ist die Frequenz der Spannungspulsfolge zumindest abschnittweise nicht konstant. Gemäss eines Ausführungsbeispiels nimmt die Amplitude der Spannung während einer Zeitspanne t2-t1 zu, während einer Zeitspanne t3-t2 ist die Spannung konstant und während einer Zeitspanne t4-t3 nimmt die Spannung ab, wobei die Dauer der Spannungspulsfolge der Zeitspanne t4-t1 entspricht.

Insbesondere kann die Frequenz während der Zeitspanne t2-t1 zunehmen, während der Zeitspanne t3-t2 konstant bleiben und während der Zeitspanne t4-t3 abnehmen.

Mittels des Modulators kann somit eine beliebige Kombination von Spannungen und Frequenzen eingestellt werden. Mittels des Modulators kann somit eine beliebige Pulsfolge eingestellt werden. Die Pulsfolge wird an den Generator übermittelt und vom Generator in eine Pulsfolge mit entsprechend höherer Spannung übertragen. Die Hochspannung wird auf die Elektrode übertragen.

Gemäss eines Ausführungsbeispiels ist eine im Gehäuse angeordnete Energiespeichereinheit zur Energieversorgung zum Betrieb der Therapievorrichtung vorgesehen, sodass die Therapievorrichtung drahtlos betreibbar ist. Das Gehäuse kann ein Anzeigeelement enthalten, mittels welchem insbesondere Therapie- und Betriebsdaten darstellbar sind.

Gemäss eines Ausführungsbeispiels umfasst die Elektrode einen Sensor, mittels welchem der über die Elektrode abgegebene Strom oder die Spannung als Messwerte erfassbar sind, wobei die Messwerte in Messdaten digitalisierbar sind, wobei die Messdaten in der Speichereinheit speicherbar sind, wobei mittels des Berechnungsmoduls der Prozessoreinheit die abgegebene Energie und/oder der zeitliche Verlauf der von der Elektrode abgegebenen Energie ermittelbar ist.

Insbesondere erfolgt mittels des Regelmoduls der Prozessoreinheit eine Regelung des Modulators anhand der Messdaten, insbesondere zu einer Regelung einer konstanten Energieabgabe und/oder zu einer Regelung, die von der Signalform unabhängig ist, sodass eine beliebige Signalform im Generator generierbar ist, beispielsweise eine Kombination von Amplituden- und Frequenzmodulation. Die Messdaten können mittels des Steuermoduls der Prozessoreinheit zur Steuerung eines Therapieverlaufs eingesetzt werden.

Gemäss eines Ausführungsbeispiels sind die Messdaten in der Prozessoreinheit mit einem Zeitstempel verknüpfbar, wobei die mit dem Zeitstempel verknüpften Messdaten in der Speichereinheit zur Speicherung des Therapieverlaufs speicherbar sind.

Gemäss eines Ausführungsbeispiels kann das Gehäuse als einer der Pole eines Kondensators zur kapazitiven Koppelung ausgebildet sein.

Die Energiespeichereinheit kann als ein wiederaufladbares Element ausgebildet ist, beispielsweise als Lithium-Ionen Element oder als Superkapazität. Insbesondere kann eine Batterieladung für einen Betrieb der Therapievorrichtung für eine maximale Dauer von 50 min ausgebildet sein. Der Betrieb der Therapievorrichtung kann kontinuierlich insbesondere für eine Dauer von bis zu 25 min erfolgen. Die Betriebsdauer kann verlängert werden, wenn die Energiespeichereinheit gekühlt wird oder der Betrieb der Therapievorrichtung für eine Zeitdauer von ungefähr 30 min unterbrochen wird.

Gemäss eines Ausführungsbeispiels umfasst das Gehäuse eine Innenseite, welche eine elektrisch leitende oder leitfähige Fläche, beispielsweise einen leitenden Kunststoff oder einen mit einem elektrisch leitfähigen Material beschichteten Kunststoff enthält. Die Aussenseite des Gehäuses ist als elektrischer Isolator ausgebildet. Insbesondere kann das Gehäuse einen Kunststoff enthalten oder aus einem Kunststoff bestehen. Beispielsweise kann das Gehäuse ABS enthalten oder aus ABS bestehen. Insbesondere für Anwendungen im Lebensmittelbereich kann das Gehäuse PLA (Polylactid) enthalten oder aus PLA bestehen.

Die Therapievorrichtung verfügt über eine verbesserte Signalabgabe, welche vom Benutzer je nach Therapieform auswählbar ausgestaltet bzw. vorprogrammiert ist. Die Therapievorrichtung weist eine wesentlich verbesserte Handhabbarkeit aufgrund der integrierten Energiespeichereinheit auf, die einen drahtlosen Betrieb ermöglicht.

Dies wird erfindungsgemäss durch mindestens eines der nachfolgenden Merkmale ermöglicht:

- einen veränderbaren Signalverlauf, beispielsweise eine variierbare Frequenz des Hochfrequenzfeldes, welches über eine Elektrode abgegeben wird,
- die Regelung der Intensität der abgegebenen Energie, wobei typischerweise die abgegebene Leistung konstant und unabhängig von der Lage und/oder der Art der Elektroden bleibt,
- die Erhöhung der Handhabbarkeit durch eine im Gehäuse angeordnete Energiespeichereinheit, welche über eine eigene interne Stromversorgung verfügt und mittels Energiezellen, typischerweise mittels eines Akkus betrieben wird.

Die drahtlose Anwendung kann mittels eines neuen bisher ungenutzten physikalischen Prinzips bei der Therapieanwendung erleichtert und verbessert werden. Damit der Energiefluss des Hochfrequenzfeldes vom Anwender auf das Behandlungsobjekt erfolgen kann, wird gemäss eines Ausführungsbeispiels eine kapazitive Koppelung verwendet. Gemäss dieses Ausführungsbeispiels wird die Elektrode auf den Anwender kapazitiv gekoppelt und verbindet diesen mit dem Behandlungsobjekt mittels der Elektrode. Der Körper des Anwenders und das Behandlungsobjekt stellen dabei den Potentialausgleich über die kapazitive Koppelung sicher.

Der Vorteil der Erfindung in Bezug auf die regelbare, programmierbare und ihn ihrer Signalform einstellbare Energieabgabe besteht darin, dass verschiedene Therapieformen eine Optimierung erfahren.

Gemäss einer beispielhaften Therapieform erfolgt eine flächige Abgabe der Energie über die veränderliche Pulsfrequenz der Energieabgabe bei gleichzeitiger Modulation der Signalform. Diese beeinflusst unter anderem dem Fachmann bekannten Begriff des Skin-Effekts. Bei diesem Effekt werden die Elektronen, welche über die Elektrode abgegebene hochfrequente Spannung auf das Behandlungsobjekt gelangen, stärker an die Oberfläche des Behandlungsobjekts gezwungen. Bei einer tieferen Amplituden- und/oder Frequenzmodulation (AM/FM) tritt eine Verringerung des Skin-Effektes ein, mit dem Resultat, dass die Eindringtiefe und Dauer der Energieabgabe gezielter erfolgt.

Die Ausführung einer veränderbaren Modulation erfolgt dabei durch den Benutzer durch Betätigung von Bedienelementen der Therapievorrichtung und / oder durch eine der Therapieform entsprechend ausgelegte Therapievorrichtung, welche die benötigte Modulation aus einer Speichereinheit ausliest.

In einer weiteren Ausführungsform wird die Energieabgabe mittels eines Sensors, beispielsweise durch Messung einer Spannung und/oder eines Stroms gemessen und das Messresultat der Prozessoreinheit zugeführt. Die Prozessoreinheit enthält ein Regelmodul, mittels welchem je nach Therapiemethode verschiedene Zustände geregelt werden können.

Gemäss eines Ausführungsbeispiels wird die Energieabgabe während der Modulation derart moduliert, dass die Energieabgabe einer eigenen wählbaren oder aus der Speichereinheit abrufbaren Signalform folgt.

Gemäss eines Ausführungsbeispiels wird die Energieabgabe unabhängig von der verwendeten Elektrode, dem unterschiedlichen Kopplungsfaktor der kapazitiven Kopplung durch die drahtlose Verwendung während der Therapie oder der modulierten Signalform stabilisiert.

Die vom Sensor ermittelten Messwerte können in einer weiteren Ausführungsform als Signal der Prozessoreinheit zur Verfügung gestellt werden, welche die Messwerte als Messdaten verarbeiten kann. Insbesondere können mittels des Berechnungsmoduls die nachfolgend erwähnten Einheiten oder Messgrössen berechnet werden. Die Einheiten können gegebenenfalls über ein Anzeigeelement dargestellt werden, über eine Schnittstelle zum Informationsaustausch ausgegeben werden oder in einer Speichereinheit abgelegt werden.

Die Einheiten können mindestens ein Element aus der nachfolgenden Aufzählung umfassen:

- gesamte abgegebene Energie in einem bestimmten Zeitintervall
- Signalform und Modulationsart (AM/FM) während der Therapie
- Absetzen der Elektrode und Zeitintervall eines oder mehrerer Unterbrechungen
- Regelung eines internen Energiemanagements
- Detektion von fehlerhaften Elektroden

Es sind weitere Berechnungen denkbar, welche den Therapieverlauf pro Behandlungsobjekt dokumentieren und registrieren. Ferner sind weitere Berechnungen unter Einbezug von externen Daten, welche über die Schnittstelle eingelesen werden, denkbar, welche den Therapieerfolg erhöhen und belegen. Dazu kann die Prozessoreinheit jegliche Art von Berechnungen ausführen, typischerweise sind dies regelnde und/oder statistische Berechnungen.

Die Resultate der Berechnungen können wiederum in das Regelmodul der Prozessoreinheit eingespeist werden und den Signalverlauf und/oder die Modulation beeinflussen. Von einer früheren Therapiesitzung kann mittels Therapiedaten ein Lerneffekt für eine spätere Therapiesitzung generiert werden. Diese Therapiedaten können über die Schnittstelle zum Informationsaustausch anderen Therapievorrichtungen mittgeteilt werden und sorgen damit dafür, dass durch jede Verwendung der erfindungsgemässen Therapievorrichtung eine kontinuierliche Qualitätsverbesserung ermöglicht wird.

Die kapazitive Koppelung kann durch die Koppelung des Anwenders und des Behandlungsobjekts mit einer Leiterfläche am Gehäuse der Therapievorrichtung erfolgen. Hierzu ist es vorstellbar, dass das Gehäuse selbst elektrisch leitend ausgelegt ist, das heisst, zumindest eine elektrisch leitende oder leitfähige Fläche aufweist oder das Gehäuse über eine innenliegende leitende oder leitfähige Fläche verfügt. Die leitende oder leitfähige Fläche selbst kann dabei als Draht, als Fläche oder als dreidimensionales Objekt ausgebildet sein.

Die Energieversorgung der Energiespeichereinheit kann über ein Ladesystem erfolgen. Dieses Ladesystem umfasst in einer Ausführungsform einen Kontaktstecker und kann die benötigte Energie aus einer handelsüblichen Ladestation beziehen. In einer anderen möglichen Ausführungsform erfolgt die Ladung der Energiespeichereinheit über eine Induktionsschleife, welche im Gehäuse angebracht ist. Diese Induktionsschleife bildet eine Sekundärspule eines Übertragungstransformators. Mittels der in der Ladestation eingebauten Primärspule kann somit ein Energiefluss erfolgen, wenn sich die Therapievorrichtung im Wirkfeld der Ladestation befindet. Solche Ladesysteme sind unterdessen standardisiert in der Elektroindustrie verfügbar.

Die Erfindung wird anhand eines Ausführungsbeispiels, welches in den Zeichnungen dargestellt ist, näher erläutert. Es zeigen:

Fig. 1a: ein Schema eines ersten Ausführungsbeispiels für eine Therapievorrichtung,

Fig. 1b: ein Schema eines zweiten Ausführungsbeispiels einer Therapievorrichtung,

Fig. 1c: eine schematische Darstellung der Komponenten für das zweite Ausführungsbeispiel der Therapievorrichtung,

Fig. 2 ein vereinfachtes Schaltschema einer vorbekannten Therapievorrichtung,

Fig. 3 ein vereinfachtes Schaltschema der Therapievorrichtung gemäss Fig. 1a,

Fig. 4a: einen möglichen zeitlichen Spannungsverlauf der in Fig. 2 dargestellten vorbekannten Therapievorrichtung,

Fig. 4b: einen möglichen zeitlichen Frequenzverlauf der in Fig. 2 dargestellten vorbekannten Therapievorrichtung,

Fig. 5a: einen möglichen zeitlichen Spannungsverlauf der in Fig. 3 dargestellten Therapievorrichtung,

Fig. 5b: einen möglichen zeitlichen Frequenzverlauf der in Fig. 3 dargestellten Therapievorrichtung.

Fig. 6a: ein erstes Teilelement eines zweiteiligen Gehäuses für eine Therapievorrichtung gemäss Fig. 1b oder Fig. 1c,

Fig. 6b: eine Ansicht einer ersten Variante einer Elektrode für eine Therapievorrichtung gemäss Fig. 1b oder Fig. 1c,

Fig. 6c: ein zweites Teilelement eines zweiteiligen Gehäuses für eine Therapievorrichtung gemäss Fig. 1b oder Fig. 1c,

Fig. 6d: eine Ansicht einer zweiten Variante einer Elektrode,

Fig. 6e: eine Ansicht einer dritten Variante einer Elektrode,

Fig. 6f: eine Ansicht einer Therapievorrichtung gemäss Fig. 1b oder Fig. 1c,

Fig. 6g: einen Längsschnitt einer vierten Variante einer Elektrode,

Fig. 7: einen Teilschnitt durch das Gehäuse mit der darin angeordneten Elektrode einer Therapievorrichtung gemäss Fig. 1b oder Fig. 1c,

Fig. 8a: einen Radialschnitt durch das erste Teilelement des Gehäuses gemäss Fig. 6a,

Fig. 8b: einen Radialschnitt durch das zweite Teilelement des Gehäuses gemäss Fig. 6c,

Fig. 9: eine Messanordnung für die Messung eines Patientenableitstroms,

Fig. 10a ein Schaubild des Patientenableitstroms in Abhängigkeit vom Abstand einer ersten Therapievorrichtung von der Kathode,

Fig. 10b ein Schaubild des Patientenableitstroms in Abhängigkeit vom Abstand einer zweiten Therapievorrichtung von der Kathode,

Fig. 10c ein Schaubild des Patientenableitstroms in Abhängigkeit vom Abstand einer dritten Therapievorrichtung von der Kathode,

Fig. 10d den Verlauf der Temperatur in Abhängigkeit vom Abstand der Elektrode von der Kathode für die zweite Therapievorrichtung,

Fig. 11 eine Messanordnung für Ermittlung der Zusammensetzung eines mittels der Therapievorrichtung generierten Sekundärplasmas,

Fig. 12a ein Schaubild der Zusammensetzung des Sekundärplasmas der ersten Therapievorrichtung,

Fig. 12b ein Schaubild der Zusammensetzung des Sekundärplasmas der zweiten Therapievorrichtung,

Fig. 12c ein Schaubild der Zusammensetzung des Sekundärplasmas der zweiten Therapievorrichtung,

Fig. 13 ein Spektrum der dritten Therapievorrichtung im Vergleich mit der ersten Therapievorrichtung,

Fig. 14 eine Messanordnung zur Bestimmung der während der Entladung gebildeten reaktiven Spezies,

Fig. 15 ein exemplarisches, mittels FTIR Spektroskopie ermitteltes Spektrum der ersten Therapievorrichtung,

Fig. 16 eine Darstellung der gemessenen Stromstärke bei der Einstellung HI.

Fig. 17a den pH-Wert in Abhängigkeit von der Behandlungsdauer für eine erste Therapievorrichtung mit der Einstellung LO,

Fig. 17b den pH-Wert in Abhängigkeit von der Behandlungsdauer für die erste Therapievorrichtung mit der Einstellung HI,

Fig. 17c den pH-Wert in Abhängigkeit von der Behandlungsdauer für die zweite Therapievorrichtung in Wasser,

Fig. 17d den pH-Wert in Abhängigkeit von der Behandlungsdauer für die zweite Therapievorrichtung in NaCl,

Fig. 17e den pH-Wert in Abhängigkeit von der Behandlungsdauer für eine erste Elektrode der dritten Therapievorrichtung in Wasser,

Fig. 17f den pH-Wert in Abhängigkeit von der Behandlungsdauer für eine zweite Elektrode der dritten Therapievorrichtung in Wasser,

Fig. 18a die Konzentration an $H_2O_2$ in Abhängigkeit von der Behandlungsdauer für die erste Therapievorrichtung,

Fig. 18b die Konzentration an $H_2O_2$ in Wasser für die zweite Therapievorrichtung,

Fig. 18c die Konzentration an $H_2O_2$ in NaCl für die zweite Therapievorrichtung,

Fig. 18d die Konzentration an $H_2O_2$ in Wasser für die erste Elektrode der dritten Therapievorrichtung,

Fig. 18e die Konzentration an $H_2O_2$ in Wasser für die zweite Elektrode der dritten Therapievorrichtung,

Fig. 19 die Konzentrationen an $NO_2^-$ in $H_2O$ und NaCl in Abhängigkeit von der Behandlungsdauer für die erste Therapievorrichtung,

Fig. 20 Resultate eines MTT Tests zur Ermittlung der Cytotoxizität für die erste Therapievorrichtung,

Fig. 21 Resultate eines MTT Tests zur Ermittlung der Cytotoxizität für die zweite Therapievorrichtung,

Fig. 22a Resultate eines MTT Tests zur Ermittlung der Cytotoxizität für die erste Elektrode der dritten Therapievorrichtung,

Fig. 22b Resultate eines MTT Tests zur Ermittlung der Cytotoxizität für die zweite Elektrode der dritten Therapievorrichtung,

Fig. 23 eine Abbildung der Agarplatten für einen Hemmhoftest für die LSE Elektrode für das Bakterium Staphylococcus aureus,

Fig. 24 ein Balkendiagramm der Resultate des Hemmhoftests für das Bakterium Staphylococcus aureus für die LSE Elektrode,

Fig. 25 eine Abbildung der Agarplatten für einen Hemmhoftest für die EWC Elektrode der ersten Therapievorrichtung für das Bakterium Staphylococcus aureus,

Fig. 26 eine Abbildung der Agarplatten für einen Hemmhoftest für die EWC Elektrode der ersten Therapievorrichtung für das Bakterium Staphylococcus epidermidis,

Fig. 27 eine Abbildung der Agarplatten für einen Hemmhoftest für die EWC Elektrode der ersten Therapievorrichtung für das Bakterium Escherichia coli,

Fig. 28 eine Abbildung der Agarplatten für einen Hemmhoftest für die EWC Elektrode der ersten Therapievorrichtung für das Bakterium Pseudomonas aeruginosa,

Fig. 29 eine Abbildung der Agarplatten für einen Hemmhoftest für die EWC Elektrode der ersten Therapievorrichtung für die Hefe Candida albicans,

Fig. 30 ein Balkendiagramm der Resultate der Hemmhoftests für sämtliche Mikroorganismen für die EWC Elektrode der ersten Therapievorrichtung,

Fig. 31 eine Abbildung der Agarplatten für einen Hemmhoftest für die EWC Elektrode der zweiten Therapievorrichtung für das Bakterium Staphylococcus aureus,

Fig. 32 eine Abbildung der Agarplatten für einen Hemmhoftest für die EWC Elektrode der zweiten Therapievorrichtung für das Bakterium Staphylococcus epidermidis,

Fig. 33 eine Abbildung der Agarplatten für einen Hemmhoftest für die EWC Elektrode der zweiten Therapievorrichtung für das Bakterium Escherichia coli,

Fig. 34 eine Abbildung der Agarplatten für einen Hemmhoftest für die EWC Elektrode der zweiten Therapievorrichtung für das Bakterium Pseudomonas aeruginosa,

Fig. 35 eine Abbildung der Agarplatten für einen Hemmhoftest für die EWC Elektrode der zweiten Therapievorrichtung für die Hefe Candida albicans,

Fig. 36 ein Balkendiagramm der Resultate der Hemmhoftests für sämtliche Mikroorganismen für die EWC Elektrode der zweiten Therapievorrichtung,

Fig. 37 eine Abbildung der Agarplatten für einen Hemmhoftest für die dritte Therapievorrichtung für das Bakterium Staphylococcus aureus,

Fig. 38 eine Abbildung der Agarplatten für einen Hemmhoftest für die dritte Therapievorrichtung für das Bakterium Staphylococcus epidermidis,

Fig. 39 eine Abbildung der Agarplatten für einen Hemmhoftest für die dritte Therapievorrichtung für das Bakterium Escherichia coli,

Fig. 40 eine Abbildung der Agarplatten für einen Hemmhoftest für die dritte Therapievorrichtung für das Bakterium Pseudomonas aeruginosa,

Fig. 41 eine Abbildung der Agarplatten für einen Hemmhoftest für die dritte Therapievorrichtung für die Hefe Candida albicans,

Fig. 42 ein Balkendiagramm der Resultate der Hemmhoftests für sämtliche Mikroorganismen für die dritte Therapievorrichtung.

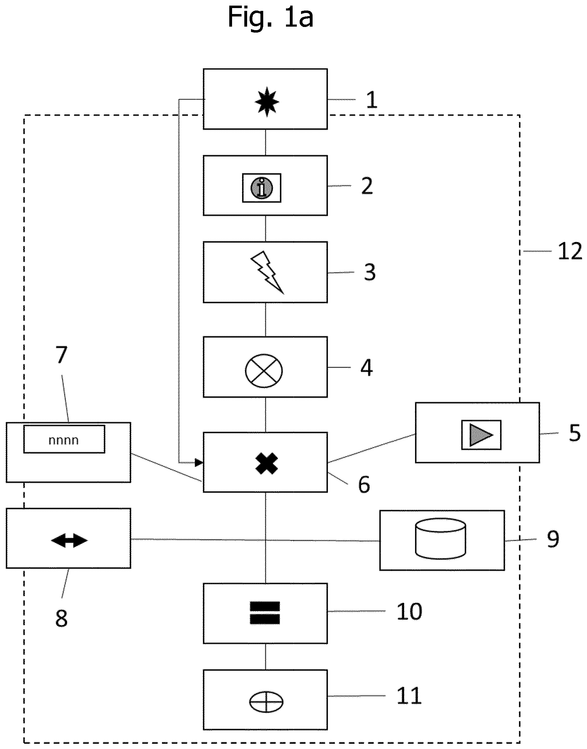

Fig. 1a zeigt schematisch erstes Ausführungsbeispiel einer Therapievorrichtung zur Zelltherapie, umfassend eine Elektrode 1, einen Generator 3 zur Erzeugung von hochfrequenten Impulsen, eine Prozessoreinheit 6 umfassend ein Steuer-, Regel- und Berechnungsmodul, ein Speicherelement 9 und Bedienelemente 5.

Die Therapievorrichtung kann mindestens eine Schnittstelle 8 zum Informationsaustausch umfassen.

Die Therapievorrichtung umfasst ferner eine Speichereinheit 9 sowie einen steuerbaren Modulator 4, welcher den Generator 3 steuert. Die Energieversorgung zum Betrieb der Therapievorrichtung erfolgt mittels einer internen Energiespeichereinheit 10.

Das Gehäuse 12 kann mindestens ein Anzeigeelement 7 enthalten, mittels welchem insbesondere Therapie- und Betriebsdaten darstellbar sind.

Zudem umfasst die Therapievorrichtung einen Sensor 2, welcher die über die Elektrode 1 abgegebene Spannung und/oder den Strom misst, wobei aus der gemessenen Spannung oder dem gemessenen Strom die über die Elektrode 1 abgegebene Energie ermittelbar ist. Mittels des Sensors werden somit der über die Elektrode 1 abgegebene Strom und/oder die Spannung als Messwerte erfasst. Die Messwerte werden digitalisiert und als Messdaten in der Speichereinheit 9 gespeichert. Aus den Messdaten ist mittels des Berechnungsmoduls der Prozessoreinheit 6 die abgegebene Energie ermittelbar. Aus den in der Speichereinheit 9 mit einem Zeitstempel gespeicherten Messdaten für Strom und/oder Spannung ist mittels des Berechnungsmoduls der Prozessoreinheit 6 der zeitliche Verlauf der von der Elektrode 1 abgegebenen Energie ermittelbar. Die ermittelten Spannungen und/oder Ströme können alternativ auch von einem Aufzeichnungsgerät aufgezeichnet werden und in Messdaten umgewandelt werden, sodass aus den aufgezeichneten Messdaten für die Spannung sowie den Strom der zeitliche Verlauf der von der Elektrode 1 abgegebenen Energie ermittelt werden kann.

Sämtliche Bauelemente der Therapievorrichtung sind in einem Gehäuse 12 angeordnet.

Insbesondere können das Gehäuse 12 und/oder der Generator 3 so ausgebildet sein, dass das Gehäuse oder der Generator einen Pol eines Kondensators ausbilden, welcher eine kapazitive Koppelung ermöglicht.

Gemäss eines Ausführungsbeispiels sind die Therapie- und Betriebsdaten aus der Speichereinheit 9 schreib- und lesbar ausgestaltet. Die in der Speichereinheit 9 abgelegten Daten können dazu dienen, den Therapieverlauf zu beeinflussen.

Gemäss eines Ausführungsbeispiels können die Therapie- und Betriebsdaten unter Berücksichtigung der vom Sensor 2 gemessenen Messwerte, die als Daten in der Speichereinheit gespeichert sein können, in der Prozessoreinheit 6 zu Steuerungsanweisungen berechnet und aufbereitet werden. Die Steuerungsanweisungen können zur Steuerung des Modulators 4 dienen.

Die Steuerung des Modulators 4 kann insbesondere derart erfolgen, dass die Energieabgabe konstant erfolgt und unabhängig von der Signalform ist.

Insbesondere kann die Steuerung des Modulators 4 derart erfolgen, dass eine beliebige Signalform im Generator 3 generiert wird, typischerweise kann eine Kombination von Amplituden- und Frequenzmodulation erfolgen.

Gemäss eines Ausführungsbeispiels enthält das Gehäuse 12 eine elektrisch leitfähige oder leitende Fläche, beispielsweise einen leitenden Kunststoff oder einen mit einem elektrisch leitfähigen Material beschichteten Kunststoff.

Die Energiespeichereinheit 10 kann gemäss eines Ausführungsbeispiels als wieder aufladbares Element ausgelegt sein, typischerweise als Lithium-Ionen Element oder als Superkapazität.

Die über die Elektrode 1 abgegebene Energie kann zur Zellstimulation oder Zelltherapie dienen.

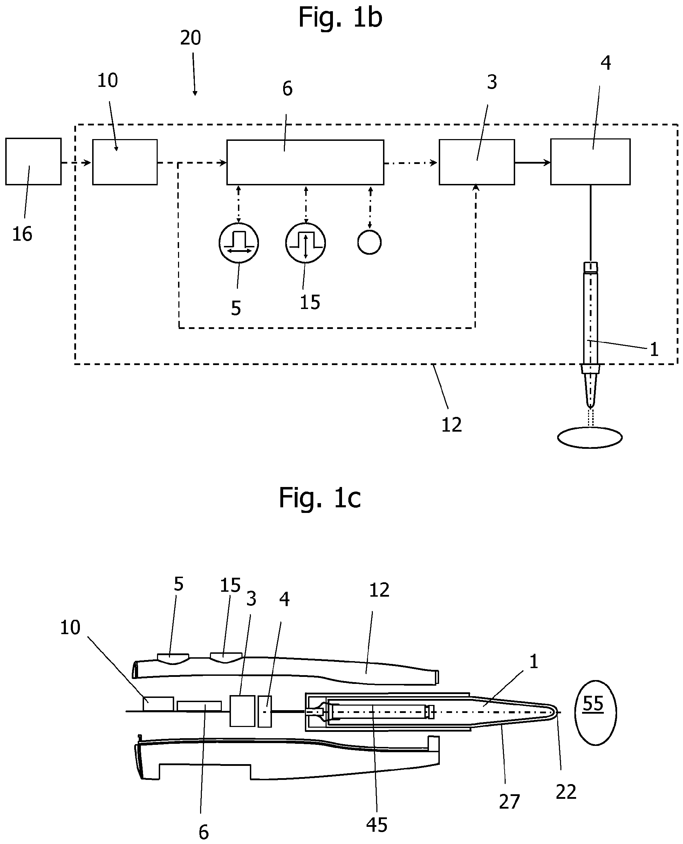

Fig. 1b zeigt ein zweites Ausführungsbeispiel einer Therapievorrichtung 20, wobei für gleiche oder gleichwirkende Bauelemente dieselben Bezugszeichen verwendet worden sind wie in Fig. 1a.

Die Therapievorrichtung 20 umfasst eine Elektrode 1, einen Generator 3 zur Erzeugung von hochfrequenten Impulsen, eine Prozessoreinheit 6 umfassend ein Steuer-, Regel- und Berechnungsmodul und Bedienelemente 5, 15 sowie eine Energiespeichereinheit 10. Die Elektrode 1, der Generator 3, die Prozessoreinheit 6 sowie die Bedienelemente 5, 15 und die Energiespeichereinheit 10 sind in zusammengebautem Zustand in einem gemeinsamen Gehäuse 12 untergebracht, was in Fig. 1b schematisch als Systemgrenze dargestellt ist.

Die Energieversorgung zum Betrieb der Therapievorrichtung 20 erfolgt mittels einer Energiespeichereinheit 10, die ebenfalls im Gehäuse 12 angebracht ist. Die Energiespeichereinheit 10 kann insbesondere einen wiederaufladbaren Akku enthalten. Die Energiespeichereinheit 10 kann gemäss eines Ausführungsbeispiels einen Lithium-Ionen-Akku oder eine Superkapazität enthalten. Die Energiespeichereinheit 10 kann mittels eines Ladegeräts 16 aufgeladen werden, welches dem Fachmann bekannt ist und daher in dieser Darstellung nicht näher spezifiziert ist.

Gemäss des vorliegenden Ausführungsbeispiels sind die Bedienelemente 5, 15 drehbar im Gehäuse 12 angeordnet, was in der schematischen Darstellung gemäss Fig. 1c sichtbar ist. Mittels des Bedienelements 5 kann die Dauer jedes Impulses eingestellt werden. Mittels des Bedienelements 15 kann die Höhe des Impulses, d.h. dessen Amplitude, eingestellt werden. Gemäss des vorliegenden Ausführungsbeispiels ist auf jedem der Bedienelemente 5, 15 eine Skala angebracht, mittels welcher die eingestellten Therapie- und Betriebsdaten angezeigt werden können. Zudem kann das Gehäuse 12 ein optisches Anzeigeelement enthalten, beispielsweise eine LED-Leuchte.

Zudem umfasst die Therapievorrichtung einen Sensor 2, welcher die über die Elektrode 1 abgegebene Spannung und/oder den Strom misst, wobei aus der gemessenen Spannung oder dem gemessenen Strom die über die Elektrode 1 abgegebene Energie ermittelbar ist. Mittels des Sensors werden somit der über die Elektrode 1 abgegebene Strom und/oder die Spannung als Messwerte erfasst. Die Messwerte werden digitalisiert und als Messdaten in der Speichereinheit 9 gespeichert. Aus den Messdaten ist mittels des Berechnungsmoduls der Prozessoreinheit 6 die abgegebene Energie ermittelbar. Aus den in der Speichereinheit 9 mit einem Zeitstempel gespeicherten Messdaten für Strom und/oder Spannung ist mittels des Berechnungsmoduls der Prozessoreinheit 6 der zeitliche Verlauf der von der Elektrode 1 abgegebenen Energie ermittelbar. Die ermittelten Spannungen und/oder Ströme können alternativ auch von einem Aufzeichnungsgerät aufgezeichnet werden und in Messdaten umgewandelt werden, sodass aus den aufgezeichneten Messdaten für die Spannung sowie den Strom der zeitliche Verlauf der von der Elektrode 1 abgegebenen Energie ermittelt werden kann.

Gemäss eines Ausführungsbeispiels können die Therapie- und Betriebsdaten unter Berücksichtigung der vom Sensor 2 gemessenen Messwerte, die als Daten in der Speichereinheit gespeichert sein können, in der Prozessoreinheit 6 zu Steuerungsanweisungen berechnet und aufbereitet werden. Die Steuerungsanweisungen können zur Steuerung des Modulators 4 dienen.

Die Steuerung des Modulators 4 kann insbesondere derart erfolgen, dass die Energieabgabe konstant erfolgt und unabhängig von der Signalform ist. Insbesondere kann die Steuerung des Modulators 4 derart erfolgen, dass eine beliebige Signalform im Modulator 4 generiert wird, typischerweise kann eine Kombination von Amplituden- und Frequenzmodulation erfolgen. Der Modulator 4 kann gemäss eines Ausführungsbeispiels als Transformator ausgebildet sein. Dieser Transformator dient zur Erzeugung der für die Ionisierung des Gases in der Elektrode erforderlichen elektrischen Spannung. Die Modulatorspannung 4 kann im Bereich von 8V bis 65 V liegen.

Die Eingangsspannung des Generators 3 kann im Bereich von 8 V bis 65 V liegen. Die Ausgangsspannung des Modulators 4 wird mittels des Generators 3 beispielsweise auf eine elektrische Spannung im Bereich von 5 kV bis einschliesslich 25 kV transformiert. Der Generator enthält somit einen Hochspannungstransformator. Gemäss eines Ausführungsbeispiels ist der Hochspannungstransformator als Tesla-Spule ausgebildet. Der Hochspannungstransformator umfasst eine Primärwicklung zur Aufnahme der vom Modulator zugeführten Leistung. An der Primärwicklung liegt somit eine Primärwicklungsspannung an, beispielsweise im Bereich von 8V bis 65 V. Der Hochspannungstransformator enthält eine Sekundärwicklung, an welcher eine Sekundärwicklungsspannung erhältlich ist. Diese Sekundärwicklungsspannung ist grösser als die Primärwicklungsspannung. Die Sekundärwicklung des als Tesla-Spule ausgebildeten Hochspannungstransformators ist konzentrisch zur Primärwicklung angeordnet, was eine besonders platzsparende Ausführung des Hochspannungstransformators ermöglicht. Die Sekundärwicklungsspannung kann mindestens 100x so gross sein wie die Primärwicklungsspannung. Insbesondere kann die Sekundärwicklungsspannung 300x bis einschliesslich 1000x so gross sein wie die Primärwicklungsspannung. Wenn die Primärwicklungsspannung 65 V beträgt, beträgt gemäss eines Ausführungsbeispiels die Sekundärwicklungsspannung 25 kV. Die Sekundärwicklungsspannung ist gemäss dieses Ausführungsbeispiels 385x höher als die Primärwicklungsspannung.

Die vom Modulator 4 erzeugten Spannungspulse werden dem Generator 3 somit auf Hochspannungspulse transformiert und der Elektrode 1 zugeführt, die eine Anode 45 enthält. Die Anode 45 befindet sich im Innenraum eines Glaskörpers 27. Die Anode enthält ein Material, aus welchem bei Anlegen einer Hochspannung elektrische Ladungsträger, insbesondere Elektronen und Ionen herausgelöst werden können. Diese elektrischen Ladungsträger gelangen in den mit einem Gas gefüllten Glaskörper 27. Die positiv geladenen elektrischen Ladungsträger bewegen sich in Richtung der Kathode 55. Die Kathode 55 wird gemäss dieses Ausführungsbeispiels durch die behandelnde Oberfläche gebildet, die schematisch dargestellt ist. Die negativ geladenen elektrischen Ladungsträger bewegen sich Richtung Anode. Wenn die negativ geladenen Ladungsträger hierbei genügend beschleunigt werden, können sie beim Auftreffen auf die Anode weitere Ladungsträger freisetzen, die in den Gasinnenraum gelangen. Beim Zusammentreffen der Elektronen mit den Gasmolekülen werden Ionen erzeugt, die als positive Ladungsträger sich in Richtung der Kathode bewegen. Wenn die angelegte Spannung im Bereich von 5 bis einschliesslich 25 kV liegt, nimmt die Anzahl der im Gas befindlichen Ladungsträger lawinenartig zu, sodass es zu einer Ionisierung des Gases und damit zur Ausbildung eines Plasmas kommt. Es handelt sich in diesem Fall um ein sogenanntes Kaltplasma, da die Elektronen nicht aufgrund thermischer Emission entstehen, sondern als Sekundärelektronen als Folge des Kontakts von Ladungsträgern mit dem Anodenmaterial entstehen.

Die Kathode 55 befindet sich erfindungsgemäss ausserhalb des Glaskörpers 27, daher wirkt das in der Elektrode 1 aufgebaute elektrische Feld auch auf Ladungsträger in der Luft, beispielsweise Sauerstoff. Das zweite Ende 22 der Elektrode 1 hat die Wirkung einer dielektrischen Barriere. Insbesondere können Sauerstoffmoleküle durch das wirkende elektrische Feld ionisiert werden, wodurch ein sogenanntes Sekundärplasma ausgebildet wird. Insbesondere wenn die Kathode sich in einem Abstand von bis zu 2 mm vom zweiten Ende 22 der Elektrode 1 befindet, kann eine dielektrisch behinderte Entladung im Luftraum gezündet werden.

Die Therapievorrichtung 20 entspricht in ihrer Wirkungsweise einem Kondensator, dessen erster Pol durch das Gehäuse 12 und dessen zweiter Pol durch die behandelte Körperstelle ausgebildet ist. Der erste Pol wird durch die Elektrode 1 ausgebildet, welche die Anode 45 enthält. Der zweite Pol wird durch die Kathode 55 gebildet. Gemäss des vorliegenden Ausführungsbeispiels bildet das Gehäuse 12, welches die Elektrode 1 enthält, einen der Pole des Kondensators aus. Das Gehäuse wird von der Person, welche das Gehäuse 12 während der Therapie in der Hand hält, mit dem Gegenpol dieses Kondensators, der zu behandelnden Körperstelle des Patienten, in Kontakt gebracht oder zumindest derart angenähert, dass es zur Ausbildung eines Sekundärplasmas kommen kann.

Fig. 1c zeigt eine Explosionszeichnung eines Gehäuses 12, in welchem die Elektrode 1, der Generator 3, der Modulator 4, die Prozessoreinheit 5 sowie die Energiespeichereinheit 10 angeordnet sind. Das Gehäuse 12 enthält die vorgängig beschriebenen Bedienelemente 5, 15.

Gemäss eines Ausführungsbeispiels enthält das Gehäuse 12 eine elektrisch leitfähige oder leitende Oberfläche, beispielsweise einen leitenden Kunststoff oder einen mit einem elektrisch leitfähigen Material beschichteten Kunststoff.

Die über die Elektrode 1 abgegebene Energie kann zur Zellstimulation oder Zelltherapie dienen.

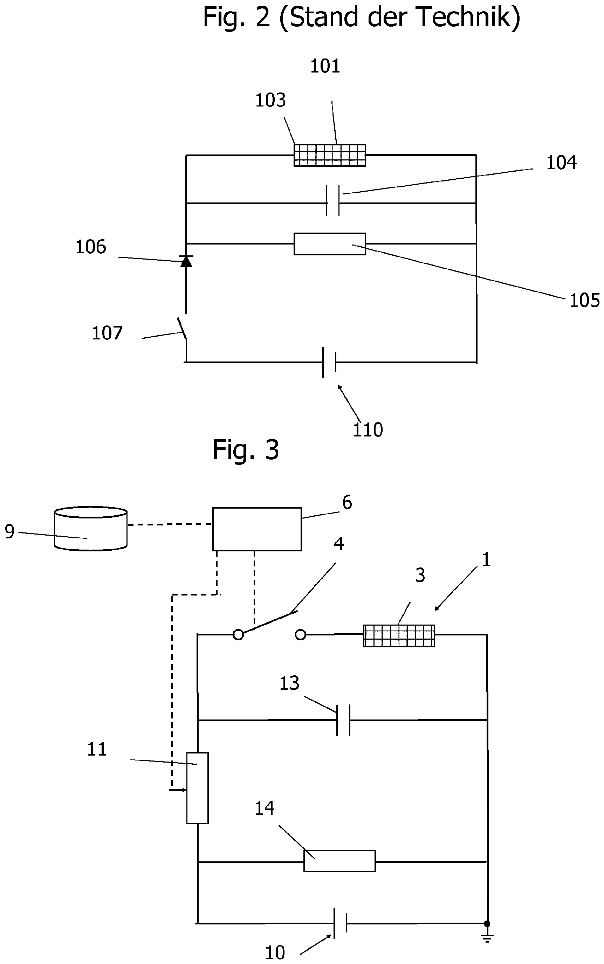

Fig. 2 zeigt ein vereinfachtes Schaubild der Therapievorrichtung gemäss EP 2397187 A1. Diese vorbekannte Therapievorrichtung wird von einer Gleichstromquelle 110, beispielsweise einer Batterie, gespeist. Ein Strom fliesst vom Kondensator 104 zur Spule 103, welche ein elektromagnetisches Feld induziert, wenn mittels des Schalters 107 die Verbindung zur Gleichstromquelle 110 unterbrochen ist. Die Spule ist gemäss dieses Ausführungsbeispiels das potentialerzeugende Element für eine Elektrode 101. Durch die Spule wird eine Potentialdifferenz erzeugt. Bedingt durch die Potentialdifferenz liegt eine elektrische Spannung an der Elektrode 101 an. Diese elektrische Spannung wird mittels der Elektrode 101, die in Kontakt mit dem Patienten ist, auf den Patienten übertragen. Eine Diode 106 verhindert den Rückfluss eines Stroms in den Batteriekreislauf. Wird der Schalter 107 geschlossen, kann der Kondensator 104 wiederum von der Batterie 110 aufgeladen werden. Da die Spule 103 in diesem Kreislauf als elektrischer Widerstand wirkt, wird der Kondensator 104 aufgeladen, an der Elektrode 101 liegt keine Potentialdifferenz an. Durch das periodische Öffnen und Schliessen des Schalters 107 kann die Potentialdifferenz an der Elektrode 101 zwischen dem Wert Null und dem von der eingebauten Spule 103 erzeugbaren Maximalwert schwanken, sodass ein gepulster Betrieb ermöglicht ist. Demzufolge wird eine gepulste elektrische Spannung erzeugt, die für therapeutische Zwecke nutzbar gemacht werden kann. Die Richtung des zum Kondensator fliessenden Stroms bei geöffnetem Schalter 107 ist umgekehrt zur Flussrichtung bei geschlossenem Schalter 107. Eine Diode 106 verhindert den Rückfluss des Stroms in den Batteriekreislauf, wenn der Schalter 107 geschlossen ist. Die Frequenz der gepulsten elektrischen Spannung wird durch somit durch die Schaltfrequenz des Schalters 107 bestimmt. Mit dieser Therapievorrichtung ist somit eine gepulste Spannung variabler Frequenz erzeugbar, allerdings ist die Amplitude der Spannung durch die verwendete Spule 103 vorgegeben. Bei dem zum Kondensator 104 parallel geschalteten Ableitwiderstand 105 handelt es sich um ein Bauelement zur Verhinderung von Stromschlägen bei Berührung der Therapievorrichtung, nachdem die Gleichstromquelle abgeschaltet worden ist. Der Kondensator 104 kann über den Ableitwiderstand 105 entladen werden.

Fig. 3 zeigt ein Schaltschema der erfindungsgemässen Therapievorrichtung in vereinfachter Form. Die Therapievorrichtung umfasst einen Generator 3, der als Spule ausgebildet ist, mittels welcher eine gepulste Spannung erzeugbar ist, die mittels der Elektrode 1 auf den zu behandelnden Körperteil eines Patienten gerichtet wird. Mittels des Modulators 4, der gemäss des vorliegenden Ausführungsbeispiels als Schalter ausgebildet ist, kann die Pulsfrequenz und die Pulsdauer eingestellt werden. Wenn der Schalter geschlossen ist, kann sich der Kondensator 13 über die Spule entladen. Das heisst, an der Spule liegt eine elektrische Spannung an, sodass ein elektromagnetisches Feld entsteht, welches den gewünschten therapeutischen Effekt auf die in seinem Bereich befindlichen Körperzellen erzeugt. Die Stromstärke kann mittels des Potentiometers 11 eingestellt werden. Hierdurch kann die Amplitude der an der Spule anliegenden Spannung verändert werden.

Wenn sich der Schalter, wie in der vorliegenden Darstellung gezeigt, in der geöffneten Stellung befindet, kann kein Strom durch die Spule fliessen, das heisst, die Spule erzeugt kein elektromagnetisches Feld. Der Kondensator 14 kann von der Energiespeichereinheit 10, das heisst, gemäss dieses Ausführungsbeispiels der Gleichstromquelle, aufgeladen werden. Bei dem zum Kondensator 13 parallel geschalteten Ableitwiderstand 14 handelt es sich wie im Stand der Technik um ein Bauelement zur Verhinderung von Stromschlägen bei Berührung der Therapievorrichtung, nachdem die Gleichstromquelle abgeschaltet worden ist. Der Kondensator 13 kann über den Ableitwiderstand 14 sicher entladen werden.

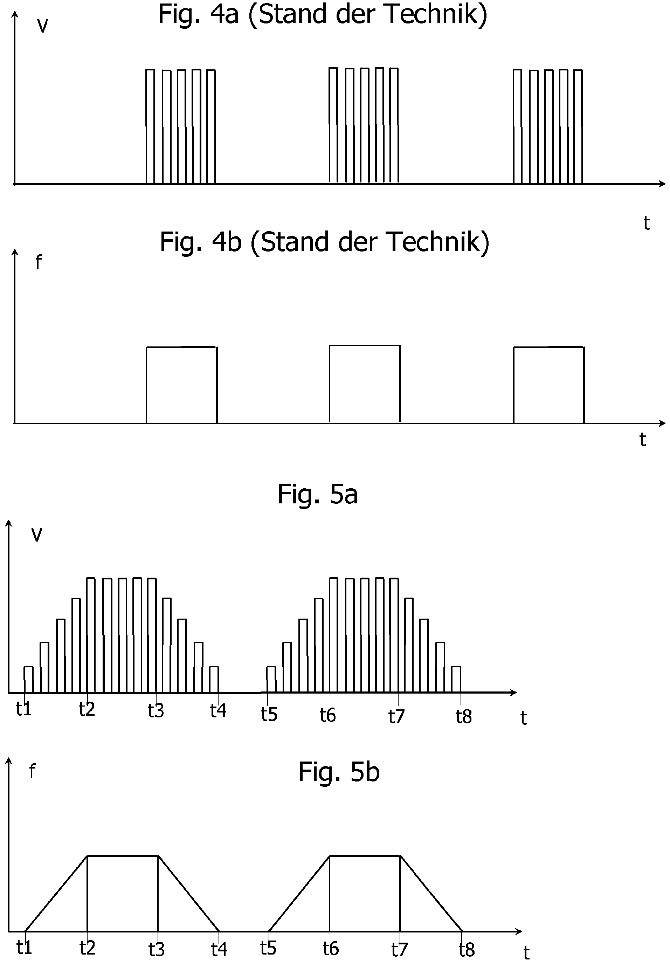

Fig. 4a zeigt einen möglichen zeitlichen Spannungsverlauf der in Fig. 2 dargestellten vorbekannten Therapievorrichtung. Die graphische Darstellung in Fig. 4a zeigt einen zeitlichen Verlauf der elektrischen Spannung, das heisst auf der Ordinate ist die elektrische Spannung in Volt eingetragen, auf der Abszisse die Zeit. Mittels der vorbekannten Therapievorrichtung können Spannungspulse erzeugt, werden, indem der Schalter 107 für eine kurze Zeitpanne geöffnet und dann wieder geschlossen wird, sodass die Spule mit Strom versorgt wird, solange der Schalter geschlossen ist, aber die Stromzufuhr unterbrochen wird, solange der Schalter geöffnet ist. Beispielsweise kann der Schalter für ca. 1 ms geschlossen werden, dann für 1ms geöffnet werden. Solange der Schalter geschlossen ist, wird durch den durch die Spule fliessenden Strom eine Spannung aufgebaut. Solange der Schalter geöffnet ist, wird keine Spannung erzeugt. Das heisst, die Zeitdauer, während derer der Schalter geschlossen ist, entspricht einem Spannungspuls. Wird der Schalter mehrmals geöffnet und wieder geschlossen, kann eine Mehrzahl von Spannungspulsen erzeugt werden, was in Fig. 4a beispielhaft für fünf Spannungspulse dargestellt ist. Danach kann der Schalter für eine längere Zeitdauer geöffnet werden. Während dieser Zeitspanne wird keine Spannung erzeugt, da kein Strom durch die Spule fliessen kann. Diese Zeitspanne kann beliebig lang sein. Falls es die Behandlung erfordert, kann eine weitere Folge von Spannungspulsen erzeugt werden, indem der Schalter wieder mehrmals für eine kurze Zeitdauer geöffnet und wieder geschlossen wird.

Fig. 4b zeigt einen möglichen zeitlichen Frequenzverlauf der in Fig. 2 dargestellten vorbekannten Therapievorrichtung. Auf der Ordinate ist die Frequenz in Hertz eingetragen, auf der Abszisse die Zeit. Jede Folge von Spannungspulsen in Fig. 4a entspricht einer Frequenz grösser Null, die in Fig. 4b als Säule dargestellt ist. Solange der Schalter 107 geöffnet ist, wird keine Spannung erzeugt, da kein Strom durch die Spule fliesst. Daher beträgt die Frequenz während dieser Zeitspanne null Hertz.

Fig. 5a zeigt einen möglichen zeitlichen Spannungsverlauf der in Fig. 3 dargestellten Therapievorrichtung gemäss eines Ausführungsbeispiels der Erfindung. Die graphische Darstellung in Fig. 5a zeigt einen zeitlichen Verlauf der elektrischen Spannung, das heisst auf der Ordinate ist die elektrische Spannung in Volt eingetragen, auf der Abszisse die Zeit. Mittels der Therapievorrichtung können Spannungspulse erzeugt, werden, indem Modulator 4 betätigt wird, beispielsweise der Schalter, für eine kurze Zeitpanne geöffnet und dann wieder geschlossen wird, sodass die Spule mit Strom versorgt wird, solange der Schalter geschlossen ist, aber die Stromzufuhr unterbrochen wird, solange der Schalter geöffnet ist. Beispielsweise kann der Schalter für ca. 1 ms geschlossen werden, dann für 1 ms geöffnet werden. Der Schalter kann auch für 0.1 s geöffnet und für 0.1 s geschlossen werden. Der Bereich, in welchem die Öffnungszeit variieren kann, kann insbesondere 0.001 s bis 0.1 s betragen. Der Bereich, in welchem die Schliesszeit variieren kann, kann insbesondere 0.001 s bis 0.1 s betragen. Solange der Schalter geschlossen ist, wird durch den durch die Spule fliessenden Strom eine Spannung aufgebaut. Solange der Schalter geöffnet ist, wird keine Spannung erzeugt. Das heisst, die Zeitdauer, während derer der Schalter geschlossen ist, entspricht einem Spannungspuls. Wird der Schalter mehrmals geöffnet und wieder geschlossen, kann eine Mehrzahl von Spannungspulsen erzeugt werden, was in Fig. 5a beispielhaft für 13 Spannungspulse dargestellt ist, die eine Spannungspulsfolge ausbilden. Danach kann der Schalter für eine längere Zeitspanne geöffnet werden. Während dieser Zeitspanne wird keine Spannung erzeugt, da kein Strom durch die Spule fliessen kann. Diese Zeitspanne kann beliebig lang sein. Beispielsweise kann die Zeitspanne im Bereich von 0.1 s bis 10 s liegen. Die Zeitspanne kann insbesondere im Bereich von 0.1 s bis 1 s liegen. Gemäss eines Ausführungsbeispiels kann die Zeitspanne 0.1 s betragen. Falls es die Behandlung erfordert, kann mindestens eine weitere Spannungspulsfolge erzeugt werden, indem der Schalter wieder mehrmals für eine kurze Zeitdauer geöffnet und wieder geschlossen wird. In Fig. 5a sind beispielhaft zwei Spannungspulsfolgen dargestellt.

Für jede der in Fig. 5a dargestellten Spannungspulsfolgen von n Spannungspulsen nimmt die Spannung eines jeden Spannungspulses während einer Zeitspanne t2-t1 bzw. t6-t5 zu, die Spannung jedes Spannungspulses bleibt während einer Zeitspanne t3-t2 bzw. t7-t6 konstant und die Spannung jedes Spannungspulses nimmt während einer Zeitspanne t4-t3 bzw. t8-t7 ab. Die mittlere Pulsdauer tm von n Spannungspulsen entspricht (t4-t1)/2n, wenn die Zeitspanne, innerhalb welcher der Schalter eingeschaltet ist, der Zeitspanne entspricht, innerhalb welcher der Schalter ausgeschaltet ist.

Wenn die Zeitdauer ts jedes der n Spannungspulse sich von der Zeitdauer tp jeder der Pausenzeiten zwischen den Spannungspulsen unterscheidet, kann die mittlere Pulsdauer tm von n Spannungspulsen und m Pausenzeiten wie folgt ermittelt werden. Die Dauer der Spannungspulsfolge D entspricht der Summe über alle tsi und der Summe über alle tpi. Mit tsi werden die i-ten Zeitspannen jedes der Spannungspulse 1 bis n bezeichnet. Mit tsi werden die i-ten Zeitspannen jeder der Pausenzeiten von 1 bis m bezeichnet. Der i-te Spannungspuls erstreckt sich beispielsweise über eine Zeitspanne tsi, der (i+1)te Spannungspuls über eine Zeitspanne ts(i+1). Die i-te Pausenzeit hat beispielsweise eine Zeitspanne tpi, die (i+1)te Pausenzeit eine Zeitspanne tp(i+1). Um die mittlere Pulsdauer tm zu erhalten, wird die Dauer der Spannungspulsfolge D durch die Anzahl (n+m) der Spannungspulse und der Pausenzeiten dividiert, wobei n der Anzahl der Spannungspulse und m der Anzahl der Pausenzeiten einer Folge von Spannungspulsen entspricht.

Gemäss des vorliegenden Ausführungsbeispiels variiert daher die Amplitude der Spannungspulse. Gemäss Fig. 5a steigt die Amplitude der Spannungspulse innerhalb der Zeitspanne t2-t1 an. Die Amplitude der Spannungspulse bleibt während der Zeitspanne t3-t2 konstant. Die Amplitude der Spannungspulse nimmt während der Zeitspanne t4-t3 ab.

Fig. 5b zeigt einen möglichen zeitlichen Frequenzverlauf der in Fig. 3 dargestellten Therapievorrichtung gemäss eines Ausführungsbeispiels der Erfindung. Auf der Ordinate ist die Frequenz in Hertz eingetragen, auf der Abszisse die Zeit. Jede Folge von Spannungspulsen in Fig. 5a entspricht einer Frequenz grösser Null, die in Fig. 5b als Trapezkörper dargestellt ist. Solange der Schalter geöffnet ist, wird keine Spannung erzeugt, da kein Strom durch die Spule fliesst. Daher beträgt die Frequenz während dieser Zeitspanne zwischen zwei benachbarten Spannungspulsfolgen null Hertz. Gemäss Fig. 5b steigt die Frequenz der Spannungspulse der ersten Spannungspulsfolge innerhalb der Zeitspanne t2-t1 an. Die Frequenz der Spannungspulse bleibt während der Zeitspanne t3-t2 konstant. Die Frequenz der Spannungspulse nimmt während der Zeitspanne t4-t3 ab. Auch die Frequenz der Spannungspulse der zweiten Spannungspulsfolge steigt innerhalb der Zeitspanne t6-t5 an. Die Frequenz der Spannungspulse bleibt während der Zeitspanne t7-t6 konstant. Die Frequenz der Spannungspulse nimmt während der Zeitspanne t8-t7 ab.

Die Darstellung gemäss Fig. 5a korreliert diesbezüglich nicht mit der Darstellung gemäss Fig. 5b. Gemäss Fig. 5a bleibt die Zeitdauer der Spannungspulse konstant, daher wäre die entsprechende Frequenz in einer der Fig. 5b entsprechenden graphischen Darstellung konstant.

Gemäss der in Fig. 5b dargestellten Variante nimmt somit die Zeitspanne tsi innerhalb der Zeitspanne t2-t1 ab, die Zeitspanne tsi ist innerhalb der Zeitspanne t3-t2 konstant. Die Zeitspanne tsi nimmt innerhalb der Zeitspanne t4-t3 zu.

Für die in Fig. 5b dargestellte zweite Spannungspulsfolge nimmt somit die Zeitspanne tsi innerhalb der Zeitspanne t6-t5 ab, die Zeitspanne tsi ist innerhalb der Zeitspanne t7-t6 konstant. Die Zeitspanne tsi nimmt innerhalb der Zeitspanne t8-t7 zu.

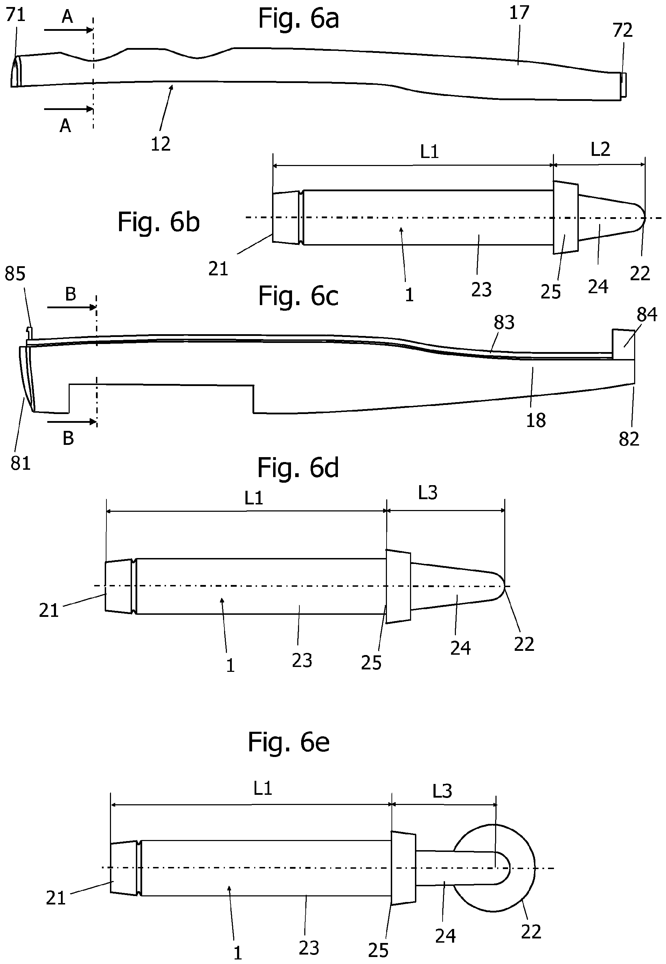

Fig. 6a zeigt ein erstes Teileelement 17 eines zweiteiligen Gehäuses 12 einer Therapievorrichtung 20.

Fig. 6b zeigt ein Ausführungsbeispiel einer ersten Variante für eine im Gehäuse 12 befindliche Elektrode 1. Die Elektrode 1 kann somit aus dem Gehäuse 12 entfernt werden und bei Bedarf durch eine andere Elektrode ersetzt werden. Die Elektrode 1 umfasst einen ersten Teilabschnitt 23 und einen zweiten Teilabschnitt 24, wobei der erste Teilabschnitt 23 eine Länge L1 aufweist und der zweite Teilabschnitt 24 eine zweite Länge L2 aufweist. Der erste Teilabschnitt 23 erstreckt sich von einem ersten Ende 21 bis zu einem Anschlagelement 25. Der zweite Teilabschnitt 24 erstreckt sich vom Anschlagelement 25 bis zu einem zweiten Ende 22 der Elektrode 1.

Fig. 6c zeigt ein zweites Teileelement 18 des zweiteiligen Gehäuses 12.

Fig. 6d zeigt ein Ausführungsbeispiel für eine zweite Variante einer Elektrode 1. Gemäss der in Fig. 6d dargestellten Variante ist die Länge L3 des zweiten Teilabschnitts 24 grösser als die Länge L2 des entsprechenden zweiten Teilabschnitts 24 gemäss Fig. 6b. Die Länge L1 des ersten Teilabschnitts entspricht der Länge L1 gemäss Fig. 6b, da die Elektrode 1 gemäss der zweiten Variante anstelle der Elektrode 1 gemäss der ersten Variante in das Gehäuse 12 eingebaut werden kann.

Fig. 6e zeigt ein Ausführungsbeispiel für eine dritte Variante einer Elektrode 1. Gemäss der in Fig. 6d dargestellten Variante ist die Länge L4 des zweiten Teilabschnitts 24 grösser als die Länge L2 des entsprechenden zweiten Teilabschnitts 24 gemäss Fig. 6b aber kleiner als die Länge L3 des zweiten Teilabschnitts 24 der Elektrode gemäss der zweiten Variante. Auch dieses Ausführungsbeispiel zeigt nur ein exemplarisches Ausführungsbeispiel. Selbstverständlich kann die Länge L4 von der vorliegenden Darstellung abweichen. Die Länge L1 des ersten Teilabschnitts entspricht der Länge L1 gemäss Fig. 6b, da die Elektrode 1 gemäss der dritten Variante anstelle der Elektrode 1 gemäss der ersten Variante in das Gehäuse 12 eingebaut werden kann. Das zweite Ende 22 dieser Elektrode 1 ist nicht als abgerundete Spitze ausgebildet, wie in den vorhergehenden Ausführungsbeispielen gezeigt, sondern enthält ein Ende, welches flanschartig ausgebildet ist. Diese Elektrode 1 wird eingesetzt, wenn eine Gasentladung über eine grössere Fläche auf ein zu behandelndes Objekt, gemäss der vorliegenden Darstellung über die Kreisfläche, erfolgen soll.

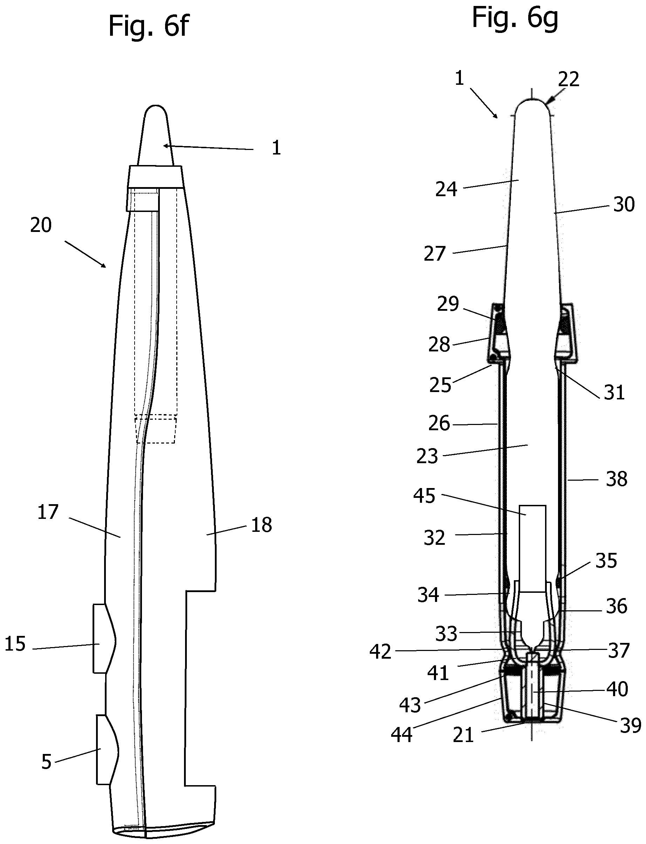

Fig. 6f zeigt die Therapievorrichtung 20 enthaltend ein Gehäuse gemäss Fig. 6a und Fig. 6c sowie eine Elektrode 1 gemäss einer der in Fig. 6b, 6d, 6e gezeigten Varianten, welche ein zweiteiliges Gehäuse 12 umfasst, in welchem eine Elektrode 1 angeordnet werden kann. Die Elektrode 1 ist austauschbar. Um die Elektrode 1 auszutauschen, können das erste Teileelement 17 des Gehäuses 12 und das zweite Teileelement 18 des Gehäuses 12 voneinander getrennt werden.

Fig. 6g zeigt ein Ausführungsbeispiel für eine vierte Variante einer Elektrode 1, welche in einem Längsschnitt dargestellt ist. Die Elektrode 1 weist ein erstes Ende 21 auf, welches zur Kopplung mit dem Modulator 4 ausgebildet ist. Die Elektrode 1 weist ein zweites Ende 22 auf, welches als eine abgerundete Spitze ausgebildet ist. Die Elektrode 1 umfasst einen Glaskörper 27, welcher in einem Halteelement 26 derart angeordnet ist, dass zumindest das zweite Ende 22 das Halteelement 26 überragt. Das Halteelement 26 erstreckt sich in zusammengebautem Zustand im Gehäuse bis zum Anschlagelement 25. Das Anschlagelement 25 ist gemäss dieses Ausführungsbeispiels Bestandteil eines konischen Endabschnitts 28. Im Innenraum des konischen Endabschnitts 28 befindet sich ein Lagerelement 29, welches zur Lagerung des Glaskörpers 27 im Halteelement 26 dient.

Der Glaskörper 27 enthält einen konischen Abschnitt 30, welcher sich vom zweiten Ende 22 bis zum konischen Endabschnitt 28 erstreckt. Der Durchmesser des konischen Abschnitts kann vom zweiten Ende 22 bis zum konischen Endabschnitt 28 kontinuierlich zunehmen. Im Bereich des konischen Endabschnitts 28 enthält der Glaskörper 27 eine Einschnürung 31, das heisst, dessen Durchmesser verringert sich im Bereich des konischen Endabschnitts 28, um sich dann im an den konischen Endabschnitt 28 anschliessenden Mittenabschnitt 38 wieder auf einen grösseren Durchmesser zu erweitern. Der Aussendurchmesser des Glaskörpers 27 im Mittenabschnitt 38 kann im Wesentlichen dem Innendurchmesser des Mittenabschnitts 38 des Halteelements 26 entsprechen. Der Bereich des Glaskörpers 27, welcher im Wesentlichen zylinderförmig ausgebildet ist, soll nachfolgend als Zentralbereich 32 bezeichnet werden.

An den Zentralbereich 32 schliesst ein Endabschnitt 33 des Glaskörpers 27 an, der eine Rille 34 sowie ein Kuppelelement 36 mit einer Spitze 37 umfasst. In der Rille 34 befindet sich ein Dichtungselement 35, welches an der Innenwand des Mittenabschnitts 38 des Halteelements 26 anliegt.

Ein Stiftelement 40 erstreckt sich von der Spitze 37 bis zum ersten Ende 21 der Elektrode. Das Stiftelement 40 ist mit einem Leiterelement 39 verbunden, welches eine elektrische Leitfähigkeit aufweist, sodass die Spannungspulse, die vom Modulator 4 erzeugt werden und vom Generator 3 auf die Hochspannung transformiert werden, in den Glaskörper 27 und dort auf das darin befindliche Gas übertragen werden können. Das Stiftelement 40 ist mit dem Leiterelement 39 verbunden, welches sich vom Stiftelement 40 zu einem Bogenelement 41 erstreckt. Das Leiterelement 39 kann beispielsweise als Draht oder als Hülse ausgebildet sein. Das Bogenelement 41 ist gemäss dieses Ausführungsbeispiels Bestandteil des Leiterelements 39. Das Stiftelement 40 und das Leiterelement 39 sind gegenüber der Umgebung durch das Halteelement 26 elektrisch isoliert. Das Halteelement 26 enthält oder besteht aus einem nicht elektrisch leitfähigen Material, beispielsweise einem Kunststoff. Das Leiterelement 39 durchdringt den Mantel des Glaskörpers 27 und führt in den Innenraum des Glaskörpers 27 zu einer dort angeordneten Anode 45.

Zudem wird das Stiftelement 40 von einem Positionierelement 43 in ihrer axialen Position positioniert und zentriert, sodass sichergestellt ist, dass die Achse des Stiftelements 40 mit der Mittenachse des Glaskörpers 27 fluchtet, d.h. das Stiftelement 40 ist koaxial zum Glaskörper 27 angeordnet. Die Hülse 39 befindet sich im Inneren des an die Rille 26 anschliessenden Endabschnitts 44 des Halteelements 26. Zwischen der Hülse 39 und dem Endabschnitt 44 wird ein ringförmiger Hohlraum ausgebildet.

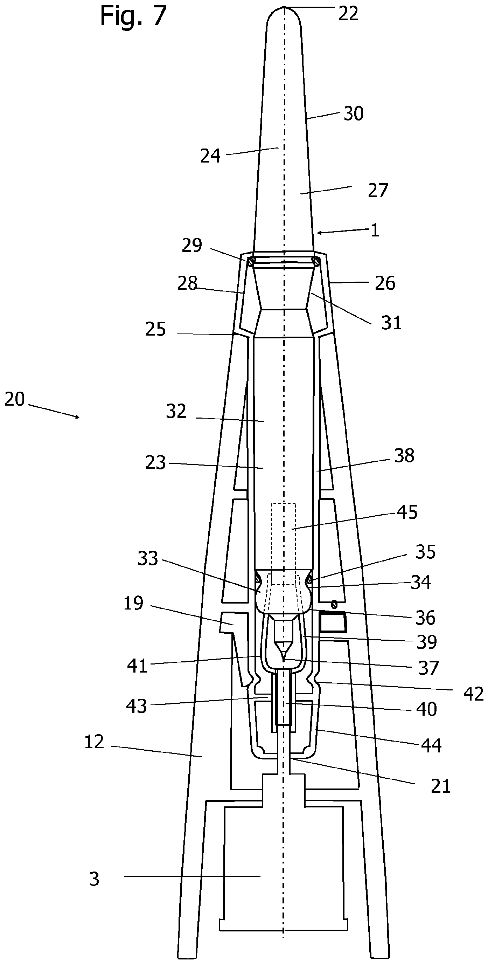

Fig. 7 zeigt einen Teilschnitt einer Therapievorrichtung 20, in welcher eine Elektrode gemäss Fig. 6g im Gehäuse 12 aufgenommen ist. Die Elektrode 1 ist mittels einer Rastverbindung 19 im Gehäuse 12 gehalten. Das Ende des Gehäuses 12 ist auf dem Anschlagelement 25 positioniert, sodass die Elektrode 1 in der gewünschten Position im Gehäuse gehalten ist.

In Fig. 7 ist auch ein Ausführungsbeispiel für die elektrische Koppelung der Elektrode 1 mit dem Generator 3, der insbesondere als Tesla-Spule ausgebildet sein kann. Der Generator 3 enthält ein Anschlusselement 56, welches in Kontakt mit dem Stiftelement 40 steht. Das Stiftelement 40 enthält ein elektrisch leitfähiges Material, sodass die vom Generator 3 erzeugte elektrische Hochspannung über das Stiftelement 40 auf die zur Anode 45 führenden Leiterelemente 39 übertragen kann. Ein Federelement kann vorgesehen sein, um den Kontakt zwischen dem Stiftelement und dem Anschlusselement 56 sicherzustellen.

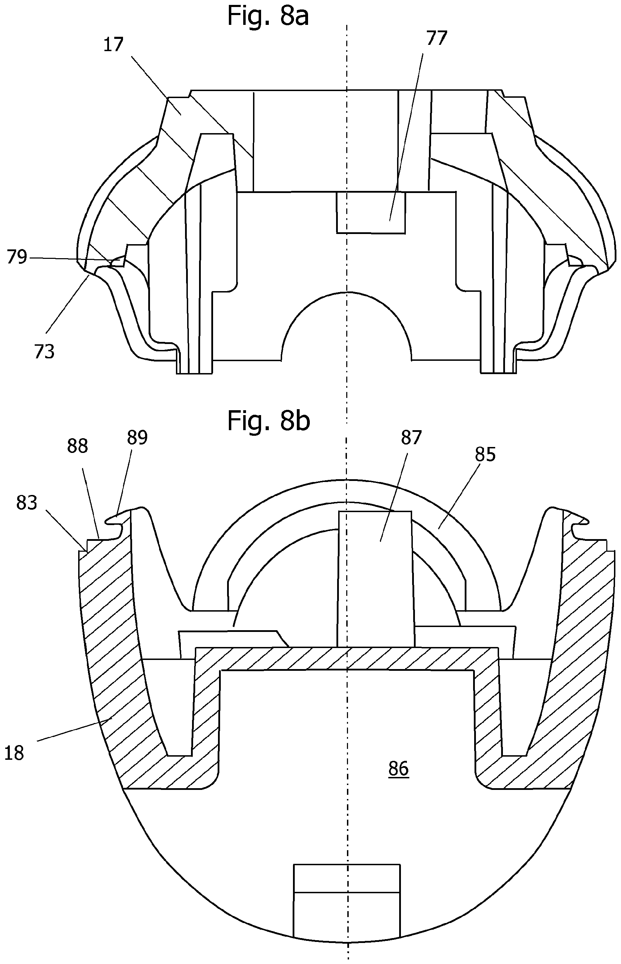

Fig. 8a zeigt einen Radialschnitt durch das erste Teilelement 17 des Gehäuses 12, wobei der Radialschnitt entlang der mit A-A bezeichneten Schnittebene in Fig. 6a gelegt ist. Das erste Teilelement 17 weist eine Kante 73 auf, die zur Auflage auf einem Absatz 83 des zweiten Teilelements 18 ausgebildet ist. Die Kante 73 erstreckt sich vom ersten Ende 71 des ersten Teilelements 17 zum zweiten Ende 72 des ersten Teilelements 17, siehe hierzu auch Fig. 6a.

Die Kante 73 enthält zwischen dem ersten Ende 71 und dem zweiten Ende 72 mindestens je eine Ausnehmung, die in Fig. 6a nicht sichtbar ist, da sie sich auf der Innenseite des Gehäuses befindet.

Fig. 8b zeigt einen Radialschnitt durch das zweite Teilelement 18 des Gehäuses 12, der entlang der mit B-B bezeichneten Schnittebene in Fig. 6c gelegt ist. Ein Absatz 83 erstreckt sich vom ersten Ende 81 des zweiten Teilelements 18 zum zweiten Ende 82 des zweiten Teilelements 18, siehe hierzu auch Fig. 6c.

Das zweite Teilelement 18 enthält an seinem zweiten Ende 82 ein Ringelement 84, welches zur Aufnehme des zweiten Endes 72 des ersten Teilelements 17 ausgebildet ist. Im Inneren des Ringelements 84 ist im zusammengebauten Zustand die Elektrode 1 aufgenommen.

An seinem ersten Ende enthält das zweite Teilelement 18 gemäss des vorliegenden Ausführungsbeispiels ein Rastelement 85, welches zur Aufnahme in einer korrespondierenden Ausnehmung im ersten Teilelement 17 ausgebildet ist. Die Ausnehmung befindet sich am ersten Ende 71 des Teilelements 17 auf der Innenseite der Stirnwand. Sie ist in Fig. 6a nicht sichtbar und auch in Fig. 8a nicht sichtbar, da sie vor der Schnittebene liegt, also in dieser Darstellung zum weggeschnittenen Teil der Fig. 8b gehörig ist.

Die Schnittebene in Fig. 8b verläuft durch die Aufnahmeöffnung für das Ladegerät 16, welches abnehmbar an der Unterseite des zweiten Teilelements anbringbar ist. Auf der der Aufnahmeöffnung 86 gegenüberliegenden Seite des zweiten Teilelements 18 befinden sich die Elektrode 1, der Modulator 4, der Generator 3, die Prozessoreinheit enthaltend ein Steuer- Regel- und Berechnungsmodul, gegebenenfalls die Speichereinheit 9 sowie die zugehörigen Anschlüsse und Verbindungsleitungen, die in Fig. 1b oder Fig. 1c schematisch dargestellt sind.

Das erste Teilelement 17 und das zweite Teilelement 18 können zusätzlich mittels einer Schraubverbindung gesichert sein. Hierzu enthält das erste Teilelement einen Stutzen 77, der eine nicht dargestellte Gewindebohrung enthält, der mit einem Stutzen 87 des zweiten Teilelements 18 fluchtet, wenn das erste Teilelement 17 und das zweite Teilelement 18 zum Gehäuse 12 zusammengebaut sind.

In Fig. 8b ist eine zusätzliche Ausnehmung 88 unmittelbar angrenzend an den Absatz 83 vorgesehen. An diese Ausnehmung 88 schliesst ein hakenförmiger Vorsprung 89 an. Dieser hakenförmige Vorsprung 89 greift im zusammengebauten Zustand in eine entsprechende Ausnehmung 79 des ersten Teilelements 17 ein. Somit rastet der hakenförmige Vorsprung 89 in die entsprechende Ausnehmung 79 ein, wodurch das erste Teilelement 17 das zweite Teilelement 18 an der Verbindungsstelle überdeckt, sodass an der Verbindungsstelle eine Doppelwand entsteht, zwischen welcher ein kleiner Luftspalt bestehen bleibt. Somit wird an der Verbindungslinie ein überraschend besserer Schutz gegen die elektrischen Spannungen erzielt, sodass die Person, die das Gerät bedient, keiner Gefahr durch die verwendeten elektrischen Spannungen ausgesetzt ist.

Mittels der erfindungsgemässen Therapievorrichtung nach einem der Ausführungsbeispiele kann mittels der Bedienelemente 5, 15 einerseits die Amplitude der Spannungspulse eingestellt werden und andererseits die Frequenz der Spannungspulse. Gemäss eines Ausführungsbeispiels kann die Amplitude der elektrischen Spannung im Bereich der Werte 1 bis 9 verändert werden. Die Frequenz kann im Bereich zwischen 10 und 100 Pulsen pro Sekunde liegen. Die leistungsstärkste Einstellung ergibt sich somit bei Auswahl der Amplitude 9 und der Auswahl von 100 Pulsen pro Sekunde. In den nachfolgenden Messbeispielen wird die leistungsstärkste Einstellung mit HI bezeichnet. Die leistungsschwächste Einstellung ergibt sich somit bei Auswahl der Amplitude 1 und der Auswahl von 10 Pulsen pro Sekunde. In den nachfolgenden Messbeispielen wird die leistungsschwächste Einstellung mit LO bezeichnet.

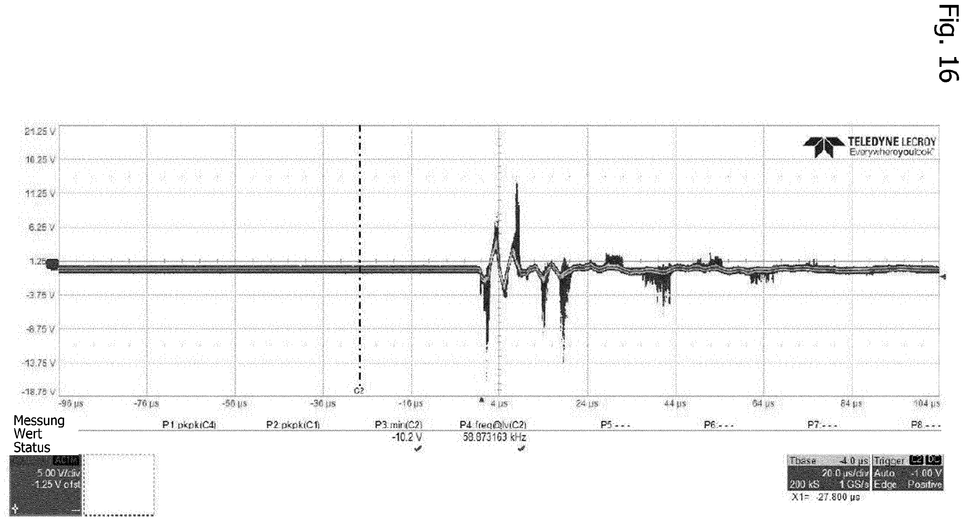

Die Stromstärke der Pulse wurde mittels eines Oszilloskops vom Typ Teledyne Le Croy Waverunner 8254M über einen elektrischen Widerstand von 100 $\Omega$ aufgezeichnet, der zwischen der Kathode und der Erdung angeordnet war, wobei das Verhältnis der elektrischen Spannungen 10:1 betrug (Teledyne Le Croy, PP024). Die optimale Distanz D zwischen der Plasmaquelle und der Kathode betrug 1 bis 2 mm, wobei die Kathode als Kupferelement ausgebildet war. Für diese Distanz wurde die Amplitude zwischen 10 und 100 Pulsen/s variiert. Die Stromstärken der Pulse waren stabil und entsprachen den Angaben auf der Therapievorrichtung für alle Einstellungen. In der Fig. 16 wurde der Verlauf elektrischen Spannung für eine Zeitdauer von ungefähr 200 $\mu$s aufgezeichnet.

Die Auswirkung auf die Stabilität der Plasmaquelle wurde durch Anpassung der Amplitude untersucht. Zu diesem Zweck wurde die Frequenz der Entladungsspitzen im Bereich von 10 Hz bis 100 Hz mittels des Oszilloskops untersucht. Die Analyse der Strompulse ergab bis zu 12 Entladungen pro Spannungspuls bei den Einstellungen HI (Amplitude 9V, 100 Pulse/s). Der Entladungsvorgang war bei der höchsten Frequenz von 100 Hz und der höchsten Amplitude am stabilsten, was in Fig. 16 gezeigt ist.

Messbeispiel 1

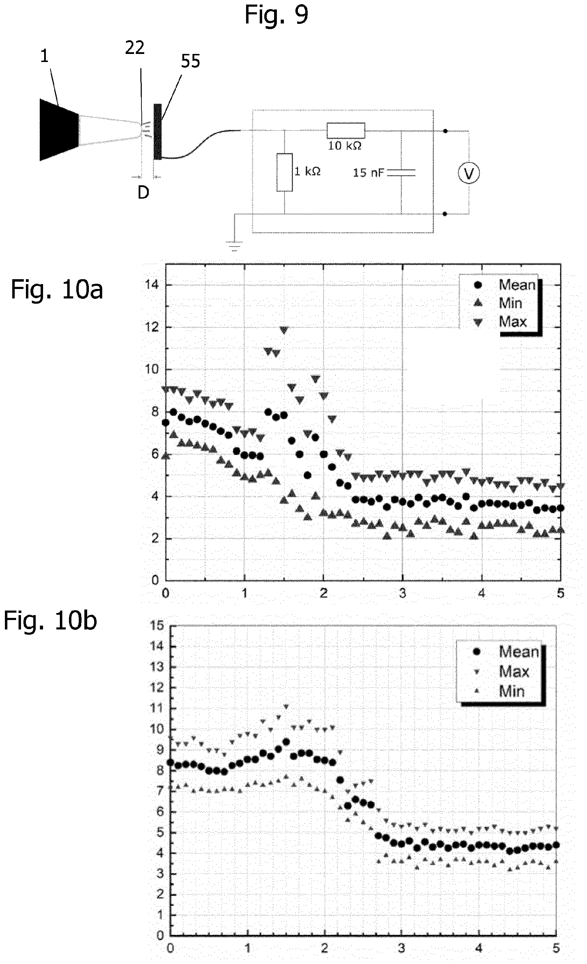

Der Patientenableitstrom (I) wurde gemäss der Messvorschrift nach DIN EN 60601-1 2] ermittelt. Die Messanordnung ist schematisch in Fig. 9 gezeigt. Der in Fig. 9 gezeigte Schaltkreis zeigt einen Tiefpassfilter, der die elektrische Antwort des menschlichen Körpers beschreibt, insbesondere berücksichtigt, dass Ströme von höherer Frequenz als weniger schädlich einzustufen sind. Da die Therapievorrichtung 20 als kabelloses Gerät ausgebildet ist, kann nur ein Wechselstrom gemessen werden. Daher wurde ein R-C Element enthaltend den Schaltkreis gemäss Fig. 9 an ein Multimeter vom Typ Fluke 116 True RMS angeschlossen, um den Patientenableitstrom zu ermitteln. Die Messanordnung besteht aus optischen Haltern, sodass eine Ebenenparallelität zwischen der Kathode 55 und dem zweiten Ende 22 der Elektrode 1, welche die Spitze der Plasmaquelle ausbildet, gewährleistet ist. Die Distanz wurde mittels einer Mikrometerschraube genau eingestellt. Der Patientenableitstrom wurde somit in Abhängigkeit von der Distanz D zwischen dem zweiten Ende 22 der Elektrode 1 und der Kathode 55 gemessen, die als Kupferplatte ausgebildet ist. Der Patientenableitstrom soll einen Maximalwert von 100 $\mu$A nicht überschreiten, damit die Therapievorrichtung für medizinische Zwecke einsetzbar sein kann. Für die Dauer von 10 s wurde der Maximalwert (▼), der Minimalwert (▲) sowie der Mittelwert (•) des Patientenableitstroms aufgezeichnet. Hierbei wurde angenommen, dass der Maximalwert entscheidend für die Beurteilung der Anwendbarkeit für medizinische Zwecke ist.

Gemäss Fig. 10a wird der Patientenableitstrom für eine erste Therapievorrichtung (TV1) auf der Ordinate in [$\mu$A] aufgetragen, auf der Abszisse wurde die Distanz D in [mm] zwischen dem zweiten Ende 22 der Elektrode 1 und einer die Kathode 55 ausbildenden Kupferplatte aufgetragen. Gemäss Fig. 10a wird auf keiner Distanz der Grenzwert von 100 [$\mu$A] erreicht, selbst mit den maximal möglichen Einstellungen. Der erreichte Maximalwert beträgt 12 [$\mu$A] auf einer Distanz von 1.5 mm. Im Bereich von 0 bis 3 mm wird ein Filament und ein stabiles Luftplasma erzeugt. Bei grösseren Distanzen entstand kein Plasma, obwohl ein Patientenableitstrom ermittelt wurde. Für die niedrigste Einstellung (LO) wurde überhaupt kein Patientenableitstrom detektiert.

Fig. 10b zeigt den Patientenableitstrom für eine zweite Therapievorrichtung (TV2). Für diese Therapievorrichtung wurde für eine Zeitdauer von 10s der maximale, der minimale Wert und der Mittelwert für den Patientenableitstrom bei einer Umgebungstemperatur von 23 °C und 45 % relativer Luftfeuchtigkeit aufgezeichnet. Die Therapievorrichtung (TV2) zeigte eine hörbare und messbare Veränderung des Plasmas in Abhängigkeit von der angelegten Spannung. Um die höchste Intensität zu erreichen wurde die Therapievorrichtung mit geerdeter Aluminiumfolie umwickelt. Die maximale Dauer für die Messungen mit einer Ladung der Energiespeichereinheit betrug 25 min. Mit zwei Ladungen kam es ab einer Betriebsdauer von ca. 50 min zu Ausfällen, wobei die Gehäusetemperatur der zweiten Therapievorrichtung (TV2) maximal 43 ° C angenommen hat. Bei einer Betriebsdauer von mehr als 50 min kann es durch die Erhöhung der Betriebstemperaturen zu Störungen in der Prozessoreinheit kommen, welche den Betrieb des Steuermoduls, Regelmoduls oder Berechnungsmoduls der Therapievorrichtung teilweise beeinträchtigen kann. Daher wurden zusätzliche Messungen nach einer Abkühlphase von ca. 30 min nach zwei Ladungszyklen durchgeführt.

Auch für die zweite Therapievorrichtung (TV2) wurde der Patientenableitstrom von 100 $\mu$A zu keinem Zeitpunkt erreicht. Der maximale Patientenableitstrom betrug 11 $\mu$A. Bei einer Distanz D von 0 bis einschliesslich 3 mm zwischen dem zweiten Ende 22 der Elektrode 1 und der Kathode 55 wurde ein stabiles sekundäres Kaltplasma in der Luft erzeugt. Die Häufigkeit der Entladungen nahm bei Distanzen von grösser als 2 mm ab, wodurch sich der Patientenableitstrom verringerte. Für die zweite Therapievorrichtung (TV2) konnte bei einer Distanz D > 3mm visuell oder hörbar kein Plasma detektiert werden, obwohl ein geringer Patientenableitstrom gemessen wurde.

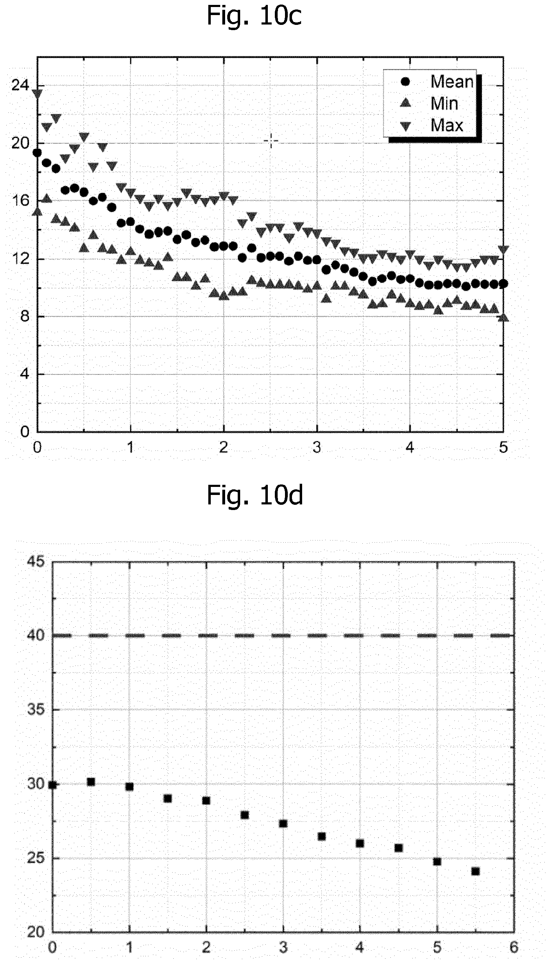

Fig. 10c zeigt den Patientenableitstrom für eine dritte Therapievorrichtung (TV3), welche eine LSE Elektrode enthält. Für diese Therapievorrichtung wurde für eine Zeitdauer von 10s der maximale, der minimale Wert und der Mittelwert für den Patientenableitstrom bei einer Umgebungstemperatur von 23 °C und 49 % relativer Luftfeuchtigkeit aufgezeichnet.

Auch für die dritte Therapievorrichtung (TV3) wurde der Patientenableitstrom von 100 $\mu$A zu keinem Zeitpunkt erreicht. Der maximale Patientenableitstrom betrug 23.5 $\mu$A, wenn die LSE Elektrode die Kathode berührt. Bei einer Distanz D von 0 bis einschliesslich 3.5 mm wurde zwischen dem zweiten tellerförmigen Ende 22 der LSE Elektrode 1 und der Kathode 55 ein stabiles sekundäres Kaltplasma in der Luft erzeugt. Die Entladungen wurden bei grösseren Distanzen diskontinuierlich, obwohl ein geringer Patientenableitstrom gemessen wurde.

Fig. 10d zeigt den Verlauf der Temperatur in Grad Celsius in Abhängigkeit von der Distanz D der Elektrode von der Kathode. Die Distanz D wird hierbei auf der Abszisse aufgetragen, die Temperatur auf der Ordinate. Die Raumtemperatur betrug zum Zeitpunkt der Messung 23 °C bei einer relativen Luftfeuchtigkeit von 51%. Für die zweite Therapievorrichtung (TV2) betrug die maximal gemessene Temperatur 30 Grad Celsius. Der Grenzwert für die Temperatur, der bei 40 Grad Celsius liegt, wurde zu keinem Zeitpunkt erreicht.


Messbeispiel 2

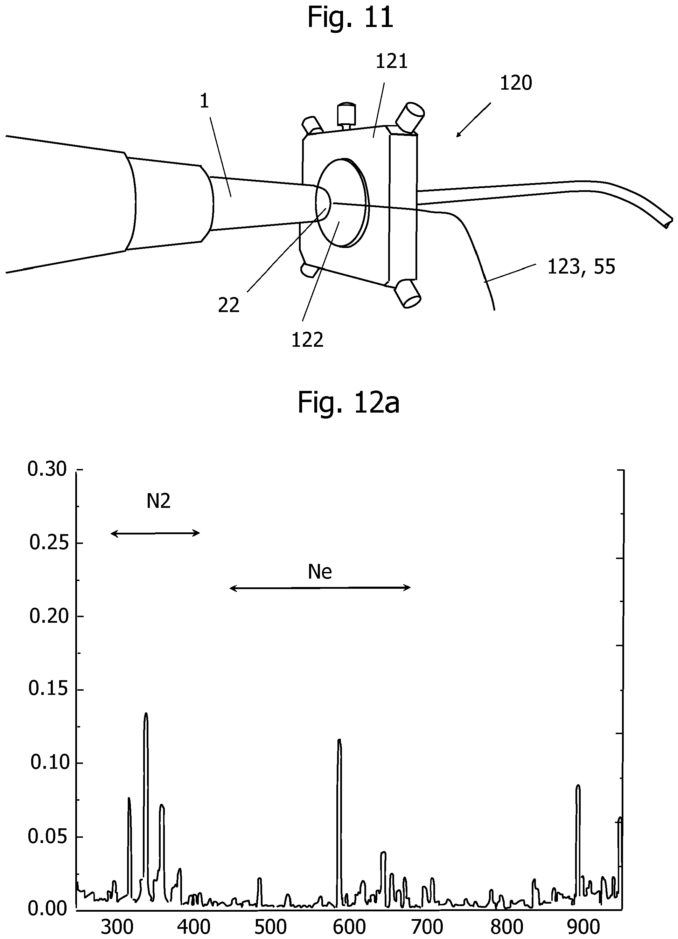

Mittels optischer Emissionsspektroskopie (OES) wurde die spektrale Zusammensetzung der optischen Plasmastrahlung ermittelt. Die entsprechende Messanordnung ist schematisch in Fig. 11 gezeigt. Die optische Emissionsspektroskopie wurde im UV-Bereich (UV), im sichtbaren Bereich (VIS) und im Nahinfrarotbereich (NIR) mittels eines kalibrierten fiberoptischen Spektrometers 120 vom Typ AvaSpec 3648-USB2, Avantes, Apeldoorn, NL durchgeführt. Die Plasmaemission wurde mittels eines Kosinuskorrektors 121 ermittelt, um den Öffnungswinkel zu vergrössern. Um den Kosinuskorrektor vor direktem Plasmakontakt zu schützen, wurde auf dessen der Elektrode zugewendeten Seite ein Quarzfenster 122 (d=2mm), angebracht, welches transparent für Wellenlängen grösser als 200 nm ist. Die erforderlichen Halterungen für das Messgerät und die erste, zweite und dritte Therapievorrichtung (TV1, TV2, TV3) wurden in der vorliegenden Darstellung weggelassen.

Ein geerdeter Draht 123 diente als Kathode 55. Aufgrund des kleinen Durchmessers des Drahts von 0.1 mm verdeckte der Draht 123 die Plasmalichtquelle kaum. Der Abstand D zwischen dem zweiten Ende 22 der Elektrode 1 und der Kathode 55 betrug ungefähr 1.5 mm, da dieser Wert dem höchsten Patientenableitstrom entsprach. Fünf Spektren wurden aufgenommen und anschliessend analysiert, wobei für jedes Spektrum eine Integrationszeit von 30 s vorgesehen war. Für medizinische Anwendungen ist die UV-Bestrahlungsstärke ist von besonderem Interesse. Sie wurde in zwei Bereichen, UV-A (315-380 nm) und UV-B (280-315nm), durch Integration der spektralen Bestrahlungsstärke E ($\lambda$) gemessen. Im UV-C Bereich (200-280 nm) wurden keine Emissionen festgestellt.

Fig. 12a zeigt das vollständige Spektrum der ersten Therapievorrichtung (TV1), welches durch die Bildung eines Mittelwerts aus 5 aufeinanderfolgenden Spektren mit einer Aufzeichnungsdauer von je 30 s ermittelt worden ist. Die Skala auf der Ordinate auf der linken Seite von Fig. 12a wurde für die Therapievorrichtung (TV1) verwendet. Das Spektrum der Therapievorrichtung (TV1) zeigt eine Emission von Neon (Ne) sowie Stickstoff ($N_2$). Die Messungen erfolgten bei einer Umgebungstemperatur von 22.7 °C und 61% relativer Luftfeuchtigkeit.

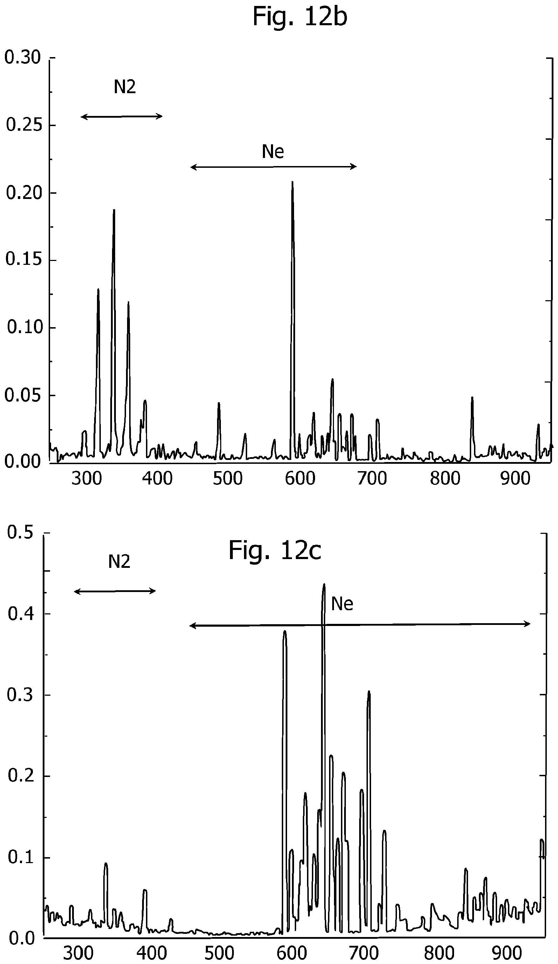

Fig. 12b zeigt ein Spektrum der zweiten Therapievorrichtung (TV2). Die Messungen erfolgten bei einer Umgebungstemperatur von 23 °C und 50% relativer Luftfeuchtigkeit, die Messung erfolgte ansonsten in gleicher Weise wie für die erste Therapievorrichtung (TV1).

Fig. 12c zeigt ein Spektrum der dritten Therapievorrichtung (TV3). Die Messungen erfolgten bei einer Umgebungstemperatur von 22.5 °C und 48% relativer Luftfeuchtigkeit, die Messung erfolgte ansonsten in gleicher Weise wie für die erste Therapievorrichtung (TV1).

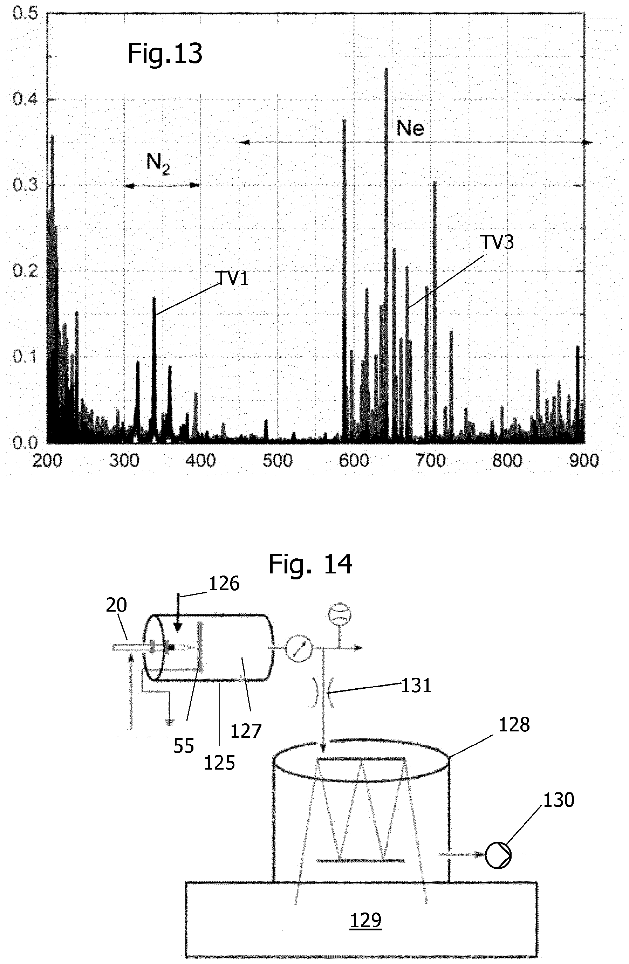

Fig. 13 zeigt ein Spektrum der dritten Therapievorrichtung (TV3) im Vergleich mit der ersten Therapievorrichtung (TV1). Im Vergleich zu (TV1) waren die Emissionen von Neon höher, aber von Stickstoff niedriger. Dies konnte mit der grösseren Fläche von leuchtendem Neon im Inneren der LSE-Elektrode erklärt werden. Mittels der Elektrode der ersten Therapievorrichtung (TV1) kann aufgrund der geometrischen Ausführung und der damit verbundenen Vergrösserung des elektrischen Feldes an dem als Spitze ausgebildeten zweiten Ende eine fokussierte Entladung auf die Kathode erfolgen. Die Entladungen erfolgten im Unterschied zur (TV1) an verschiedenen Stellen des geerdeten Drahts. Das Plasma konnte aufgrund der Positionierung des Drahtes nicht in seiner Gesamtheit erfasst werden, daher ergeben sich für die erste Therapievorrichtung (TV1) höhere Werte für die Stickstoffemissionen. Die Bestrahlungsstärken im UV-A und UV-B Bereich sowie die effektive Bestrahlungsstärke der dritten Therapievorrichtung (TV3) am Messpunkt mit der höchsten Intensität wurde mit den Werten der ersten Therapievorrichtung (TV1) verglichen. Die Bestrahlungsstärken sind relativ niedrig, wie das Spektrum bereits angedeutet hat. Die maximale tägliche Behandlungszeit tmax ist mit ungefähr 1h sehr hoch. Allerdings sind die UV-B Werte sowie die effektiven Bestrahlungsstärken höher als für die erste Therapievorrichtung (TV1). Dies kann mit der höheren Neonemission zusammenhängen, allerdings kann die Messung in den gewichteten Regionen unter 300 nm auch grossem Rauschen unterliegen.

Die internationale Kommission für den Schutz vor nicht-ionisierender Strahlung hat ein Verfahren zur Bestimmung einer effektiven Bestrahlungsstärke publiziert, weil verschiedene Wellenlängen eine unterschiedliche Schädigung auf der menschlichen Haut verursachen. Eine spektrale Gewichtungsfunktion $S(\lambda)$ muss mit der spektralen Bestrahlungsstärke $E(\lambda)$ multipliziert werden und über den gesamten UV-Bereich von 200 bis 380 nm integriert werden, um eine effektive Bestrahlungsstärke $E_{eff}$ nach der folgenden Formel zu berechnen:

$$E_{eff} = {_{\lambda 1}}\int^{\lambda 2} E(\lambda) \cdot S(\lambda) d(\lambda)$$

Eine maximale tägliche Bestrahlungszeit $t_{max}$ kann aus der effektiven Bestrahlungsstärke $E_{eff}$ berechnet werden, wenn die maximale tägliche Dosis von $D_{max} = 3 \ mJ/cm^2$ gemäss nachfolgender Formel verwendet wird:

$$t_{max} = D_{max} / E_{eff}$$

In der nachfolgenden Tabelle 1 sind die Bestrahlungsstärken für UV-A und UV-B sowie die effektive Bestrahlungsstärke $E_{eff}$ der ersten zweiten und dritten Therapievorrichtungen (TV1, TV2, TV3) gezeigt. Die Bestrahlungsstärken sind relativ niedrig, wie im Spektrum bereits angedeutet. Im UV-C Bereich erfolgten keine signifikanten Emissionen. Die maximale tägliche Behandlungszeit ist mit 6h für die erste Therapievorrichtung und mit 5h für die zweite Therapievorrichtung sehr hoch.

Tabelle 1

| Vorrichtung, D | $E_{UV-A}$ | $E_{UV-B}$ | $E_{eff}$ | $t_{max}$ |
|---|---|---|---|---|
| Dimension | $\mu W/cm^2$ | $\mu W/cm^2$ | $\mu W/cm^2$ | h |
| (TV1) 1.5 mm | $0.91 \pm 0.03$ | $0.19 \pm 0.03$ | $0.14 \pm 0.2$ | ~6 |
| (TV2) 1.5 mm | $1.21 \pm 0.05$ | $0.22 \pm 0.01$ | $0.16 \pm 0.2$ | ~5 |
| (TV3) 1.5 mm | $0.68 \pm 0.04$ | $0.37 \pm 0.02$ | $0.86 \pm 0.07$ | ~1 |

Messbeispiel 3

Im dritten Messbeispiel wurde eine FTIR-Spektroskopie durchgeführt, eine Fourier-transformierte Infrarot-Absorptionsspektroskopie, nachfolgend als FTIR bezeichnet. FTIR wurde zur qualitativen und quantitativen Bestimmung der Zusammensetzung der reaktiven Spezies, die während der Entladung gebildet wurden, verwendet. Eine Absorption im Infrarotbereich korrespondierend zur Anregung von Molekülvibrationen und Rotationen ist ein charakteristisches Merkmal von heteronuklearen Molekülen, wie verschiedene Stickoxide oder auch Ozon. Die Bestimmung erfolgt durch Messung einer Hintergrundstrahlungsintensität $I_0$ und einer Strahlungsintensität nach der Absorption durch die Probe $I$, welche beide von der Wellenzahl $v$ abhängig sind. Hiermit wird die Bestimmung eines Absorptionskoeffizienten $A$ ermöglicht, wobei gilt $A = -\ln(I(v)/I_0(v)) = \Sigma_i (n_i\sigma_i(v)L$. Der Absorptionskoeffizient $A$ wird von der Länge der optischen Wegstrecke sowie der Dichte $n$ und des von der Wellennummer abhängigen Absorptionsquerschnitts $\sigma$ für jede Spezies

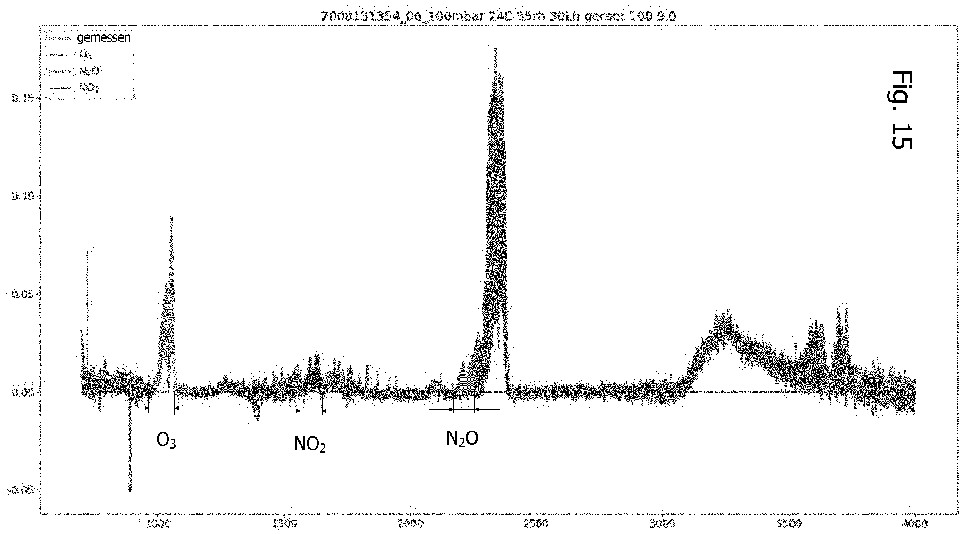

i beeinflusst. Plasma-chemische Verfahren enthalten komplexe Reaktionsnetzwerke, die auf unterschiedlichen Zeitskalen ablaufen, daher ist die Zusammensetzung der Spezies während der Entladung veränderlich und konvergiert zu einer stationären Mischung von langlebigen Komponenten im Nachleuchten. Die in Fig. 15 dargestellten Messergebnisse beziehen sich auf den stationären Zustand.

Fig. 14 zeigt die Messanordnung zur Durchführung der FTIR-Spektroskopie. Das mittels der Therapievorrichtung 20 behandelte Gas, welches einem Behälter 125 durch einen Raumlufteinlass 126 zugeführt wird, wird in einer Gassammelzelle 127 gesammelt und in eine Absorptionszelle mit mehreren Durchgängen (MPC) 128 eingesaugt, die an ein Bruker Vertex 80v Spektrometer 129 angeschlossen ist. Die MPC 128 weist eine optische Weglänge L von 32 m auf und ermöglicht daher auch die Messung von kleinen Dichten n von absorbierenden Spezies. Eine Vakuumpumpe 130 ermöglicht das Einleiten der Raumluft in den Behälter 125 und in die MPC 128. Mittels der Vakuumpumpe 130 und einer Zuflussdrosselung durch ein Drosselventil 131 wurde ein Druck von 100 mbar in der MPC 128 erreicht. Die Messungen wurden für die leistungsstärkste Kombination (HI) und für die leistungsschwächste Kombination (LO) der entsprechenden Therapievorrichtung 20 durchgeführt. Die Luftdurchsatzmenge betrug für alle Messungen 30l/h und wurde von einem Durchflussmessgerät vom Typ Omega SMA66C gemessen und überprüft.

Eine geerdete Kathode 55 wurde im Behälter 125 in einem Abstand D=1 mm vom zweiten Ende 22 der Elektrode 1 der Therapievorrichtung 20 angebracht.

Die Messungen decken einen Wellennummernbereich von 700 bis 4000 cm$^{-1}$ mit einer Auflösung von 0.2 cm$^{-1}$ ab, was die Detektion von Spezies erlaubt, die typisch für atmosphärische Kaltplasmen sind, nämlich $O_3$, $NO$, $NO_2$, $N_2O$, $N_2O_5$, $HNO_3$, $HNO_2$ und $H_2O_2$. Die Veränderung der Konzentrationen an $CO_2$ und $H_2O$ in der Raumluft wurden ebenfalls erfasst. Fig. 15 zeigt ein exemplarisches Spektrum der ersten Therapievorrichtung (TV1) mit eingefügten Referenzspektren für die identifizierten Spezies $O_3$, $N_2O$, $NO_2$. Die weiteren Absorptionspeaks betreffen $CO_2$ und $H_2O$ oder betreffen weitere Absorptionsbänder der identifizierten Spezies. Auf der Abszisse von Fig. 15 ist die Wellennummer in [cm$^{-1}$] und auf der Ordinate der Absorptionskoeffizient A aufgetragen. Die Messung wurde bei 100 mbar bei einer Raumtemperatur von 24°C und einer relativen Luftfeuchtigkeit von 55% durchgeführt. Die Luftdurchsatzmenge betrug 30 l/h mit der Einstellung der leistungsstärksten Kombination (HI) auf der Therapievorrichtung.

Insbesondere wurde die Konzentration der mittels der Therapievorrichtung erzeugten langlebigen Sauerstoff- und Stickstoffspezies (RONS) mittels FTIR Spektroskopie gemessen. Diese Spezies werden für medizinische Anwendungen als einer der Schlüsselmechanismen angesehen, um erwünschte Behandlungseffekte zu erzielen. Die zuverlässige Identifizierung und exakte Quantifizierung der RONS ist essenziell für die Erfüllung der DIN SPEC 91315. Als Spezies mit den höchsten Konzentrationen wurden $O_3$, $N_2O$, $NO_2$ gemessen. Da die Konzentrationen von $N_2O$ und $NO_2$ bereits im Bereich von 1 ppm lagen, somit am unteren Ende des messbaren Bereichs liegen, konnten weitere Spezies mit geringeren Konzentrationen nicht mehr verlässlich identifiziert werden. Die Konzentration von $O_3$ lag bei 15 ppm $\pm$ 4 ppm. Die Konzentration von $N_2O$ lag bei 1 ppm $\pm$ 0.05 ppm, die Konzentration von $NO_2$ lag bei 2 ppm $\pm$ 0.5 ppm. Diese Werte wurden für die leistungsstärkste Einstellung (HI) der Therapievorrichtung 20 ermittelt. Für die leistungsschwächste Einstellung (LO) wurden keine Emissionen detektiert.

5. Messbeispiel

Für die Therapievorrichtung wurden chemische Spezies in der flüssigen Phase ermittelt. Hierzu wurde eine Salzlösung in einer 24-Well-Titerplatte zubereitet, die aus 500 $\mu$l Wasser und 500 $\mu$l NaCl-Salzlösung besteht. Das zweite Ende 22 der Elektrode 1 wurde in einem Abstand von 1 bis 2 mm vertikal über der Flüssigkeitsoberfläche positioniert. Eine Plasmabehandlung wurde für 10 s, 30 s, 60 s, 180 s, 300 s direkt auf die Flüssigkeitsoberfläche vorgenommen.

Zur Ermittlung der Stabilität sowie zum Vergleich der Herstellung von reaktiven Sauerstoff Spezies (ROS) von sieben Elektroden, wurde die Anreicherung von $H_2O_2$ unmittelbar nach dem Kontakt mit dem Plasma ermittelt. Chemische Parameter wurden unmittelbar nach dem Kontakt mit dem Plasma ermittelt.

Es wurden pH-Werte mittels des pH Messgeräts HANNA edge blu (Hanna instruments) basierend auf einer Glaskörperelektrode in Wasser und NaCl ermittelt.

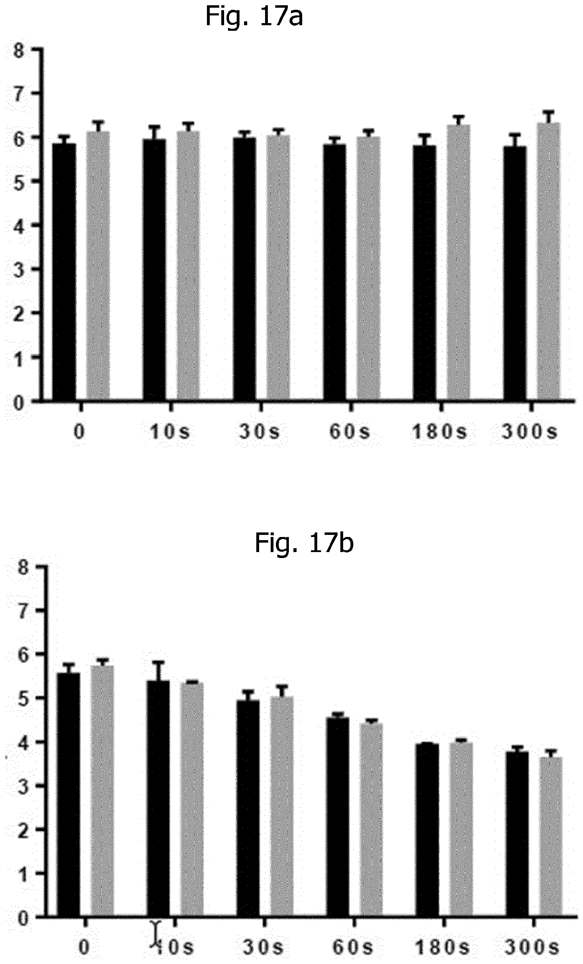

Fig. 17a zeigt den pH-Wert, der auf der Ordinate aufgetragen ist, in Abhängigkeit von der gewählten Messdauer, die auf der Abszisse aufgetragen ist für die erste Therapievorrichtung (TV1). Die Messwerte wurden hier für die Behandlungsdauer von 0 s (Referenzwert), 10 s, 30 s, 60 s, 180 s, 300 s für die LO-Einstellung der Therapievorrichtung (TV1) ermittelt. Der jeweils linksseitige Balken (schwarz) entspricht dem pH-Messwert für $H_2O$, der korrespondierende rechtsseitige Balken (grau) entspricht dem korrespondierenden Messwert für NaCl. Der pH-Messwert bleibt für die LO-Einstellung sowohl für $H_2O$ als auch für NaCl weitgehend konstant, d.h. ändert sich nicht bei zunehmender Behandlungsdauer.

Fig. 17b zeigt den pH-Wert, der auf der Ordinate aufgetragen ist, in Abhängigkeit von der gewählten Messdauer, die auf der Abszisse aufgetragen ist für die erste Therapievorrichtung (TV1). Die Messwerte wurden hier für die Behandlungsdauer von 0 s (Referenzwert), 10 s, 30 s, 60 s, 180 s, 300 s für die HI-Einstellung der Therapievorrichtung (TV1) ermittelt. Der jeweils linksseitige Balken (schwarz) entspricht dem pH-Messwert für HzO, der korrespondierende rechtsseitige Balken (grau) entspricht dem korrespondierenden Messwert für NaCl. Der pH-Messwert nimmt für die HI-Einstellung sowohl für $H_2O$ als auch für NaCl mit zunehmender Behandlungsdauer ab, das heisst in beiden Flüssigkeiten erfolgt eine zunehmende Ansäuerung bei zunehmender Behandlungsdauer. Die Ansäuerung in beiden Flüssigkeiten korreliert mit der Behandlungsdauer, für $H_2O$ nahm der pH-Messwert von 5.57 auf 3.78 ab, für NaCl nahm der pH-Messwert von 5.73 auf 3.66 ab.

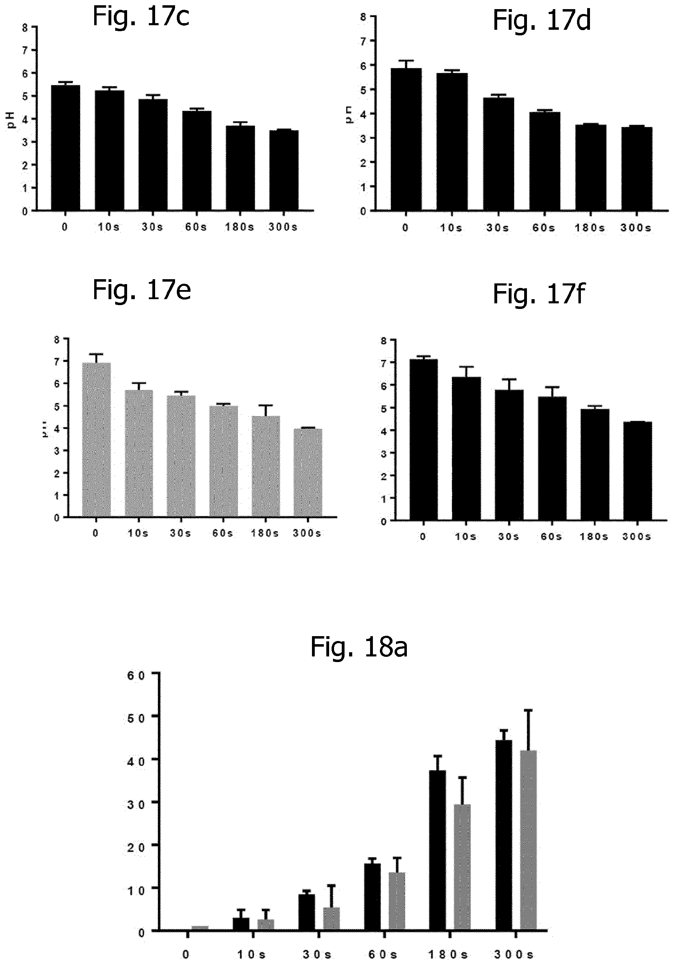

Fig. 17c zeigt den pH-Wert, der auf der Ordinate aufgetragen ist, in Abhängigkeit von der gewählten Messdauer, die auf der Abszisse aufgetragen ist, für die Therapievorrichtung (TV2) in Wasser. Die Messwerte wurden hier für die Behandlungsdauer von 0 s (Referenzwert), 10 s, 30 s, 60 s, 180 s, 300 s ermittelt.

Fig. 17d zeigt den pH-Wert, der auf der Ordinate aufgetragen ist, in Abhängigkeit von der gewählten Messdauer, die auf der Abszisse aufgetragen ist, für die Therapievorrichtung (TV2) in NaCl. Die Messwerte wurden hier für die Behandlungsdauer von 0 s (Referenzwert), 10 s, 30 s, 60 s, 180 s, 300 s ermittelt.

Der pH-Messwert nimmt für die zweite Therapievorrichtung (TV2) sowohl für $H_2O$ als auch für NaCl mit zunehmender Behandlungsdauer ab, das heisst in beiden Flüssigkeiten erfolgt eine zunehmende Ansäuerung bei zunehmender Behandlungsdauer. Die Ansäuerung in beiden Flüssigkeiten korreliert mit der Behandlungsdauer, für $H_2O$ nahm der pH-Messwert von 5.46 (+/- 0.148) auf 3.51 (+/- 0.03) ab, für NaCl nahm der pH-Messwert von 5.87 (+/- 0.3) auf 3.44 (+/-0.04) ab.

Fig. 17e zeigt den pH-Wert, der auf der Ordinate aufgetragen ist, in Abhängigkeit von der gewählten Messdauer, die auf der Abszisse aufgetragen ist, für die Therapievorrichtung (TV3) mit einer ersten Elektrode in Wasser. Die Messwerte wurden hier für die Behandlungsdauer von 0 s (Referenzwert), 10 s, 30 s, 60 s, 180 s, 300 s ermittelt. Der pH-Wert nahm in Wasser mit zunehmender Behandlungsdauer ab, d.h. es erfolgt eine Ansäuerung. Der mittlere pH-Wert fiel von 6.92 auf 3.98.

Fig. 17f zeigt den pH-Wert, der auf der Ordinate aufgetragen ist, in Abhängigkeit von der gewählten Messdauer, die auf der Abszisse aufgetragen ist, für die Therapievorrichtung (TV3) mit einer zweiten Elektrode in Wasser. Die Messwerte wurden hier für die Behandlungsdauer von 0 s (Referenzwert), 10 s, 30 s, 60 s, 180 s, 300 s ermittelt. Der pH-Wert nahm in Wasser mit zunehmender Behandlungsdauer ab, d.h. es erfolgt eine Ansäuerung. Der mittlere pH-Wert fiel von 7.15 auf 4.37.

Die Konzentration von $H_2O_2$ wurde für die erste und die dritte Therapievorrichtung (TV1, TV3) mittels einer photometrischen Probe mittels dem kommerziell erhältlichen Amplex Red Reagenz (10-acetyl-3,7 - dihydroxyphenoaxazin, Molekülformel $C_{14}H_{11}NO_4$, CAS-Name/Nr: 10H-Phenoxazine-3,7-diol, 10-acteyl -119171-73-2, Molekulargewicht 257.25) ermittelt. Eine Farbreaktion zeigt die Präsenz von $H_2O_2$ an. Die Absorption wurde photometrisch bei einer Wellenlänge von 535 nm mittels eines Infinite ® M200 PRO Tecan Mikroplattenphotometers quantifiziert. Die Messungen erfolgten vierfach (n=4) für die HI und LO Einstellungen der Therapievorrichtung (TV1) mit Ausnahme des Vergleichs der Elektroden für welche nur eine HI Einstellung für n=3 verwendet worden ist.

Fig. 18a zeigt die Gesamtkonzentration an $H_2O_2$ in [$\mu M$], aufgetragen auf der Ordinate, in Abhängigkeit von der Behandlungsdauer, aufgetragen auf der Abszisse. Die Messungen wurden nur für die HI Einstellungen der ersten Therapievorrichtung (TV1) dokumentiert, für die LO Einstellungen lagen die Konzentrationen an $H_2O_2$ unterhalb der Detektionsschwelle und ausserhalb des untersten Standardpunkts. Der jeweils linksseitige Balken (schwarz) entspricht dem HzOz-Messwert für HzO, der korrespondierende rechtsseitige Balken (grau) entspricht dem korrespondierenden HzOz-Messwert für NaCl. Die Gesamtkonzentration nahm bei zunehmender Behandlungsdauer sowohl für $H_2O$ als auch für NaCl zu. Die Konzentration von $H_2O_2$ in Wasser lag nach 10 s bei 3 $\mu M$ mit einer Standardabweichung von 0.1 ppm und erreichte 44.35 $\mu M$ mit einer Standardabweichung von 1.51 ppm nach 300 s. Die Konzentration von $H_2O_2$ in NaCl lag nach 10 s bei 2.58 $\mu M$ mit einer Standardabweichung von 0.08 ppm und erreichte 42 $\mu M$ mit einer Standardabweichung von 1.43 ppm nach 300 s. Die Konzentrationen in NaCl scheinen tiefer zu liegen als in Wasser, die Abweichungen lagen aber im Rahmen der Standardabweichungen.

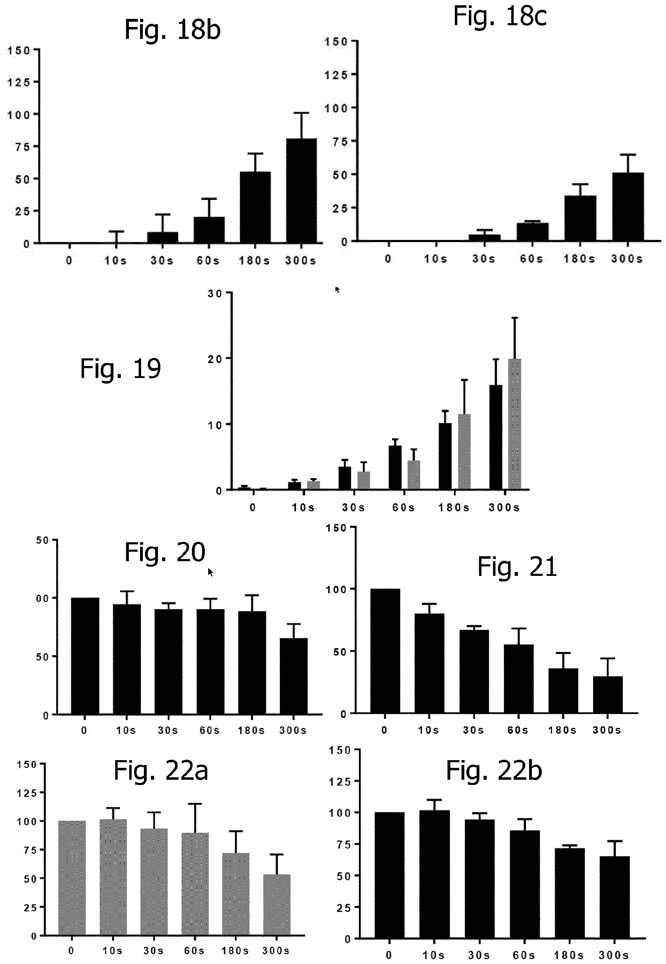

Fig. 18b zeigt die Konzentration an $H_2O_2$ in [$\mu M$], aufgetragen auf der Ordinate, in Abhängigkeit von der Behandlungsdauer, aufgetragen auf der Abszisse in Wasser für die zweite Therapievorrichtung (TV2). Für die zweite Therapievorrichtung wurden die $H_2O_2$ Konzentrationen mittels einer photometrischen Versuchsreihe basierend auf Titanlyl(IV) oxysulfat ($TiOSO_4$) ermittelt. $TiOSO_4$ reagiert in der Gegenwart von $H_2O_2$ zu einem gelb-orangen Komplex. Die Absorption wurde photometrisch bei einer Wellenlänge von 407 nm mittels eines Infinite ® M200 PRO Tecan Mikroplattenphotometers quantifiziert. Die Messungen erfolgten vierfach (n=4). Die Konzentrationen wurden gemäss einer Standardkurve für $H_2O_2$ bei unterschiedlichen Verdünnungen ermittelt. Bei sehr kurzen Expositionszeiten (0 s, 10 s) waren die Werte unter dem Detektionslimit und daher nicht in den Berechnungen und sind auch nicht in Tabelle 2 enthalten.

Tabelle 2

| t | NaCl | | | H$_2$O | | |
|---|---|---|---|---|---|---|
| | MW | SD | n | MW | SD | n |
| 30 s | 4.86 | 3.28 | 3 | 19.56 | 8.31 | 2 |
| 60 s | 13.56 | 1.19 | 2 | 20.28 | 13.98 | 4 |
| 180 s | 33.89 | 8.61 | 4 | 55.27 | 14.13 | 4 |
| 300 s | 51.17 | 13.47 | 4 | 81.04 | 19.89 | 4 |

Die Konzentrationen erhöhten sich mit zunehmender Behandlungsdauer in Wasser, wie in Fig. 18e gezeigt ist, als auch in NaCl.. Nach kurzen Behandlungsdauern von 10 s oder 30 s liegt die Konzentration von H$_2$O$_2$ im Bereich des Detektionslimits der Versuchsvorrichtung (< 5$\mu$M). Die Standardabweichungen sind daher höher für eine Behandlungsdauer von > 60 s. Die maximale Konzentration war in Wasser (81.04 $\mu$M) höher als in NaCl (51.17) bei einer Behandlungsdauer von 300s.

Fig. 18c zeigt die Konzentration an H$_2$O$_2$ in [$\mu$M], aufgetragen auf der Ordinate, in Abhängigkeit von der Behandlungsdauer, aufgetragen auf der Abszisse, in NaCl für die zweite Therapievorrichtung (TV2).

Fig. 18d zeigt die Konzentration an H$_2$O$_2$ in [$\mu$M], aufgetragen auf der Ordinate, in Abhängigkeit von der Behandlungsdauer, aufgetragen auf der Abszisse, in Wasser für die erste Elektrode der dritten Therapievorrichtung (TV3) und Fig. 18d die Konzentration an H$_2$O$_2$ für die zweite Elektrode der dritten Therapievorrichtung. Für beide Elektroden erfolgte eine Anreicherung an H$_2$O$_2$ in Abhängigkeit von der Behandlungsdauer, wobei für die erste Elektrode eine Konzentration von 30 $\mu$M (1.015 ppm) und für die zweite Elektrode eine Konzentration von 18.34 $\mu$M (0.624ppm) nach 300 s Behandlungsdauer erreicht wurde.

Für die Bestimmung von Nitriten und Nitraten wurde ein kolorimetrisches Reagenz verwendet (Griess assay; Cayment chemicals), wobei die Bestimmung mittels einer Mikrotiterplatte durchgeführt worden ist. Um die gesamte Nitrat/Nitrit-Konzentration zu messen, wird in einem ersten Schritt Nitrat zu Nitrit mittels Nitratreduktase umgewandelt und in einem zweiten Schritt das Nitrit in unter Zugabe des Griess-Reagenz eine dunkellila Azoverbindung umgewandelt, wobei das Nitrit bestimmt wurde, ohne dass eine Umwandlung der Nitratreduktase erfolgt. Standardkurven für beide Verbindungen wurden im Prüfverfahren eingeschlossen. Eine photometrische Messung des Absorptionskoeffizienten bei einer Wellenlänge von 540 nm mittels des Infinite ® M200 PRO Tecan Mikroplattenphotometers ermittelt die genauen Konzentrationen von Nitrit und Nitrat. Die Messungen wurden für die erste Therapievorrichtung (TV1) zweimal mit n=3, für die zweite Therapievorrichtung (TV2) zweimal mit n=5 wiederholt.

Fig. 19 zeigt die Gesamtkonzentration an NO$_2^-$ und/oder NO$_3^-$ in [$\mu$M], aufgetragen auf der Ordinate, in Abhängigkeit von der Behandlungsdauer, aufgetragen auf der Abszisse für die erste Therapievorrichtung (TV1). Die Messungen wurden nur für die HI Einstellungen der Therapievorrichtung 20 dokumentiert, für die LO Einstellungen lagen die Konzentrationen an NO$_2^-$ und NO$_3^-$ unterhalb der Detektionsschwelle und ausserhalb des untersten Standardpunkts. In Fig. 19a entspricht jeweils linksseitige Balken (grau) dem NO$_2^-$-Messwert in HzO, der korrespondierende rechtsseitige Balken (schwarz) entspricht dem NO$_3^-$ -Messwert in HzO. Die Gesamtkonzentration nahm bei zunehmender Behandlungsdauer sowohl für NOz' als auch für NO$_3^-$ zu. Somit zeigen die Konzentrationsverläufe für die HI Einstellungen eine von der Behandlungsdauer abhängige Zunahme sowohl der Nitratkonzentration (NO$_3^-$) also auch der Nitritkonzentration (NO$_2^-$) in Wasser. Der Anteil an Nitrit ist geringer als der Anteil an Nitrat in Wasser.

In Fig. 19 entspricht jeweils linksseitige Balken (schwarz) dem NO$_2^-$-Messwert in HzO, der korrespondierende rechtsseitige Balken (grau) entspricht dem NOz' - Messwert in NaCl. Die Gesamtkonzentration nahm bei zunehmender Behandlungsdauer sowohl für NOz' in Wasser als auch für NOz' in NaCl zu. Somit zeigen die Konzentrationsverläufe für die HI Einstellungen eine von der Behandlungsdauer abhängige Zunahme der Nitritkonzentration (NO$_2^-$) sowohl in Wasser als auch in NaCl. Die Konzentration von Nitrit scheint in Wasser niedriger zu sein als in NaCl.

6. Messbeispiel

Die Cytotoxizität wurde mittels eines MTT-Tests (MTT assay) mittels der anhaftenden Hautfibroblasten-Zelllinie GM00637 ermittelt, wie in DIN SPEC 91315 beschrieben. Hierzu wird ein gelbes, wasserlösliches 3-(4,5-dimethylthiazol-2-yl)-2,5-diphenyltetrazoliumbromid (MTT) in ein blau-lila Formazan umgewandelt und der photometrische Absorptionskoeffizient überwacht, woraus auf die Lebensfähigkeit der Zellen geschlossen werden kann. Der Absorptionskoeffizient wurde mittels eines Infinite ® M200 PRO Tecan Mikroplattenphotometers bei 550 nm aufgezeichnet.

Die Zellen wurden vom Coriell Institue (Camden, New Jersey, USA) bezogen und in einem DMEM High Glucose w/L Glutamine (Corning ®) Medium aufbewahrt, welchem 10% fetales Kälberserum (fetal bovine serum, FBS; Biochrome AG, Berlin, Deutschland) und 1% Penicillin/Strepomycin (Corning ®) zugesetzt waren. Die Kultur wurde bei einer Temperatur von 37°C und 5% $CO_2$ aufbewahrt.

Am Vortag der Behandlung wurden $0.5 \times 10^5$ Zellen pro Mikrotiterplatte auf eine 24-Well-Titerplatte ausgebracht und geimpft wie vorgehend beschrieben. Die Anzahl der Zellen und die verwendeten Volumina wurden entsprechend DIN SPEC 91315 auf die 24 Well-Titerplatte angepasst, entsprechend der Grösse der Plasmaquelle der Therapievorrichtung.

Vor der Plasmabehandlung wurde das Zellkulturmedium entfernt, die Zellen wurden zweimal mit phosphatgepufferter Kochsalzlösung PBS (phosphate buffered saline, pH=7.4) gewaschen und mit 150 µl PBS bedeckt. Die Zellen wurden der Plasmaquelle 10 s, 30 s, 60 s, 180 s, 300 s ausgesetzt (in dreifach durchgeführten Messungen). Unbehandelte Zellen dienten als Referenz. Unmittelbar im Anschluss an die punktuellen Plasmabehandlungen, d.h. maximal 5 min nach der Plasmabehandlung) wurden pro Well (Vertiefung) 450 µl frisches DMEM mit 13% FBS hinzugefügt. Die Inkubation der Zellkulturplatten erfolgte für eine Zeitdauer von 48h. Im Anschluss wurde das Überstandsmedium durch frisches DMEM mit 10% FCS enthaltend 15 µl MTT Lösung (5 mg/ml in PBS) ersetzt.

Nach Ablauf von 2h wurde die MTT-Mediumslösung entfernt und die Zellen wurden zweimal mit PBS gewaschen, danach wurden 300 ml Zelllyselösung (DMSO/unverdünnte Essigsäure/SDS) zugesetzt. Abschliessend wurde der Absorptionskoeffizient überwacht und die Zytotoxizität wurde im Verhältnis zu einer unbehandelten Referenz, die 100% entspricht, ermittelt. Eine IC-50 Zeit wurde berechnet, welche 50% Lebensfähigkeit der Zellen entspricht.

In Fig. 20 sind die Resultate des MTT Tests für die erste Therapievorrichtung (TV1) dargestellt, wobei auf der Abszisse die Behandlungsdauer aufgetragen ist und auf der Ordinate die Zytotoxizität in % aufgetragen ist. Die Plasmabehandlung wurde von den Zellen gut toleriert. Nach einer Expositionsdauer von 300 s betrug die Lebensfähigkeit der Zellen noch immer 65.5 %, das heisst, die IC-50 Zeit für die Therapievorrichtung liegt oberhalb von 300 s. Für alle anderen Behandlungszeiten betrug die Lebensfähigkeit ungefähr 90% mit einer marginalen Abnahme bei zunehmender Behandlungsdauer.

In Fig. 21 sind die Resultate des MTT Tests für die zweite Therapievorrichtung (TV2) dargestellt. Die Raumtemperatur betrug 22.1 Grad Celsius und die relative Luftfeuchtigkeit lag im Mittel bei 56.6% (Bereich von 51 bis 60.3%). Auf der Abszisse ist die Behandlungsdauer aufgetragen und auf der Ordinate die Zytotoxizität in % aufgetragen ist. Mit zunehmender Behandlungsdauer nahm Lebensfähigkeit der Zellen ab, was einer Zunahme der Zytotoxizität bei erhöhter Plasmaexpositionsdauer entspricht. Die berechnete IC-50 Zeit für die zweite Therapievorrichtung lag bei 65.14 s. Nach der längsten Behandlungszeit von 300 s waren im Mittel nur noch 26.5% der Zellen vital. Die Standardabweichungen bei den Behandlungszeiten 60 s, 180 s und 300 s lagen mit > 10% relativ hoch.

Tabelle 3 zeigt die der Fig. 21 zugrundeliegenden Messungen:

Tabelle 3

| Zeit | Test 1 | Test 2 | Test 3 | MW | SD |
|---|---|---|---|---|---|
| 10 s | 71.37 | 84.53 | 84.76 | 80.217 | 7.663 |
| 30 s | 64.42 | 66.02 | 70.49 | 66.977 | 3.145 |
| 60 s | 40.98 | 58.60 | 66.19 | 55.254 | 12.935 |
| 180 s | 22.51 | 39.52 | 46.45 | 36.16 | 12.318 |
| 300 s | 19.04 | 46.00 | 23.97 | 29.67 | 14.357 |

In Fig. 22a und Fig. 22b sind die Resultate des MTT Tests für die erste und zweite Elektrode der dritten Therapievorrichtung (TV3) dargestellt. Auf der Abszisse ist die Behandlungsdauer aufgetragen und auf der Ordinate die Zytotoxizität in % aufgetragen. Nach der längsten Behandlungsdauer verringerte sich die Lebensfähigkeit der Zellen auf 53.59% (±17.22%) für die erste Elektrode und auf 65.28% (±12.05%) für die zweite Elektrode. Die IC-50 Zeit war grösser als 300 s für beide Elektroden.

7. Messbeispiel

Ein Hemmhoftest (inhibition zone assay) wurde zur Bestimmung der antimikrobiellen Wirksamkeit der Plasmaquelle in Form der LSE Elektrode gemäss DIN SPEC 91315:2014-06 verwendet. Für die Messung wurde das Bakterium Staphylococcus aureus DSM 799/ATCC 6538 sowie das Bakterium Staphylococcus epidermidis DSM 20044/ATCC 14990 (DSM German Collection of Microorganisms and Cell Cultures; ATCC American Type Culture Collection) verwendet. Für eine weitere Messung wurde das Bakterium Escherichia coli K-12 DSM 11250/NCTC 10538 (NCTC National Collection of Type Cultures) verwendet. Für eine weitere Messung wurde das Bakterium Pseudomonas aeruginosa DSM 50071/ATCC 10145 verwendet. Für eine weitere Messung wurde die Hefe Candida albicans DSM 1386/ATCC 10321 verwendet.

Für die Tests mit der LSE Elektrode wurde 100 μl einer Lösung des Bakteriums Staphylococcus aureus (Anzahl Zellen ungefähr $10^6$/ml -colony forming units/ml) auf ein feuchtes festes Medium verteilt (Sojabohnen-Kasein-Verdauungsagar Carl Roth GmbH & Co. KG, Karlsruhe, Deutschland) und punktuell mit der Plasmaquelle, der LSE-Elektrode behandelt. Die Behandlungszeit betrug 1, 2, 3, 4 oder 5 min, wobei das Inhibitionszonen Assay auf der feuchten Agaroberfläche erfolgt (N=6). Der Abstand zwischen der Plasmaquelle und der Oberfläche des feuchten Feststoffs betrug ungefähr 1.5 mm. Unter der Agarplatte wurde eine Kathode positioniert.

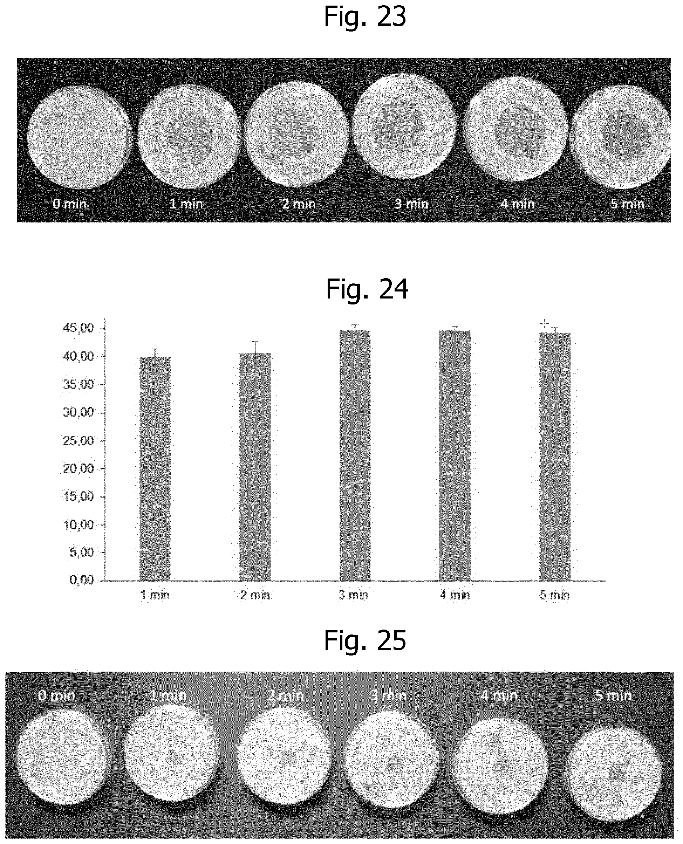

Nach einer Inkubation der Agarplatten mit einem Durchmesser von 84 mm bei einer Temperatur von 37° C wurden die Abmessungen der Hemmhöfe (growth inhibition zones) in mm gemessen, wobei die Hemmhöfe als Fläche ohne sichtbares Wachstum von Mikroorganismen definiert werden. Wenn die Hemmhof nicht kreisförmig war, wurde der mittlere Durchmesser aus den Messungen des grössten und kleinsten Durchmessers ermittelt. Zum Vergleich wurden Agarplatten geimpft, aber keiner Plasmabehandlung unterworfen. Sie sind in Fig. 23 zu den Zeiten t=0 min bis t=5 min gezeigt.

Tabelle 4a

|   | 1 min |       | DM [mm] | 2 min |       | DM [mm] |
|---|-------|-------|---------|-------|-------|---------|
| 1 | 35,00 | 40,00 | 37,50   | 40,00 | 45,00 | 42,50   |
| 2 | 41,00 | 42,00 | 41,50   | 42,00 | 43,00 | 42,50   |
| 3 | 38,00 | 41,00 | 39,50   | 41,00 | 42,00 | 41,50   |
| 4 | 40,00 | 42,00 | 41,00   | 39,00 | 40,00 | 39,50   |
| 5 | 40,00 | 41,00 | 40,50   | 36,00 | 38,00 | 37,00   |
| 6 | 39,00 | 40,00 | 39,50   | 40,00 | 41,00 | 40,50   |
| MW |      |       | 39,92   |       |       | 40,58   |
| SD |      |       | 1,43    |       |       | 2,11    |

Tabelle 4b

|   | 3 min |       | DM [mm] | 4 min |       | DM [mm] |
|---|-------|-------|---------|-------|-------|---------|
| 1 | 44,00 | 45,00 | 44,50   | 42,00 | 45,00 | 43,50   |
| 2 | 42,00 | 44,00 | 43,00   | 45,00 | 45,00 | 45,00   |
| 3 | 43,00 | 45,00 | 44,00   | 45,00 | 46,00 | 45,50   |
| 4 | 44,00 | 45,00 | 44,50   | 44,00 | 45,00 | 44,50   |
| 5 | 45,00 | 48,00 | 46,50   | 43,00 | 45,00 | 44,00   |
| 6 | 45,00 | 45,00 | 45,00   | 45,00 | 45,00 | 45,00   |
| MW |      |       | 44,58   |       |       | 44,58   |
| SD |      |       | 1,16    |       |       | 0,74    |

Tabelle 4c

|   | 5 min |       | DM [mm] |
|---|-------|-------|---------|
| 1 | 42,00 | 45,00 | 43,50   |
| 2 | 45,00 | 45,00 | 45,00   |
| 3 | 43,00 | 44,00 | 43,50   |
| 4 | 43,00 | 43,00 | 43,00   |
| 5 | 45,00 | 44,00 | 44,50   |

(fortgesetzt)

|  | 5 min |  | DM [mm] |
| --- | --- | --- | --- |
| 6 | 45,00 | 46,00 | 45,50 |
| MW |  |  | 44,17 |
| SD |  |  | 0,98 |

Die Behandlung der beschriebenen mit Bakterien gemäss obigen Ausführungen versetzten Agarplatten mit der Plasmaquelle der LSE Elektrode der Therapievorrichtung ergab somit Hemmhöfe für Staphylococcus aureus. Die Grösse der Zonen war abhängig von der Behandlungsdauer. Es wurden jeweils 6 Agarplatten 1 min, 2 min, 3 min, 4 min, 5 min mit der Plasmaquelle behandelt. Ein exemplarisches Beispiel ist in Fig. 23 gezeigt.

Fig. 24 ist eine graphische Darstellung der Mittelwerte (MW) gemäss Tabelle 4a bis Tabelle 4c sowie der zugehörigen Standardabweichungen (SD) in einem Balkendiagramm, in welchem auf der Abszisse die Behandlungszeiten und auf der Ordinate die mittleren Durchmesser in mm aufgetragen sind.

Die Grösse des Hemmhofs vergrösserte sich bei zunehmender Behandlungsdauer nur teilweise. Daher war der Einfluss auf die Behandlungszeit auf die Grösse des Hemmhofs weniger ausgeprägt als für die Bildung von ROS, siehe hierzu insbesondere Fig. 22. Die antimikrobielle Fläche war geringfügig grösser als die korrespondierende Fläche, die durch die ROS-Bildung entstanden war, sodass zusätzliche antimikrobielle Wirkungen angenommen werden können. Allerdings nahm die Anzahl der verbleibenden Kolonien mit zunehmender Behandlungsdauer innerhalb des Hemmhofs stetig ab.

Im Vergleich hierzu zeigten die Messungen mit EWC Elektroden Hemmhöfe mit Durchmessern von 14 mm bei 1 min Behandlungsdauer bis 16 mm bei 5 min Behandlungsdauer für Staphylococcus aureus.

Für die Tests der ersten Therapievorrichtung (TV1) wurde 100 $\mu$l der entsprechenden Mikroorganismus-Lösung (Anzahl Zellen ungefähr $10^6$/ml -colony forming units/ml) auf ein feuchtes festes Medium verteilt (Sojabohnen-Kasein-Verdauungsagar Carl Roth GmbH & Co. KG, Karlsruhe, Deutschland) und punktuell mit der Plasmaquelle, der EWC-Elektrode behandelt. Die Behandlungszeit betrug 1, 2, 3, 4 oder 5 min, wobei der Hemmhoftest auf der feuchten Agaroberfläche erfolgt (N=6). Der Abstand zwischen der Plasmaquelle und der Oberfläche des feuchten Feststoffs betrug ungefähr 1.5 mm. Unter dem Agarplatten wurde eine Kathode positioniert.

Nach einer Inkubation der Agarplatten mit einem Durchmesser von 84 mm bei einer Temperatur von 37° C wurden die Abmessungen der Hemmhöfe (growth inhibition zones) in mm gemessen, wobei die Hemmhöfe als Fläche ohne sichtbares Wachstum von Mikroorganismen definiert werden. Wenn der Hemmhof nicht kreisförmig war, wurde der mittlere Durchmesser aus den Messungen des grössten und kleinsten Durchmessers ermittelt. Zum Vergleich wurden Agarplatten geimpft, aber keiner Plasmabehandlung unterworfen. Für das Bakterium Staphylococcus aureus sind sie in Fig. 25 zu den Zeiten t=0 min bis t=5 min gezeigt.

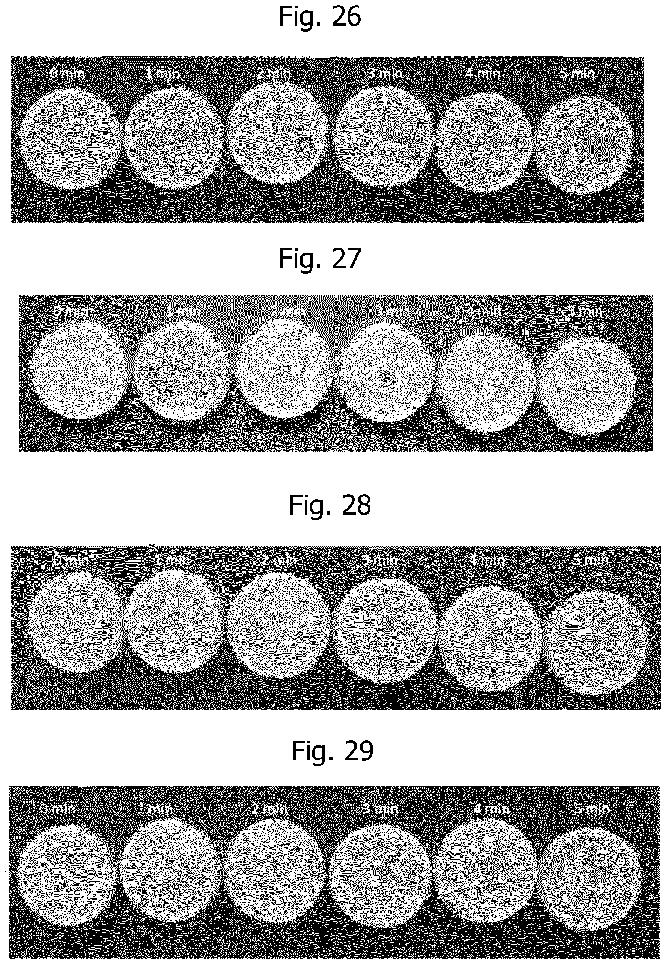

Für das Bakterium Staphylococcus epidermidis sind sie in Fig. 26 zu den Zeiten t=0 min bis t=5 min gezeigt.

Für das Bakterium Escherichia coli sind sie in Fig. 27 zu den Zeiten t=0 min bis t=5 min gezeigt.

Für das Bakterium Pseudomonas aeruginosa sind sie in Fig. 28 zu den Zeiten t=0 min bis t=5 min gezeigt.

Für die Hefe Candida albicans sind sie in Fig. 29 zu den Zeiten t=0 min bis t=5 min gezeigt.

Die nachfolgende Tabelle 5a, 5b, 5c zeigt die Messwerte für das Bakterium Staphylococcus aureus zu den Zeiten t=0 min bis t=5 min bei 25.4 °C und einer relativen Luftfeuchtigkeit von 46 %.

Tabelle 5a

|  | 1 min |  | DM [mm] | 2 min |  | DM [mm] |
| --- | --- | --- | --- | --- | --- | --- |
| 1 | 14,50 | 13,00 | 13,75 | 16,50 | 14,00 | 15,25 |
| 2 | 16,00 | 14,00 | 15,00 | 17,00 | 15,00 | 16,00 |
| 3 | 16,50 | 15,00 | 15,75 | 16,50 | 14,00 | 15,25 |
| 4 | 15,50 | 15,00 | 15,25 | 17,50 | 13,00 | 15,25 |
| 5 | 17,00 | 15,00 | 16,00 | 19,50 | 19,00 | 19,25 |
| 6 | 15,50 | 14,00 | 14,75 | 17,50 | 16,00 | 16,75 |
| MW |  |  | 15,08 |  |  | 16,29 |
| SD |  |  | 0,80 |  |  | 1,57 |

Tabelle 5b

|  | 3 min |  | DM [mm] | 4 min |  | DM [mm] |
|---|---|---|---|---|---|---|
| 1 | 18,50 | 16,00 | 17,25 | 20,00 | 16,00 | 18,00 |
| 2 | 18,00 | 14,00 | 16,00 | 19,50 | 17,00 | 18,25 |
| 3 | 19,00 | 15,00 | 17,00 | 18,50 | 16,00 | 17,25 |
| 4 | 18,50 | 16,00 | 17,25 | 21,50 | 17,00 | 19,25 |
| 5 | 19,50 | 16,00 | 17,75 | 20,50 | 18,00 | 19,25 |
| 6 | 19,50 | 16,00 | 17,75 | 19,00 | 16,00 | 17,50 |
| MW |  |  | 17,17 |  |  | 18,25 |
| SD |  |  | 0,65 |  |  | 0,85 |

Tabelle 5c

|  | 5 min |  | DM [mm] |
|---|---|---|---|
| 1 | 19,00 | 16,00 | 17,50 |
| 2 | 20,00 | 16,00 | 18,00 |
| 3 | 19,50 | 16,00 | 17,75 |
| 4 | 19,00 | 17,00 | 18,00 |
| 5 | 20,50 | 17,00 | 18,75 |
| 6 | 22,00 | 18,00 | 20,00 |
| MW |  |  | 18,33 |
| SD |  |  | 0,92 |

Die nachfolgende Tabelle 6a, 6b, 6c zeigt die Messwerte für das Bakterium Staphylococcus epidermidis zu den Zeiten t=0 min bis t=5 min bei 25.6 °C und einer relativen Luftfeuchtigkeit von 44 %.

Tabelle 6a

|  | 1 min |  | DM [mm] | 2 min |  | DM [mm] |
|---|---|---|---|---|---|---|
| 1 | 19,00 | 17,00 | 18,00 | 24,00 | 23,00 | 23,50 |
| 2 | 17,00 | 15,00 | 16,00 | 20,00 | 16,00 | 18,00 |
| 3 | 18,00 | 15,00 | 16,50 | 21,50 | 19,00 | 20,25 |
| 4 | 17,00 | 12,00 | 14,50 | 22,00 | 19,00 | 20,50 |
| 5 | 17,00 | 13,00 | 15,00 | 20,00 | 20,00 | 20,00 |
| 6 | 18,00 | 13,00 | 15,50 | 21,00 | 17,00 | 19,00 |
| MW |  |  | 15,92 |  |  | 20,21 |
| SD |  |  | 1,24 |  |  | 1,86 |

Tabelle 6b

|  | 3 min |  | DM [mm] | 4 min |  | DM [mm] |
|---|---|---|---|---|---|---|
| 1 | 21,00 | 19,00 | 20,00 | 26,00 | 23,00 | 24,50 |
| 2 | 28,50 | 23,00 | 25,75 | 29,50 | 30,00 | 29,75 |

(fortgesetzt)

| | 3 min | | DM [mm] | 4 min | | DM [mm] |
|---|---|---|---|---|---|---|
| 3 | 27,50 | 23,00 | 25,25 | 25,00 | 25,00 | 25,00 |
| 4 | 20,50 | 17,00 | 18,75 | 29,00 | 29,00 | 29,00 |
| 5 | 24,00 | 21,00 | 22,50 | 26,00 | 25,00 | 25,50 |
| 6 | 22,50 | 20,00 | 21,25 | 23,00 | 20,00 | 21,50 |
| MW | | | 22,25 | | | 25,88 |
| SD | | | 2,82 | | | 3,06 |

Tabelle 6c

| | 5 min | | DM [mm] |
|---|---|---|---|
| 1 | 23,00 | 23,00 | 23,00 |
| 2 | 23,00 | 19,00 | 21,00 |
| 3 | 25,00 | 25,00 | 25,00 |
| 4 | 25,00 | 23,00 | 24,00 |
| 5 | 27,00 | 25,00 | 26,00 |
| 6 | 23,50 | 20,00 | 21,75 |
| MW | | | 23,46 |
| SD | | | 1,91 |

Die nachfolgende Tabelle 7a, 7b, 7c zeigt die Messwerte für das Bakterium Escherichia coli zu den Zeiten t=0 min bis t=5 min bei 24.5 °C und einer relativen Luftfeuchtigkeit von 49 %.

Tabelle 7a

| | 1 min | | DM [mm] | 2 min | | DM [mm] |
|---|---|---|---|---|---|---|
| 1 | 16,50 | 10,00 | 13,25 | 15,50 | 11,00 | 13,25 |
| 2 | 14,00 | 11,00 | 12,50 | 16,00 | 12,00 | 14,00 |
| 3 | 14,50 | 11,00 | 12,75 | 15,00 | 12,00 | 13,50 |
| 4 | 15,00 | 11,00 | 13,00 | 16,50 | 13,00 | 14,75 |
| 5 | 13,50 | 9,00 | 11,25 | 15,00 | 11,00 | 13,00 |
| 6 | 15,00 | 10,00 | 12,50 | 16,00 | 13,00 | 14,50 |
| MW | | | 15,92 | | | 20,21 |
| SD | | | 1,24 | | | 1,86 |

Tabelle 7b

| | 3 min | | DM [mm] | 4 min | | DM [mm] |
|---|---|---|---|---|---|---|
| 1 | 16,50 | 14,00 | 15,25 | 16,50 | 13,00 | 14,75 |
| 2 | 17,00 | 15,00 | 16,00 | 17,00 | 13,00 | 15,00 |
| 3 | 16,00 | 11,00 | 13,50 | 16,50 | 14,00 | 15,25 |
| 4 | 16,50 | 13,00 | 14,75 | 18,50 | 16,00 | 17,25 |

(fortgesetzt)

|  | 3 min |  | DM [mm] | 4 min |  | DM [mm] |
|---|---|---|---|---|---|---|
| 5 | 16,50 | 14,00 | 15,25 | 17,00 | 13,00 | 15,00 |
| 6 | 17,00 | 14,00 | 15,50 | 17,00 | 14,00 | 15,50 |
| MW |  |  | 22,25 |  |  | 25,88 |
| SD |  |  | 2,82 |  |  | 3,06 |

Tabelle 7c

|  | 5 min |  | DM [mm] |
|---|---|---|---|
| 1 | 15,50 | 11,00 | 13,25 |
| 2 | 18,50 | 14,00 | 16,25 |
| 3 | 16,50 | 12,00 | 14,25 |
| 4 | 16,50 | 13,00 | 14,75 |
| 5 | 16,00 | 12,00 | 14,00 |
| 6 | 17,00 | 16,00 | 16,50 |
| MW |  |  | 23,46 |
| SD |  |  | 1,91 |

Die nachfolgende Tabelle 8a, 8b, 8c zeigt die Messwerte für das Bakterium Pseudomonas aeruginosa zu den Zeiten t=0 min bis t=5 min bei 25.6 °C und einer relativen Luftfeuchtigkeit von 44 %.

Tabelle 8a

|  | 1 min |  | DM [mm] | 2 min |  | DM [mm] |
|---|---|---|---|---|---|---|
| 1 | 10,00 | 9,00 | 9,50 | 10,00 | 9,00 | 9,50 |
| 2 | 11,50 | 10,00 | 10,75 | 11,50 | 10,00 | 10,75 |
| 3 | 11,00 | 10,00 | 10,50 | 11,00 | 10,00 | 10,50 |
| 4 | 11,00 | 9,00 | 10,00 | 11,00 | 9,00 | 10,00 |
| 5 | 10,00 | 9,00 | 9,50 | 10,00 | 9,00 | 9,50 |
| 6 | 10,00 | 9,00 | 9,50 | 10,00 | 9,00 | 9,50 |
| MW |  |  | 9,96 |  |  | 9,96 |
| SD |  |  | 0,56 |  |  | 0,56 |

Tabelle 8b

|  | 3 min |  | DM [mm] | 4 min |  | DM [mm] |
|---|---|---|---|---|---|---|
| 1 | 15,00 | 11,00 | 13,00 | 14,00 | 11,00 | 12,50 |
| 2 | 14,50 | 11,00 | 12,75 | 14,50 | 11,00 | 12,75 |
| 3 | 14,00 | 11,00 | 12,50 | 14,50 | 12,00 | 13,25 |
| 4 | 15,00 | 11,00 | 13,00 | 12,00 | 11,00 | 11,50 |
| 5 | 14,00 | 11,00 | 12,50 | 12,00 | 10,00 | 11,00 |
| 6 | 14,00 | 10,00 | 12,00 | 13,00 | 12,00 | 12,50 |

(fortgesetzt)

|  | 3 min |  | DM [mm] | 4 min |  | DM [mm] |
|---|---|---|---|---|---|---|
| MW |  |  | 12,63 |  |  | 12,25 |
| SD |  |  | 0,38 |  |  | 0,84 |

Tabelle 8c

|  | 5 min |  | DM [mm] |
|---|---|---|---|
| 1 | 12,00 | 9,00 | 10,50 |
| 2 | 13,00 | 10,00 | 11,50 |
| 3 | 13,00 | 10,00 | 11,50 |
| 4 | 14,00 | 11,00 | 12,50 |
| 5 | 13,00 | 10,00 | 11,50 |
| 6 | 14,00 | 11,00 | 12,50 |
| MW |  |  | 11,67 |
| SD |  |  | 0,75 |

Die nachfolgende Tabelle 9a, 9b, 9c zeigt die Messwerte für den Mikroorganismus Candida albicans zu den Zeiten t=0 min bis t=5 min bei 25.1 °C und einer relativen Luftfeuchtigkeit von 47 %.

Tabelle 9a

|  | 1 min |  | DM [mm] | 2 min |  | DM [mm] |
|---|---|---|---|---|---|---|
| 1 | 11,50 | 9,00 | 10,25 | 14,50 | 11,00 | 12,75 |
| 2 | 13,00 | 10,00 | 11,50 | 16,00 | 13,00 | 14,50 |
| 3 | 12,50 | 10,00 | 11,25 | 15,00 | 12,00 | 13,50 |
| 4 | 11,00 | 9,00 | 10,00 | 16,00 | 14,00 | 15,00 |
| 5 | 12,00 | 10,00 | 11,00 | 15,00 | 12,00 | 13,50 |
| 6 | 12,00 | 10,00 | 11,00 | 15,50 | 11,00 | 13,25 |
| MW |  |  | 10,83 |  |  | 13,75 |
| SD |  |  | 0,58 |  |  | 0,84 |

Tabelle 9b

|  | 3 min |  | DM [mm] | 4 min |  | DM [mm] |
|---|---|---|---|---|---|---|
| 1 | 17,50 | 14,00 | 15,75 | 17,00 | 12,00 | 14,50 |
| 2 | 17,00 | 14,00 | 15,50 | 20,00 | 17,00 | 18,50 |
| 3 | 18,00 | 14,00 | 16,00 | 17,00 | 14,00 | 15,50 |
| 4 | 17,00 | 13,00 | 15,00 | 20,00 | 15,00 | 17,50 |
| 5 | 16,50 | 11,00 | 13,75 | 19,00 | 14,00 | 16,50 |
| 6 | 16,00 | 13,00 | 14,50 | 17,00 | 12,00 | 14,50 |
| MW |  |  | 15,08 |  |  | 16,17 |
| SD |  |  | 0,85 |  |  | 1,63 |

Tabelle 9c

|  | 5 min |  | DM [mm] |
|---|---|---|---|
| 1 | 16,00 | 12,00 | 14,00 |
| 2 | 16,00 | 12,00 | 14,00 |
| 3 | 16,00 | 13,00 | 14,50 |
| 4 | 18,50 | 14,00 | 16,25 |
| 5 | 17,00 | 12,00 | 14,50 |
| 6 | 19,00 | 14,00 | 16,50 |
| MW |  |  | 14,96 |
| SD |  |  | 1,12 |

Die grammpositiven Bakterien Staphylococcus aureus (15.08 - 18.33 mm) und Staphylococcus epidermidis (15.92-25.88 mm) wiesen die höchsten Hemmhofdurchmesser auf, während die grammnegativen Stämme Escherichia coli (12.54 - 15.46 mm) und Pseudomonas aeruginosa (9.96 - 12.63 mm) und die Hefe Candida albicans (10.83 - 16.17 mm) durch die Plasmabehandlung mit der EWC Elektrode weniger stark beeinflusst wurden.

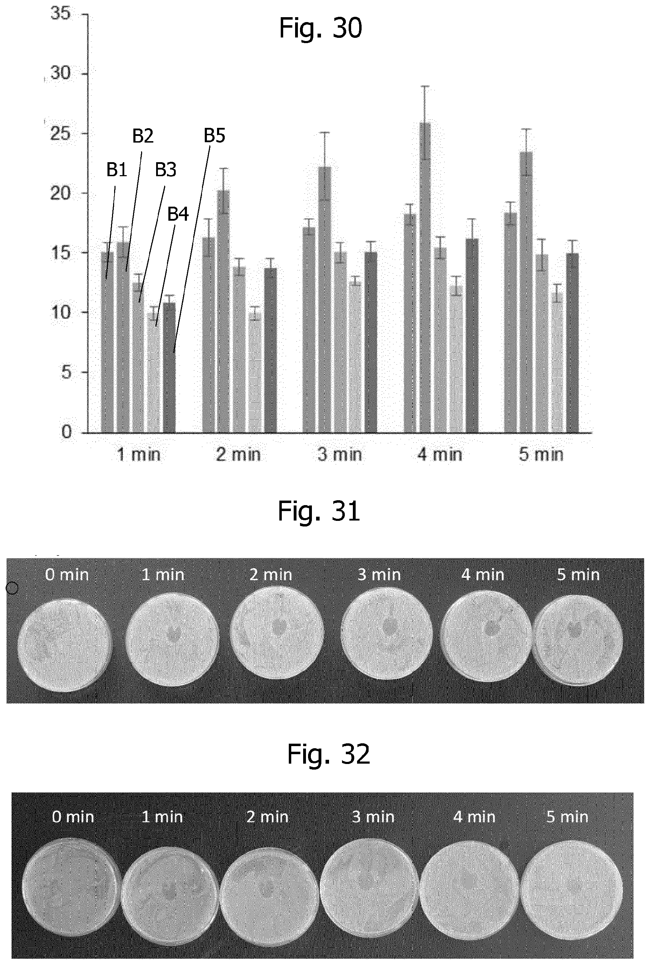

Fig. 30 zeigt einen Vergleich der Durchmesserwerte für die Bakterien Staphylococcus aureus (Balken B1), Staphylococcus epidermidis (Balken B2), Escherichia coli (Balken B3), Pseudomonas aeruginosa (Balken B4) sowie der Hefe Candida albicans (Balken B5). Auf der Abszisse wurde die Behandlungsdauer in min aufgetragen, auf der Ordinate der Hemmhofdurchmesser in mm. Für jede Testserie wurde eine Abnahme der antimikrobiellen Effizienz in der nachfolgenden Reihenfolge festgestellt:

Staphylococcus epidermidis > Staphylococcus aureus > Escherichia coli = Candida albicans > Pseudomonas aeruginosa.

Die Grösse des Hemmhofs vergrösserte sich mit zunehmender Behandlungsdauer teilweise.

Wie für die erste Therapievorrichtung (TV1) wurden Hemmhoftests für die zweite Therapievorrichtung (TV2) für dieselben Mikroorganismen durchgeführt.

Für das Bakterium Staphylococcus aureus sind sie in Fig. 31 zu den Zeiten t=0 min bis t=5 min gezeigt.

Für das Bakterium Staphylococcus epidermidis sind sie in Fig. 32 zu den Zeiten t=0 min bis t=5 min gezeigt.

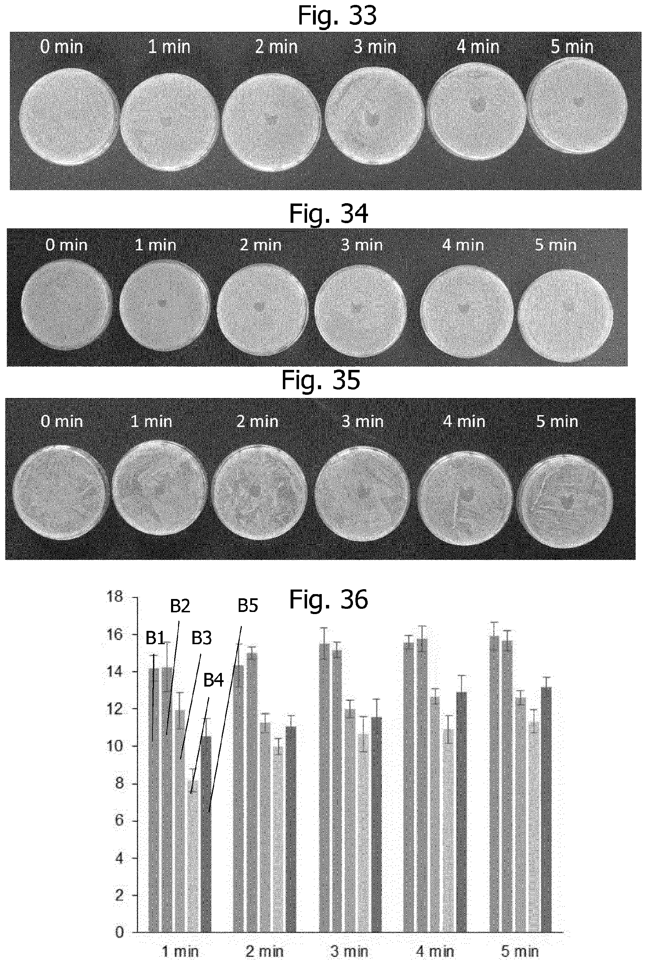

Für das Bakterium Escherichia coli sind sie in Fig. 33 zu den Zeiten t=0 min bis t=5 min gezeigt.

Für das Bakterium Pseudomonas aeruginosa sind sie in Fig. 34 zu den Zeiten t=0 min bis t=5 min gezeigt.

Für die Hefe Candida albicans sind sie in Fig. 35 zu den Zeiten t=0 min bis t=5 min gezeigt.

Fig. 36 zeigt einen Vergleich der Durchmesserwerte für die Bakterien Staphylococcus aureus (Balken B1), Staphylococcus epidermidis (Balken B2), Escherichia coli (Balken B3), Pseudomonas aeruginosa (Balken B4) sowie der Hefe Candida albicans (Balken B5). Auf der Abszisse wurde die Behandlungsdauer in min aufgetragen, auf der Ordinate der Hemmhofdurchmesser in mm. Die grammpositiven Bakterien Staphylococcus aureus (14.17 - 15.92 mm) und Staphylococcus epidermidis (14.25-15.67 mm) wiesen die höchsten Hemmhofdurchmesser auf, während die grammnegativen Stämme Escherichia coli (11.92 - 12.58 mm) und Pseudomonas aeruginosa (8.17 - 11.33mm) und die Hefe Candida albicans (10.54 - 13.17 mm) durch die Plasmabehandlung mit der EWC Elektrode weniger stark beeinflusst wurden.

Für jede Testserie wurde eine Abnahme der antimikrobiellen Effizienz in der nachfolgenden Reihenfolge festgestellt:

Staphylococcus epidermidis = Staphylococcus aureus > Escherichia coli $\geq$ Candida albicans > Pseudomonas aeruginosa.

Die Grösse des Hemmhofs vergrösserte sich mit zunehmender Behandlungsdauer. In dem Zeitraum von 1 min bis 5 min wurde eine Zunahme von 5% für das Bakterium Escherichia coli bis zu 28% für das Bakterium Pseudomonas aeruginosa festgestellt.

Wie für die erste Therapievorrichtung (TV1) wurden Hemmhoftests für die dritte Therapievorrichtung (TV3) für dieselben Mikroorganismen durchgeführt.

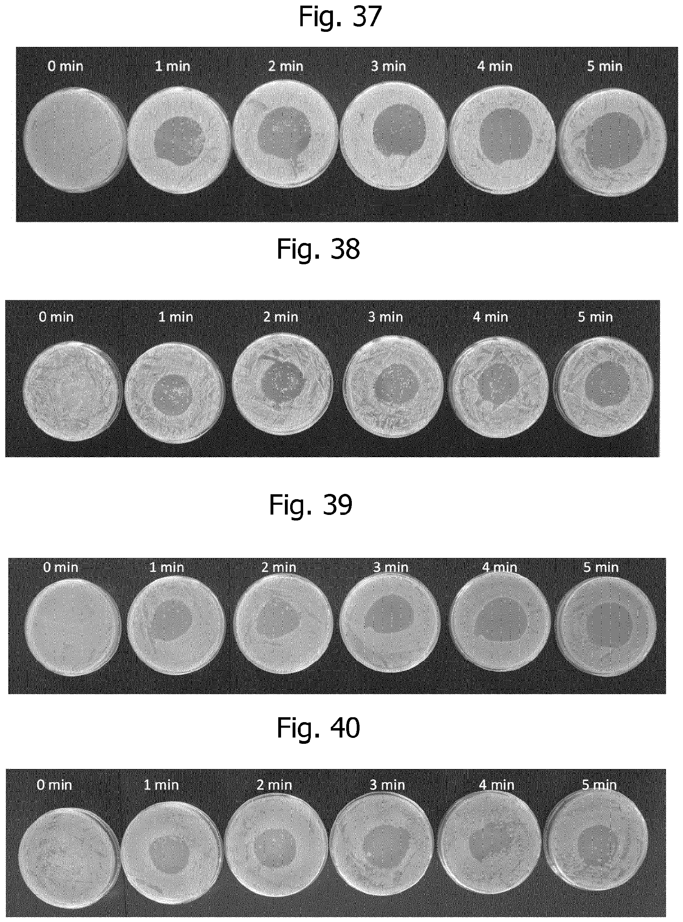

Für das Bakterium Staphylococcus aureus sind sie in Fig. 37 zu den Zeiten t=0 min bis t=5 min gezeigt.

Für das Bakterium Staphylococcus epidermidis sind sie in Fig. 38 zu den Zeiten t=0 min bis t=5 min gezeigt.

Für das Bakterium Escherichia coli sind sie in Fig. 39 zu den Zeiten t=0 min bis t=5 min gezeigt.

Für das Bakterium Pseudomonas aeruginosa sind sie in Fig. 40 zu den Zeiten t=0 min bis t=5 min gezeigt.

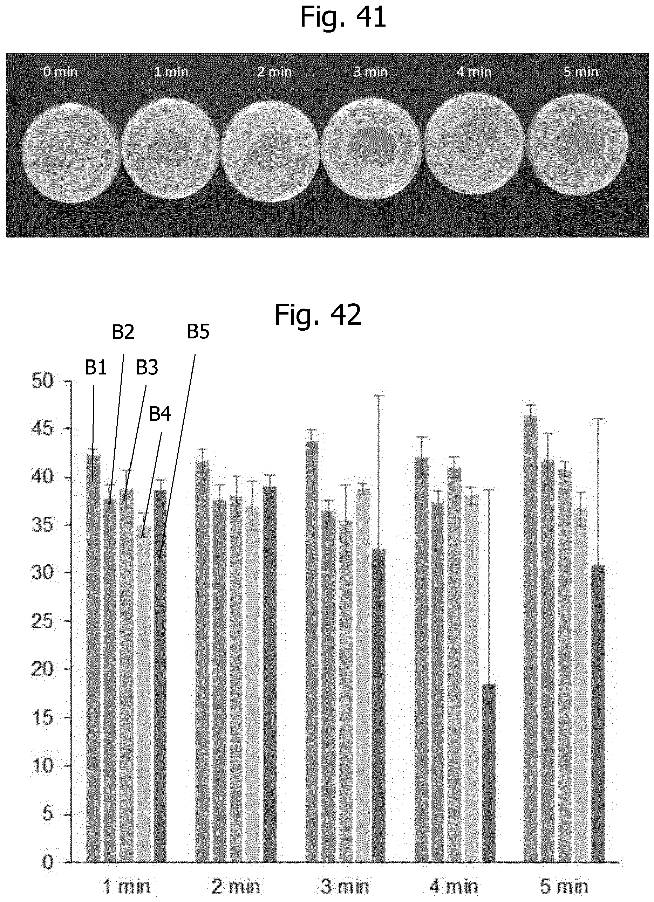

Für die Hefe Candida albicans sind sie in Fig. 41 zu den Zeiten t=0 min bis t=5 min gezeigt.

Fig. 42 zeigt einen Vergleich der Durchmesserwerte für die Bakterien Staphylococcus aureus (Balken B1), Staphylococcus epidermidis (Balken B2), Escherichia coli (Balken B3), Pseudomonas aeruginosa (Balken B4) sowie der Hefe Candida albicans (Balken B5). Auf der Abszisse wurde die Behandlungsdauer in min aufgetragen, auf der Ordinate der Hemmhofdurchmesser in mm. Das grammpositive Bakterium Staphylococcus aureus (41.67 - 46.42 mm) wies die höchsten Hemmhofdurchmesser auf. Das zweite grammpositive Bakterium Staphylococcus epidermidis (36.42.25-41.83 mm) sowie die grammnegativen Stämme Escherichia coli (35.50 - 41.00 mm), Pseudomonas aeruginosa (35.00-38.75mm) und die Hefe Candida albicans (18.42 - 39.00 mm) wurden durch die Plasmabehandlung weniger stark beeinflusst. Für die Hefe Candida albicans konnte für 5 Petrischalen kein Hemmhof bei höheren Behandlungszeiten festgestellt werden (t=3,4,5 min)

Für jede Testserie wurde eine Abnahme der antimikrobiellen Effizienz in der nachfolgenden Reihenfolge festgestellt:

Staphylococcus aureus > Staphylococcus epidermidis = Escherichia coli ($\geq$ Candida albicans für 1 und 2 min)> Pseudomonas aeruginosa.

Die Grösse des Hemmhofs vergrösserte sich mit zunehmender Behandlungsdauer teilweise. Die Resultate für Staphylococcus aureus waren vergleichbar und daher konsistent.

Tabelle 10 zeigt einen Vergleich der Messergebnisse aus den obigen Messbeispielen für die ersten zweiten und dritten Therapievorrichtungen (TV1, TV2, TV3). Allfällige behördlich festgelegte Grenzwerte (L) sind in der Übersicht ebenfalls zur Orientierung enthalten. Die Resultate für den Patientenableitstrom (I), die Temperatur, die UV-Strahlung sowie die Konzentrationen der emittierten Gase liegen innerhalb der Sicherheitsgrenzwerte.

Tabelle 10

| Bsp. Nr. | Parameter | Dim. | L | TV1 (HI) | TV2 (HI) | TV3 (HI) |
|---|---|---|---|---|---|---|
| 1 | I | $\mu$A | 100 | 12 | 11 | 16 |
| 2 | $E_{eff}$ | $\mu$W/cm$^2$ | - | 0.14$\pm$0.2 | 0.16 | 0.86$\pm$0.07 |
| 2 | $t_{max}$ | h, min | - | 6h | 5h | 1h |
| 5 | $\Delta$pH(5 min) | - | - | 2.43 | 2.43 | 2.86 |
| 5 | $H_2O_2$ (5 min) | $\mu$M | - | 44.35 | 81$\pm$20 | 24.16$\pm$9.3 |
| 5 | $NO_3^-$ (5 min) | $\mu$M | - | 49.4$\pm$10.8 | 27.896$\pm$4. 36 | 24.8$\pm$11.5 |
| 5 | $NO_2^-$ (5 min) | $\mu$M | - | 16.0$\pm$3.9 | 11.67$\pm$2.2 1 | 8.82$\pm$2.1 |
| 6 | DM | IC-50 | s | - | > 300 | 65 |
| 7 | S. Aureus DM | mm | - | 17.2 | 15.92 | 44.80 |
| 7 | S. epidermidis DM | mm | - | 22.3 | 15.67 | 36.40 |
| 7 | E. Coli DM | mm | - | 15.0 | 12.58 | 35.90 |
| 7 | P. aeruginosa DM | mm | - | 12.6 | 11.33 | 38.80 |
| 7 | C. albicans DM | mm | - | 15.0 | 13.17 | 32.90 |
| 8 | IC-50 | s | - | > 300 | 65 | > 300 |

Für den Fachmann ist offensichtlich, dass viele weitere Varianten zusätzlich zu den beschriebenen Ausführungsbeispielen möglich sind, ohne vom erfinderischen Konzept abzuweichen. Der Gegenstand der Erfindung wird somit durch die vorangehende Beschreibung nicht eingeschränkt und ist durch den Schutzbereich bestimmt, der durch die Ansprüche festgelegt ist.

**Patentansprüche**

1. Therapievorrichtung zur Zellstimulation oder Zelltherapie, umfassend ein Gehäuse (12), welches eine Elektrode (1), einen Generator (3) zur Erzeugung von hochfrequenten Spannungspulsen, eine Prozessoreinheit (6) umfassend ein Steuer-, Regel- und Berechnungsmodul, eine Speichereinheit (9), mindestens ein Bedienelement (5, 15) und einen steuerbaren Modulator (4) enthält, mittels welchem der Generator (3) steuerbar ist, wobei mittels des Modulators (4) eine Spannungspulsfolge umfassend eine Mehrzahl von Spannungspulsen erzeugbar ist, wobei mittels des Modulators (4) die Frequenz und die Dauer der Spannungspulse beliebig einstellbar sind, wobei die Elektrode (1), der Generator (3), die Prozessoreinheit (6), die Speichereinheit (9), das Bedienelement (5) und der Modulator (4) im Gehäuse (12) angeordnet sind, wobei die Elektrode (1) einen Glaskörper (27) enthält, der einen Hohlraum enthält, in welchem sich ein Gas befindet, wobei die Elektrode (1) ein erstes Ende (21) aufweist, welches mit dem Modulator (4) koppelbar ist, wobei die Elektrode ein zweites kuppelförmiges Ende (22) umfasst, wobei das Gas durch die auf die Elektrode (1) übertragenen Spannungspulse in den Zustand eines ein nicht-thermischen Primärplasmas versetzbar ist, wobei ein Sekundärplasma durch Ionisierung der Luft erzeugbar ist, die sich im Umgebungsbereich des zweiten Endes (22) der Elektrode (1) befindet.

2. Therapievorrichtung nach Anspruch 1, wobei die Frequenz der Spannungspulsfolge zumindest abschnittweise nicht konstant ist.

3. Therapievorrichtung nach einem der Ansprüche 1 oder 2, wobei die Amplitude der Spannung während einer Zeitspanne t2-t1 zunimmt, während einer Zeitspanne t3-t2 konstant ist und während einer Zeitspanne t4-t3 abnimmt, wobei die Dauer der Spannungspulsfolge der Zeitspanne t4-t1 entspricht und/oder wobei die Frequenz während der Zeitspanne t2-t1 zunimmt, während der Zeitspanne t3-t2 konstant ist und während der Zeitspanne t4-t3 abnimmt.

4. Therapievorrichtung nach einem der vorhergehenden Ansprüche, wobei die maximale Frequenz im Bereich von 10 bis 100 Hz liegt.

5. Therapievorrichtung nach einem der vorhergehenden Ansprüche, wobei die Spannung am Ausgang des Modulators (4) im Bereich von 8 V bis 65 V liegt.

6. Therapievorrichtung nach einem der vorhergehenden Ansprüche, wobei die Spannung am Ausgang des Generators (3) im Bereich von 5 kV bis einschliesslich 25 kV liegt.

7. Therapievorrichtung nach einem der vorhergehenden Ansprüche, wobei eine im Gehäuse (12) angeordnete Energiespeichereinheit (10) zur Energieversorgung zum Betrieb der Therapievorrichtung vorgesehen ist, sodass die Therapievorrichtung drahtlos betreibbar ist und/oder wobei das Gehäuse (12) ein Anzeigeelement (7) enthält, mittels welchem insbesondere Therapie- und Betriebsdaten darstellbar sind.

8. Therapievorrichtung nach einem der vorhergehenden Ansprüche, wobei die Elektrode (1) einen Sensor (2) umfasst, mittels welchem der über die Elektrode (1) abgegebene Strom oder die Spannung als Messwerte erfassbar sind, wobei die Messwerte in Messdaten digitalisierbar sind, wobei die Messdaten in der Speichereinheit (9) speicherbar sind, wobei mittels des Berechnungsmoduls der Prozessoreinheit (6) die abgegebene Energie und/oder der zeitliche Verlauf der von der Elektrode (1) abgegebenen Energie ermittelbar ist.

9. Therapievorrichtung nach Anspruch 8, wobei mittels des Regelmoduls der Prozessoreinheit (6) eine Regelung des Modulators (4) anhand der Messdaten erfolgt, insbesondere zu einer Regelung einer konstanten Energieabgabe und/oder zu einer Regelung, die von der Signalform unabhängig ist, sodass eine beliebige Signalform im Generator (3) generierbar ist, beispielsweise eine Kombination von Amplituden- und Frequenzmodulation.

10. Therapievorrichtung nach einem der Ansprüche 8 oder 9, wobei die Messdaten mittels des Steuermoduls der Prozessoreinheit (6) zur Steuerung eines Therapieverlaufs einsetzbar sind.

11. Therapievorrichtung nach Anspruch 10, wobei die Messdaten in der Prozessoreinheit (6) mit einem Zeitstempel verknüpfbar sind, wobei die mit dem Zeitstempel verknüpften Messdaten in der Speichereinheit zur Speicherung des Therapieverlaufs speicherbar sind.

12. Therapievorrichtung nach einem der vorhergehenden Ansprüche, wobei das Gehäuse (12) als einer der Pole eines Kondensators zur kapazitiven Koppelung ausgebildet ist.

13. Therapievorrichtung nach einem der vorhergehenden Ansprüche, wobei die Energiespeichereinheit (10) als ein wieder aufladbares Element ausgebildet ist, typischerweise als Lithium-Ionen Element oder als Superkapazität.

14. Therapievorrichtung nach Anspruch 13, wobei die Energiespeichereinheit einen Minuspol umfasst, der als einer der Pole eines Kondensators zur kapazitiven Koppelung ausgebildet ist.

15. Therapievorrichtung nach einem der vorhergehenden Ansprüche, wobei das Gehäuse (12) eine Innenseite umfasst, die eine elektrisch leitende oder leitfähige Fläche, beispielsweise einen leitenden Kunststoff oder einen mit einem elektrisch leitfähigen Material beschichteten Kunststoff, enthält.

**Claims**

1. Therapeutic device for cell stimulation or cell therapy, comprising a housing (12) containing an electrode (1), a generator (3) for generating high-frequency voltage pulses, a processor unit (6) comprising a control, regulation and calculation module, a memory unit (9), at least one operating element (5, 15) and a controllable modulator (4) for controlling the generator (3), wherein a voltage pulse sequence comprising a plurality of voltage pulses can be generated by means of the modulator (4), wherein a frequency and duration of the voltage pulses is adjustable by means of the modulator (4), wherein the electrode (1), the generator (3), the processor unit (6), the memory unit (9), the operating element (5) and the modulator (4) are arranged in the housing (12), wherein the electrode (1) contains a glass body (27) comprising a cavity containing a gas, wherein the electrode (1) comprises a first end (21) which can be coupled to the modulator (4), wherein the electrode comprises a second dome-shaped end (22), wherein the gas is transformable into the state of a non-thermal primary plasma by the voltage pulses transmitted to the electrode (1), wherein a secondary plasma can be generated by ionizing the air present in the region surrounding the second end (22) of the electrode (1).

2. The therapeutic device of claim 1, wherein the frequency of the voltage pulse sequence is at least partially not constant.

3. The therapeutic device of one of claims 1 or 2, wherein the amplitude of the voltage increases during a period of time t2-t1, is constant during a period of time t3-t2 and decreases during a period of time t4-t3, wherein the duration of the voltage pulse sequence corresponds to the period of time t4-t1 and/or wherein the frequency increases during the period t2-t1, is constant during the period t3-t2 and decreases during the period t4-t3.

4. The therapeutic device of one of the preceding claims, wherein the maximum frequency is in the range of 10 up to and including 100 Hz.

5. The therapeutic device of one of the preceding claims, wherein the voltage at the output of the modulator (4) is in the range of 8 V up to and including 65 V.

6. The therapeutic device of one of the preceding claims, wherein the voltage at the output of the generator (3) is in the range of 5 kV up to and including 25 kV.

7. The therapeutic device of one of the preceding claims, wherein an energy storage unit (10) arranged in the housing (12) is provided for supplying energy for the operation of the therapeutic device, so that the therapeutic device can be operated wirelessly and/or wherein the housing (12) contains a display element (7) by means of which in particular therapy and operating data can be displayed.

8. The therapeutic device of one of the preceding claims, wherein the electrode (1) comprises a sensor (2) by means of which the current or the voltage emitted via the electrode (1) can be recorded as measured values, the measured values being digitizable in measured data, wherein the measured data can be stored in the memory unit (9), wherein the calculation module of the processor unit (6) is configured to determine the energy delivered and/or the time profile of the energy emitted by the electrode (1).

9. The therapeutic device of claim 8, wherein the control module of the processor unit (6) configured to control the modulator (4) on the basis of the measured data, in particular for controlling a constant energy output and/or for a control independent of the signal form, so that any desired signal form can be generated in the generator (3), for example a combination of amplitude modulation and frequency modulation.

10. The therapeutic device of one of claims 8 or 9, wherein the measured data are configured to control a course of therapy by means of the control module of the processor unit (6).

11. The therapeutic device of claim 10, wherein the measured data in the processor unit (6) can be linked to a time stamp, wherein the measured data linked to the time stamp are configured to be stored in the memory unit for storing the course of therapy.

12. The therapeutic device of one of the preceding claims, wherein the housing (12) is configured as one of the poles of a capacitor for capacitive coupling.

13. The therapeutic device of one of the preceding claims, wherein the energy storage unit (10) is configured as a rechargeable element, for example as a lithium-ion element or as a supercapacitance.

14. The therapeutic device of claim 13, wherein the energy storage unit includes a negative pole configured as one of the poles of a capacitor for a capacitive coupling.

15. The therapeutic device of one of the preceding claims, wherein the housing (12) comprises an inner side which contains an electrically conductive or conductible surface, for example a conductive plastic or a plastic coated with an electrically conductible material.

**Revendications**

1. Dispositif thérapeutique pour la stimulation cellulaire ou la thérapie cellulaire, comprenant un boîtier (12) contenant une électrode (1), un générateur (3) pour générer des impulsions de tension à haute fréquence, une unité de traitement (6) comprenant un module de commande, de régulation et de calcul, une unité de mémoire (9), au moins un élément de commande (5, 15) et un modulateur contrôlable (4) pour contrôler le générateur (3), dans lequel une séquence d'impulsions de tension comprenant une pluralité d'impulsions de tension peut être générée au moyen du modulateur (4), dans lequel une fréquence et une durée des impulsions de tension sont réglables au moyen du modulateur (4), dans lequel l'électrode (1), le générateur (3), l'unité de traitement (6), l'unité de mémoire (9), l'élément de commande (5) et le modulateur (4) sont disposés dans le boîtier (12), dans lequel l'électrode (1) contient un corps en verre (27) comprenant une cavité contenant un gaz, dans lequel l'électrode (1) comprend une première extrémité (21) qui peut être couplée au modulateur (4), dans laquelle l'électrode comprend une deuxième extrémité en forme de dôme (22), dans laquelle le gaz peut être transformé en un plasma primaire non thermique par les impulsions de tension transmises à l'électrode (1), dans laquelle un plasma secondaire peut être généré en ionisant l'air présent dans la région entourant la deuxième extrémité (22) de l'électrode (1).

2. Le dispositif thérapeutique de la revendication 1, dans lequel la fréquence de la séquence d'impulsions de tension est au moins partiellement non constante.

3. Le dispositif thérapeutique de l'une des revendications 1 ou 2, dans lequel l'amplitude de la tension augmente pendant une période de temps t2-t1, est constante pendant une période de temps t3-t2 et diminue pendant une période de temps t4-t3, dans lequel la durée de la séquence d'impulsions de tension correspond à la période de temps t4-t1 et/ou dans lequel la fréquence augmente pendant la période de temps t2-t1, est constante pendant la période de temps t3-t2 et diminue pendant la période de temps t4-t3.

4. Le dispositif thérapeutique de l'une des revendications précédentes, dans lequel la fréquence maximale est comprise entre 10 et 100 Hz inclus.

5. Le dispositif thérapeutique de l'une des revendications précédentes, dans lequel la tension à la sortie du modulateur (4) est comprise entre 8 V et 65 V inclus.

6. Le dispositif thérapeutique de l'une des revendications précédentes, dans lequel la tension à la sortie du générateur (3) est comprise entre 5 kV et 25 kV.

7. Le dispositif thérapeutique de l'une des revendications précédentes, dans lequel une unité de stockage d'énergie (10) disposée dans le boîtier (12) est prévue pour fournir l'énergie nécessaire au fonctionnement de l'appareil thérapeutique, de sorte que l'appareil thérapeutique peut être utilisé sans fil et/ou dans lequel le boîtier (12) contient un élément d'affichage (7) au moyen duquel des données thérapeutiques et de fonctionnement peuvent notamment être affichées.

8. Le dispositif thérapeutique de l'une des revendications précédentes, dans lequel l'électrode (1) comprend un capteur (2) au moyen duquel le courant ou la tension émis par l'électrode (1) peut être enregistré sous forme de valeurs mesurées, les valeurs mesurées étant numérisables en données mesurées, les données mesurées pouvant être stockées dans l'unité de mémoire (9), le module de calcul de l'unité de traitement (6) étant configuré pour déterminer l'énergie délivrée et/ou le profil temporel de l'énergie émise par l'électrode (1).

9. Le dispositif thérapeutique de la revendication 8, dans lequel le module de commande de l'unité de traitement (6) est configuré pour commander le modulateur (4) sur la base des données mesurées, en particulier pour commander une sortie d'énergie constante et/ou pour une commande indépendante de la forme du signal, de sorte que toute forme de signal souhaitée puisse être générée dans le générateur (3), par exemple une combinaison de modulation d'amplitude et de modulation de fréquence.

10. Le dispositif thérapeutique de l'une des revendications 8 ou 9, dans lequel les données mesurées sont configurées pour contrôler un traitement au moyen du module de commande de l'unité de traitement (6).

11. Le dispositif thérapeutique de la revendication 10, dans lequel les données mesurées dans l'unité de traitement (6) peuvent être liées à un horodatage, les données mesurées liées à l'horodatage étant configurées pour être stockées dans l'unité de mémoire pour stocker le cours de la thérapie.

12. Le dispositif thérapeutique de l'une des revendications précédentes, dans lequel le boîtier (12) est configuré comme l'un des pôles d'un condensateur pour le couplage capacitif.

13. Le dispositif thérapeutique de l'une des revendications précédentes, dans lequel l'unité de stockage d'énergie (10) est configurée comme un élément rechargeable, par exemple comme un élément lithium-ion ou comme une supercapacité.

14. Le dispositif thérapeutique de la revendication 13, dans lequel l'unité de stockage d'énergie comprend un pôle négatif configuré comme l'un des pôles d'un condensateur pour un couplage capacitif.

15. Le dispositif thérapeutique de l'une des revendications précédentes, dans lequel le boîtier (12) comprend une face interne qui contient une surface conductrice ou conduite d'électricité, par exemple un plastique conducteur ou un plastique revêtu d'un matériau conducteur d'électricité.

# Fig. 1a

## Fig. 1b

## Fig. 1c

# Fig. 2 (Stand der Technik)

101

103

104

106

105

107

110

# Fig. 3

9

6

4

3

1

13

11

14

10

## Fig. 4a (Stand der Technik)

## Fig. 4b (Stand der Technik)

## Fig. 5a

## Fig. 5b

## Fig. 6a

71   A   17   72
A
12

## Fig. 6b

L1   L2

21   1   23   25   24   22

## Fig. 6c

85   B   83   84
B
81   18   82

## Fig. 6d

L1   L3

21   1   23   25   24   22

## Fig. 6e

L1   L3

21   1   23   25   24   22

# Fig. 6f

# Fig. 6g

# Fig. 7

## Fig. 8a

## Fig. 8b

## Fig. 9

## Fig. 10a

## Fig. 10b

## Fig. 10c

## Fig. 10d

# Fig. 11

# Fig. 12a

## Fig. 12b

## Fig. 12c

Fig.13

Fig. 14

Fig. 15

Fig. 16

## Fig. 17a

## Fig. 17b

Fig. 17c

Fig. 17d

Fig. 17e

Fig. 17f

Fig. 18a

# Fig. 18b

# Fig. 18c

# Fig. 19

# Fig. 20

# Fig. 21

# Fig. 22a

# Fig. 22b

## Fig. 23

## Fig. 24

## Fig. 25

# Fig. 26

# Fig. 27

# Fig. 28

# Fig. 29

## Fig. 30

## Fig. 31

## Fig. 32

## Fig. 33

## Fig. 34

## Fig. 35

## Fig. 36

## Fig. 37

## Fig. 38

## Fig. 39

## Fig. 40

# Fig. 41

# Fig. 42

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2822892 A1
- EP 2397187 A1
- DE 10324926 B3
- DE 102008045830 A1
- EP 2163143 B1
- US 6866082 A
- US 2014219894 A1

**In der Beschreibung aufgeführte Nicht-Patentliteratur**

- *CHEMICAL ABSTRACTS,* 119171-73-2